(19) Europäisches Patentamt / European Patent Office / Office européen des brevets

(11) **EP 4 504 829 B1**

(12) **EUROPÄISCHE PATENTSCHRIFT**

(45) Veröffentlichungstag und Bekanntmachung des Hinweises auf die Patenterteilung:
**08.04.2026 Patentblatt 2026/15**

(21) Anmeldenummer: **22818690.4**

(22) Anmeldetag: **18.11.2022**

(51) Internationale Patentklassifikation (IPC):
***C07C 67/03*** (2006.01) ***C25B 13/07*** (2021.01)
***C25B 15/08*** (2006.01) ***C25B 3/07*** (2021.01)
***C25B 3/25*** (2021.01) ***C25B 9/19*** (2021.01)
***C25B 9/21*** (2021.01) ***C08G 63/183*** (2006.01)
***C08J 11/22*** (2006.01) ***C08J 11/24*** (2006.01)

(52) Gemeinsame Patentklassifikation (CPC):
(C-Sets verfügbar)
**C08J 11/22; C07C 67/03; C08G 63/183; C08J 11/24; C25B 3/07; C25B 3/25; C25B 9/19; C25B 9/21; C25B 13/07; C25B 15/081;**
C08J 2367/02; Y02W 30/62 (Forts.)

(86) Internationale Anmeldenummer:
**PCT/EP2022/082364**

(87) Internationale Veröffentlichungsnummer:
**WO 2023/193940 (12.10.2023 Gazette 2023/41)**

# (54) VERBESSERTES VERFAHREN ZUR DEPOLYMERISIERUNG VON POLYETHYLENTEREPHTHALAT

IMPROVED METHOD FOR THE DEPOLYMERISATION OF POLYETHYLENE TEREPHTHALATE

PROCÉDÉ AMÉLIORÉ DE DÉPOLYMÉRISATION DU POLYÉTHYLÈNE TÉRÉPHTALATE

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **04.04.2022 EP 22166553**

(43) Veröffentlichungstag der Anmeldung:
**12.02.2025 Patentblatt 2025/07**

(73) Patentinhaber: **Evonik Operations GmbH**
**45128 Essen (DE)**

(72) Erfinder:
- **REINSBERG, Philip Heinrich**
  **53359 Rheinbach (DE)**
- **HORN, Michael**
  **53859 Niederkassel (DE)**
- **RUWWE, Johannes**
  **63457 Hanau (DE)**
- **WEINER, Marc**
  **53840 Troisdorf (DE)**

(74) Vertreter: **Evonik Patent Association**
**c/o Evonik Industries AG**
**IP Management**
**Postcode 84/339**
**Rodenbacher Chaussee 4**
**63457 Hanau (DE)**

(56) Entgegenhaltungen:
**EP-A1- 1 457 479**
**DE-A1- 10 360 758**
**JP-A- 2003 160 650**
**JP-B1- S 499 115**
**US-A- 5 425 856**
**US-A1- 2006 226 022**
**US-A1- 2008 142 373**
**US-A1- 2008 173 540**
**US-A1- 2008 173 551**
**US-A1- 2021 301 407**
**US-B2- 11 174 559**

- **DUQUE-INGUNZA I. ET AL: "Process optimization for catalytic glycolysis of post-consumer PET wastes", JOURNAL OF CHEMICAL TECHNOLOGY AND BIOTECHNOLOGY, vol. 89, no. 1, 1 January 2014 (2014-01-01), Hoboken, USA, pages 97 - 103, XP055956431, ISSN: 0268-2575, DOI: 10.1002/jctb.4101**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 4 504 829 B1



**(Forts. nächste Seite)**

- **ANONYMOUS: "NASICON", 25 June 2014 (2014-06-25), XP002807449, Retrieved from the Internet <URL:https://en.wikipedia.org/wiki/NASICON> [retrieved on 20220902]**

(52) Gemeinsame Patentklassifikation (CPC): (Forts.)

C-Sets
**C07C 67/03, C07C 69/82**

## Beschreibung

Die vorliegende Erfindung betrifft ein Verfahren zur Depolymerisierung von Polyethylenterephthalat (= **"PET"**), in welchem **PET** mit elektrolytisch hergestelltem Alkalimetallglykolat, insbesondere Natrium- oder Kaliumglykolat, zu einer Mischung $\mathbf{M_1}$ umfassend Bis-2-hydroxyethylterephthalat (= "**BHET**"; CAS-Nr.: 959-26-2) umgesetzt wird.

Das erfindungsgemäße Verfahren zeichnet sich dadurch aus, dass **BHET** einen besonders hohen Anteil unter den Spaltprodukten in der Mischung $\mathbf{M_1}$ bildet. Dadurch liefert das erfindungsgemäße Verfahren eine hohe Ausbeute an **BHET,** welches direkt für die erneute **PET**-Herstellung eingesetzt werden kann.

Die vorliegende Erfindung betrifft somit auch ein Verfahren zum Recycling von **PET,** in dem das im Verfahren zur Depolymerisierung von **PET** erhaltene **BHET,** gegebenenfalls nach weiterer Reinigung aus $\mathbf{M_1}$, wieder zu **PET** polymerisiert wird.

## Hintergrund der Erfindung

Polyethylenterephthalat (= **"PET")** ist einer der bedeutendsten Kunststoffe, der in Textilfasern, als Folien und als Material für Kunststoffflaschen verwendet wird. Allein 2007 lag die in Kunststoffflaschen verwendete Menge bei - $10^7$ t (W. Caseri, Polyethylenterephthalate, RD-16-03258 (2009) in F. Böckler, B. Dill, G. Eisenbrand, F. Faupel, B. Fugmann, T. Gamse, R. Matissek, G. Pohnert, A. Rühling, S. Schmidt, G. Sprenger, RÖMPP [Online], Stuttgart, Georg Thieme Verlag, Januar 2022).

Aufgrund seiner Haltbarkeit und die auf **PET** zurückzuführenden Müllmengen stellt es eine der größten ökologischen Herausforderungen der Gegenwart dar. Die Lösung dieses Problems liegt in der Vermeidung und in der effizienten Wiederverwertung von **PET.**

Im Stand der Technik werden mehrere Verfahren zur Spaltung von **PET** vorgeschlagen.

GB 784,248 A beschreibt die Methanolyse von **PET.**

Hydrolytische Verfahren zur Depolymerisierung von **PET** beschreiben JP 2000-309663 A, US 4,355,175 A und T. Yoshioka, N. Okayama, A. Okuwaki, Ind. Eng. Chem. Res. 1998, 37, 336 - 340.

Die Umsetzung von **PET** mit Glykol wird in der EP 0723951 A1, der US 3,222,299 A, der

WO 2020/002999 A2, von S.R. Shukla, A.M. Harad, Journal of Applied Polymer Science 2005, 97, 513 - 517 (im Folgenden "Shukla & Harad") und von N.D. Pingale, S.R. Shukla, European Polymer Journal 2008,44, 4151 - 4156 beschrieben. EP1457479A1 offenbart ein Verfahren zur Synthese und Isolierung von BHET aus PET mit NaOH in 1,2-Ethylenglykol. In EP1457479A1 wird die Verwendung einer Elektrolysezelle zur Herstellung eines Glykolytes nicht offenbart.

Shukla & Harad beschreiben, dass bei der PET-Glykolyse Bis-2-hydroxyethylterephthalat (= **"BHET")** entsteht. Dieses Spaltprodukt kann gleichzeitig als Edukt zur Herstellung neuen **PETs** eingesetzt werden.

Es besteht demnach ein Interesse an Verfahren zur Depolymerisierung von **PET,** bei dem ein möglichst hoher Anteil an **BHET** unter den Spaltprodukten erhalten wird.

Die Aufgabe der vorliegenden Erfindung bestand darin, ein solches Verfahren zur Verfügung zu stellen.

## Kurzbeschreibung der Erfindung

Es wurde nun überraschend ein Verfahren gefunden, das die erfindungsgemäße Aufgabe löst.

Die vorliegende Erfindung betrifft ein Verfahren zur Depolymerisierung von Polyethylenterephthalat **PET,** umfassend die folgenden Schritte:

(a) Herstellung einer Lösung $\mathbf{L_1}$ <21> von $M_A$-Glykolat in Glykol, wobei $M_A$ ein Alkalimetallkation ist, insbesondere ausgewählt aus Lithium, Kalium, Natrium ist, bevorzugt ausgewählt aus Kalium, Natrium ist, und am bevorzugtesten Natrium ist, in einer Elektrolysezelle **E** <1>, umfassend

- mindestens eine Anodenkammer $\mathbf{K_A}$ <11> mit mindestens einem Zulauf $\mathbf{Z_{KA}}$ <110>, mindestens einem Ablauf $\mathbf{A_{KA}}$ <111> und einem Innenraum $I_{KA}$ <112>, der eine anodische Elektrode $\mathbf{E_A}$ <113> umfasst,

- mindestens eine Kathodenkammer $\mathbf{K_K}$ <12> mit mindestens einem Zulauf $\mathbf{Z_{KK}}$ <120>, mindestens einem Ablauf $\mathbf{A_{KK}}$ <121> und einem Innenraum $\mathbf{I_{KK}}$ <122>, der eine kathodische Elektrode $\mathbf{E_K}$ <123> umfasst,

- und gegebenenfalls mindestens eine dazwischen liegende Mittelkammer $\mathbf{K_M}$ <13> mit mindestens einem Zulauf $\mathbf{Z_{KM}}$ <130>, mindestens einem Ablauf $\mathbf{A_{KM}}$ <131> und einem Innenraum $\mathbf{I_{KM}}$ <132>,
  wobei dann $\mathbf{I_{KA}}$ <112> und $\mathbf{I_{KM}}$ <132> durch eine Diffusionsbarriere **D** <14> voneinander abgetrennt sind, und $\mathbf{A_{KM}}$ <131> durch eine Verbindung $\mathbf{V_{AM}}$ <15> mit dem Zulauf $\mathbf{Z_{KA}}$ <110> verbunden ist, so dass durch die

Verbindung $\mathbf{V_{AM}}$ <15> Flüssigkeit aus $\mathbf{I_{KM}}$ <132> in $\mathbf{I_{KA}}$ <112> geleitet werden kann, wobei

- in den Fällen, in denen die Elektrolysezelle **E** <1> keine Mittelkammer $\mathbf{K_M}$ <13> umfasst, $\mathbf{I_{KA}}$ <112> und $\mathbf{I_{KK}}$ <122> durch eine Trennwand **W** <16> voneinander abgetrennt sind,

- in den Fällen, in denen die Elektrolysezelle **E** <1> mindestens eine Mittelkammer $\mathbf{K_M}$ <13> umfasst, $\mathbf{I_{KK}}$ <122> und $\mathbf{I_{KM}}$ <132> durch eine Trennwand **W** <16> voneinander abgetrennt sind,

wobei die Trennwand **W** <16> eine Seite $\mathbf{S_{KK}}$ <161> mit der Oberfläche $\mathbf{O_{KK}}$ <163> und eine der Seite $\mathbf{S_{KK}}$ <161> gegenüberliegende Seite $\mathbf{S_{A/MK}}$ <162> mit der Oberfläche $\mathbf{O_{A/MK}}$ <164> aufweist, wobei die Trennwand **W** <16> mindestens eine alkalikationenleitende Festelektrolytkeramik $\mathbf{F_A}$ <18> dergestalt umfasst, dass die von der Trennwand **W** <16> umfasste alkalikationenleitende Festelektrolytkeramik $\mathbf{F_A}$ <18> den Innenraum $\mathbf{I_{KK}}$ <122> auf der Seite $\mathbf{S_{KK}}$ <161> über die Oberfläche $\mathbf{O_{KK}}$ <163> direkt kontaktiert,

und wobei

- in den Fällen, in denen die Elektrolysezelle **E** <1> keine Mittelkammer $\mathbf{K_M}$ <13> umfasst, die von der Trennwand **W** <16> umfasste alkalikationenleitende Festelektrolytkeramik $\mathbf{F_A}$ <18> den Innenraum $\mathbf{I_{KA}}$ <112> auf der Seite $\mathbf{S_{A/MK}}$ <162> über die Oberfläche $\mathbf{O_{A/MK}}$ <164> direkt kontaktiert,

- in den Fällen, in denen die Elektrolysezelle **E** <1> mindestens eine Mittelkammer $\mathbf{K_M}$ <13> umfasst, die von der Trennwand **W** <16> umfasste alkalikationenleitende Festelektrolytkeramik $\mathbf{F_A}$ <18> den Innenraum $\mathbf{I_{KM}}$ <132> auf der Seite $\mathbf{S_{A/MK}}$ <162> über die Oberfläche $\mathbf{O_{A/MK}}$ <164> direkt kontaktiert,

($\alpha$) wobei in der Elektrolysezelle **E** <1>, wenn diese keine Mittelkammer $\mathbf{K_M}$ <13> umfasst, die folgenden, gleichzeitig ablaufenden Schritte ($\alpha$1), ($\alpha$2), ($\alpha$3) durchgeführt werden:

($\alpha$1) eine Lösung $\mathbf{L_2}$ <22> umfassend Glykol wird durch $\mathbf{I_{KK}}$ <122> geleitet,

($\alpha$2) eine neutrale oder alkalische, wässrige Lösung $\mathbf{L_3}$ <23> eines Salzes **S** umfassend $M_A$ als Kation wird durch $\mathbf{I_{KA}}$ <112> geleitet,

($\alpha$3) zwischen $\mathbf{E_A}$ <113> und $\mathbf{E_K}$ <123> wird Spannung angelegt,
oder

($\beta$) wobei in der Elektrolysezelle **E** <1>, wenn diese mindestens eine Mittelkammer $\mathbf{K_M}$ <13> umfasst, die folgenden, gleichzeitig ablaufenden Schritte ($\beta$1), ($\beta$2), ($\beta$3) durchgeführt werden:

($\beta$1) eine Lösung $\mathbf{L_2}$ <22> umfassend Glykol wird durch $\mathbf{I_{KK}}$ <122> geleitet,

($\beta$2) eine neutrale oder alkalische, wässrige Lösung $\mathbf{L_3}$ <23> eines Salzes **S** umfassend $M_A$ als Kation wird durch $\mathbf{I_{KM}}$ <132>, dann über $\mathbf{V_{AM}}$ <15>, dann durch $\mathbf{I_{KA}}$ <112> geleitet,

($\beta$3) zwischen $\mathbf{E_A}$ <113> und $\mathbf{E_K}$ <123> wird Spannung angelegt,

wodurch am Ablauf $\mathbf{A_{KK}}$ <121> die Lösung $\mathbf{L_1}$ <21> erhalten wird, wobei die Konzentration von $M_A$-Glykolat in $\mathbf{L_1}$ <21> höher ist als in $\mathbf{L_2}$ <22>,

und wodurch am Ablauf $\mathbf{A_{KA}}$ <111> eine wässrige Lösung $\mathbf{L_4}$ <24> von **S** erhalten wird, wobei die Konzentration von **S** in $\mathbf{L_4}$ <24> geringer ist als in $\mathbf{L_3}$ <23>;

(b) Umsetzung der Lösung $\mathbf{L_1}$ <21> mit **PET** zu einer Mischung $\mathbf{M_1}$ umfassend Bis-2-hydroxyethylterephthalat (= **"BHET").**

In einem weiteren Aspekt betrifft die vorliegende Erfindung ein Verfahren zum Recycling von **PET,** in welchem in einem Schritt ($\zeta$) das im erfindungsgemäßen Verfahren zur Depolymerisierung erhaltene **BHET** zu **PET** polymerisiert wird.

Es wurde überraschend gefunden, dass bei Umsetzung des **PETs** mit der durch das erfindungsgemäße elektrolytische Verfahren erhaltenen Lösung **$L_1$** <21> ein höherer Anteil an **BHET** erhalten wird als in herkömmlichen Verfahren, in denen die alkalische Alkalimetall-Glykolatlösung durch Mischung des Glykols im entsprechenden Alkalimetallhydroxid erhalten wird.

**Abbildungen**

**Abbildungen 1 A und 1 B**

Abbildung 1 A (= "Fig. 1 A") zeigt das erfindungsgemäße Verfahren zur Herstellung der Natriumglykolatlösung **$L_1$** <21> in einer Elektrolysezelle **E** <1>. Diese umfasst eine Kathodenkammer **$K_K$** <12> und eine Anodenkammer **$K_A$** <11>.
Die Kathodenkammer **$K_K$** <12> umfasst eine kathodische Elektrode **$E_K$** <123> im Innenraum $I_{KK}$ <122>, einen Zulauf **$Z_{KK}$** <120> und einen Ablauf **$A_{KK}$** <121>.
Die Anodenkammer **$K_A$** <11> umfasst eine anodische Elektrode **$E_A$** <113> im Innenraum **$I_{KA}$** <112>, einen Zulauf **$Z_{KA}$** <110> und einen Ablauf **$A_{KA}$** <111>.
Die beiden Kammern **$K_A$** <11> und **$K_K$** <12> werden von einer Außenwand **$W_A$** <80> der Zweikammerzelle **E** <1> begrenzt. Der Innenraum **$I_{KK}$** <122> ist außerdem durch eine Trennwand **W** <16>, die aus einer Scheibe einer für Natriumionen selektiv permeablen NaSICON-Festelektrolytkeramik **$F_A$** <18> besteht, vom Innenraum **$I_{KA}$** <112> abgetrennt. Die NaSICON-Festelektrolytkeramik **$F_A$** <18> erstreckt sich über die gesamte Tiefe und Höhe der Zweikammerzelle **E** <1>. Die Trennwand weist zwei Seiten **$S_{KK}$** <161> und **$S_{A/MK}$** <162> auf, deren Oberflächen **$O_{KK}$** <163> und **$O_{A/MK}$** <164> den jeweiligen Innenraum **$I_{KK}$** <122> bzw. **$I_{KA}$** <112> kontaktieren.
Eine wässrige Lösung von Natriumchlorid **$L_3$** <23> mit pH 10.5 wird über den Zulauf **$Z_{KA}$** <110> entgegen der Schwerkraft in den Innenraum **$I_{KA}$** <112> gegeben.
Über den Zulauf **$Z_{KK}$** <120> wird eine Lösung von 1 Gew.-% Natriumglykolat in Glykol **$L_2$** <22> in den Innenraum **$I_{KK}$** <122> geleitet.
Es wird dabei eine Spannung zwischen der kathodischen Elektrode **$E_K$** <123> und der anodischen Elektrode **$E_A$** <113> angelegt. Dadurch wird im Innenraum **$I_{KK}$** <122> Glykol im Elektrolyten **$L_2$** <22> zu Glykolat und $H_2$ reduziert ($HOCH_2CH_2OH + e^- \rightarrow HOCH_2CH_2O^- + \frac{1}{2} H_2$; daneben auch $HOCH_2CH_2O^- + e^- \rightarrow {}^-OCH_2CH_2O^- + \frac{1}{2} H_2$). Natriumionen diffundieren dabei vom Innenraum **$I_{KA}$** <112> durch die NaSICON-Festelektrolytkeramik **$F_A$** <18> in den Innenraum **$I_{KK}$** <122>. Insgesamt erhöht sich dadurch die Konzentration von Natriumglykolat im Innenraum **$I_{KK}$** <122>, wodurch am Ablauf **$A_{KK}$** <121> eine glykolische Lösung von Natriumglykolat **$L_1$** <21> erhalten wird, deren Konzentration an Natriumglykolat gegenüber **$L_2$** <22> erhöht ist und bei ~ 20 Gew.-% Na-Glykolat in Glykol liegt.
Im Innenraum **$I_{KA}$** <112> findet die Oxidation von Chloridionen zu molekularem Chlor statt ($Cl^- \rightarrow \frac{1}{2} Cl_2 + e^-$). Am Ablauf **$A_{KA}$** <111> wird eine wässrige Lösung **$L_4$** <24> erhalten, in der der Gehalt an NaCl gegenüber **$L_3$** <23> verringert ist. Chlorgas $Cl_2$ bildet in Wasser gemäß der Reaktion $Cl_2 + H_2O \rightarrow HOCl + HCl$ hypochlorige Säure und Salzsäure, welche mit weiteren Wassermolekülen sauer reagieren. Die Acidität schädigt die NaSICON-Festelektrolytkeramik **$F_A$** <18>.
Abbildung 1 B (= "Fig. 1 B") zeigt eine weitere Ausführungsform des erfindungsgemäßen Verfahrens anhand einer Elektrolysezelle **E** <1>, die eine Mittelkammer **$K_M$** <13> umfasst. Diese Dreikammerzelle **E** <1> umfasst demnach eine Kathodenkammer **$K_K$** <12>, eine Anodenkammer **$K_A$** <11> und eine dazwischen liegende Mittelkammer **$K_M$** <13>.
Die Kathodenkammer **$K_K$** <12> umfasst eine kathodische Elektrode **$E_K$** <123> im Innenraum **$I_{KK}$** <122>, einen Zulauf **$Z_{KK}$** <120> und einen Ablauf **$A_{KK}$** <121>.
Die Anodenkammer **$K_A$** <11> umfasst eine anodische Elektrode **$E_A$** <113> im Innenraum **$I_{KA}$** <112>, einen Zulauf **$Z_{KA}$** <110> und einen Ablauf **$A_{KA}$** <111>.
Die Mittelkammer **$K_M$** <13> umfasst einen Innenraum **$I_{KM}$** <132>, einen Zulauf **$Z_{KM}$** <130> und einen Ablauf **$A_{KM}$** <131>.
Der Innenraum **$I_{KA}$** <112> ist mit dem Innenraum **$I_{KM}$** <132> über die Verbindung **$V_{AM}$** <15> verbunden.
Die drei Kammern werden von einer Außenwand **$W_A$** <80> der Dreikammerzelle **E** <1> begrenzt. Der Innenraum **$I_{KM}$** <132> der Mittelkammer **$K_M$** <13> ist außerdem durch eine Trennwand **W** <16>, die aus einer Scheibe einer für Natriumionen selektiv permeablen NaSICON-Festelektrolytkeramik **$F_A$** <18> besteht, vom Innenraum **$I_{KK}$** <122> der Kathodenkammer **$K_K$** <12> abgetrennt. Die NaSICON-Festelektrolytkeramik **$F_A$** <18> erstreckt sich über die gesamte Tiefe und Höhe der Dreikammerzelle **E** <1>. Die Trennwand weist zwei Seiten **$S_{KK}$** <161> und **$S_{A/MK}$** <162> auf, deren Oberflächen **$O_{KK}$** <163> und **$O_{A/MK}$** <164> den jeweiligen Innenraum **$I_{KK}$** <122> bzw. **$I_{KM}$** <132> kontaktieren.
Der Innenraum **$I_{KM}$** <132> der Mittelkammer **$K_M$** <13> ist zusätzlich wiederum durch eine Diffusionsbarriere **D** <14> vom Innenraum **$I_{KA}$** <112> der Anodenkammer **$K_A$** <11> abgetrennt. Die NaSICON-Festelektrolytkeramik **$F_A$** <18> und die Diffusionsbarriere **D** <14> erstrecken sich über die gesamte Tiefe und Höhe der Dreikammerzelle **E** <1>. Die Diffusionsbarriere **D** <14> ist eine Kationenaustauschermembran (sulfoniertes PTFE).
In der Ausführungsform gemäß Abbildung 1 B wird die Verbindung **$V_{AM}$** <15> außerhalb der Elektrolysezelle **E** <1> ausgebildet, insbesondere durch ein Rohr oder Schlauch, dessen Material aus Gummi, Metall oder Kunststoff

ausgewählt sein kann. Durch die Verbindung **$V_{AM}$** <15> kann Flüssigkeit aus dem Innenraum **$I_{KM}$** <132> der Mittelkammer **$K_M$** <13> in den Innenraum **$I_{KA}$** <112> der Anodenkammer **$K_A$** <11> außerhalb der Außenwand **$W_A$** <80> der Dreikammerzelle **E** <1> geleitet werden. Die Verbindung **$V_{AM}$** <15> verbindet den Ablauf **$A_{KM}$** <131 >, der am Boden der Mittelkammer **$K_M$** <13> die Außenwand **$W_A$** <80> der Elektrolysezelle **E** <1> durchbricht, mit dem Zulauf **$Z_{KA}$** <110>, der am Boden der Anodenkammer **$K_A$** <11> die Außenwand **$W_A$** <80> der Elektrolysezelle **E** <1> durchbricht.

Eine wässrige Lösung von Natriumchlorid **$L_3$** <23> mit pH 10.5 wird über den Zulauf **$Z_{KM}$** <130> gleichgerichtet mit der Schwerkraft in den Innenraum **$I_{KM}$** <132> der Mittelkammer **$K_M$** <13> gegeben. Durch die Verbindung **$V_{AM}$** <15> ist der Innenraum **$I_{KM}$** <132> der Mittelkammer **$K_M$** <13> mit dem Innenraum **$I_{KA}$** <112> der Anodenkammer **$K_A$** <11> verbunden. Natriumchloridlösung **$L_3$** <23> wird durch diese Verbindung **$V_{AM}$** <15> vom Innenraum **$I_{KM}$** <132> in den Innenraum **$I_{KM}$** <112> geleitet.

Über den Zulauf **$Z_{KK}$** <120> wird eine Lösung von ~ 1 Gew.-% Natriumglykolat in Glykol **$L_2$** <22> in den Innenraum **$I_{KK}$** <122> geleitet.

Es wird dabei eine Spannung zwischen der kathodischen Elektrode **$E_K$** <123> und der anodischen Elektrode **$E_A$** <113> angelegt. Dadurch wird im Innenraum **$I_{KK}$** <122> Glykol im Elektrolyten **$L_2$** <22> zu Glykolat und $H_2$ reduziert ($HOCH_2CH_2OH + e^- \rightarrow HOCH_2CH_2O^- + ½\ H_2$; daneben auch $HOCH_2CH_2O^- + e^- \rightarrow {}^-OCH_2CH_2O- + ½\ H_2$). Natriumionen diffundieren dabei vom Innenraum **$I_{KM}$** <132> der Mittelkammer **$K_M$** <103> durch die NaSICON-Festelektrolytkeramik **$F_A$** <18> in den Innenraum **$I_{KK}$** <122>. Insgesamt erhöht sich dadurch die Konzentration von Natriumglykolat im Innenraum **$I_{KK}$** <122>, wodurch am Ablauf **$A_{KK}$** <121> eine glykolische Lösung von Natriumglykolat **$L_1$** <21> erhalten wird, deren Konzentration an Natriumglykolat gegenüber **$L_2$** <22> auf ~ 20 Gew.-% Natriumglykolat in Glykol erhöht ist.

Im Innenraum **$I_{KA}$** <112> findet die Oxidation von Chloridionen zu molekularem Chlor statt ($Cl^- \rightarrow ½\ Cl_2 + e^-$). Am Ablauf **$A_{KA}$** <111> wird eine wässrige Lösung **$L_4$** <24> erhalten, in der der Gehalt an NaCl gegenüber **$L_3$** <23> verringert ist. Chlorgas $Cl_2$ bildet in Wasser gemäß der Reaktion $Cl_2 + H_2O \rightarrow HOCl + HCl$ hypochlorige Säure und Salzsäure, welche mit weiteren Wassermolekülen sauer reagieren. Die Acidität würde die NaSICON-Festelektrolytkeramik **$F_A$** <18> schädigen, wird aber durch die Anordnung in der Dreikammerzelle auf die Anodenkammer **$K_A$** <11> begrenzt und somit in der Elektrolysezelle **E** <1> von der NaSICON-Festelektrolytkeramik **$F_A$** <18> ferngehalten. Dadurch erhöht sich deren Lebensdauer beträchtlich.

### Abbildungen 2 A und 2 B

Abbildung 2 A (= "Fig. 2 A") zeigt eine bevorzugte Trennwand **W** <16>. Diese umfasst zwei NaSICON-Festelektrolytkeramiken **$F_A$** <18> und **$F_B$** <19>, die durch ein Trennelement **T** <17> voneinander getrennt sind und jeweils lückenlos daran befestigt sind. Das Trennelement **T** <17> weist die geometrische Form eines Quaders auf, an dessen gegenüberliegenden Seiten **$F_A$** <18> und **$F_B$** <19> lückenlos befestigt sind (z.B. durch Klebstoff).

Die Seite **$S_{KK}$** <161> mit der Oberfläche **$O_{KK}$** <163> liegt in der Bildebene, die Seite **$S_{A/MK}$** <162> mit der nicht in Fig. 2 A sichtbaren Oberfläche **$O_{A/MK}$** <164> hinter der Bildebene.

Abbildung 2 B (= Fig. 2 B) zeigt eine andere Ausführungsform einer bevrozugten Trennwand **W** <16>. Diese umfasst vier NaSICON-Festelektrolytkeramiken **$F_A$** <18>, **$F_B$** <19>, **$F_C$** <28>, **$F_D$** <29>, die durch ein Trennelement **T** <17> voneinander getrennt sind und jeweils lückenlos daran befestigt sind. Das Trennelement **T** <17> weist die Form eines Kreuzes auf, an dessen gegenüberliegenden Seiten **$F_A$** <18>, **$F_B$** <19>, **$F_C$** <28> und **$F_D$** <29> festgeklebt sind. Die Seite **$S_{KK}$** <161> mit der Oberfläche **$O_{KK}$** <163> liegt in der Bildebene, die Seite **$S_{A/MK}$** <162> mit der nicht in Fig. 2 B sichtbaren Oberfläche **$O_{A/MK}$** <164> hinter der Bildebene.

### Abbildungen 3 A bis 3 C

Abbildung 3 A (= "Fig. 3 A") zeigt die Detailansicht, welche in den Abbildungen 2 A und 2 B durch einen gestrichelten Kreis hervorgehoben ist. Wie beschrieben sind die jeweiligen Festelektrolytkeramiken **$F_A$** <18> und **$F_B$** <19> am Trennelement **T** <17> beispielsweise durch Klebstoff befestigt.

Abbildung 3 B (= "Fig. 3 B") illustriert eine weitere Ausführungsform einer bevorzugten Trennwand **W.** Hier weist das Trennelement **T** <17> zwei konkave Vertiefungen (Rillen) auf, in welche die beiden Festelektrolytkeramiken **$F_A$** <18> und **$F_B$** <19> eingepasst werden. Dazu kann die Form der Ränder der Festelektrolytkeramiken **$F_A$** <18> und **$F_B$** <19> entsprechend mechanisch angepasst werden. Zusätzlich wird eine Dichtung **Di** <40> eingesetzt, die zum Beispiel mit einem Klebstoff am Trennelement **T** <17> und der jeweiligen Festelektrolytkeramik **$F_A$** <18> bzw. **$F_B$** <19> angebracht wird. Das Trennelement **T** <17> kann dabei aus zwei oder mehreren Teilen <171> und <172> bestehen, die, wie durch die gestrichelte Linie in Fig. 3 B angedeutet, aneinander befestigt werden können. Bei einer entsprechenden Geometrie und Anpassung der Form der Ränder der Festelektrolytkeramiken **$F_A$** <18> bzw. **$F_B$** <19> können letztere zwischen die beiden Teile <171> und <172> geklemmt werden, was die Stabilität der Verbindung Trennelement **T** <17> / Keramik **$F_A$** <18> bzw. **$F_B$** <19> und die Dichtheit der Trennwand **W** <16> weiter verbessert.

Abbildung 3 C (= "Fig. 3 C") illustriert eine weitere Ausführungsform einer bevorzugten Trennwand **W.** Diese

entspricht der in Abbildung 3 B beschriebenen, bis darauf, dass die Vertiefungen (Rillen) im Trennelement **T** <17>, in welche die beiden Festelektrolytkeramiken $\mathbf{F_A}$ <18> und $\mathbf{F_B}$ <19> eingepasst werden, nicht konkav, sondern spitz zulaufend sind.

**Abbildungen 4 A bis 4 D**

Die Abbildungen 4 A (= "Fig. 4 A") bis 4 D zeigen weitere Ausführungsformen bevorzugter Trennwände **W** <16>.

Die in Abbildung 4 A dargestellt Trennwand **W** <16> entspricht der in Abbildung 2 A dargestellten Trennwand **W** <16>, bis darauf, dass sie auch ein Rahmenelement **R** <20> umfasst. Dieses bedeckt alle Oberflächen der Trennwand **W** <16> außer $\mathbf{O_{KK}}$ <163> und $\mathbf{O_{A/MK}}$ <164> vollständig. Das Rahmenelement **R** <20> ist nicht einstückig mit dem Trennelement **T** <17> ausgeführt.

Abbildung 4 B (= "Fig. 4 B") zeigt eine weitere Ausführungsform einer bevorzugten Trennwand **W** <16>. Diese entspricht der in Abbildung 4 A dargestellten Ausführungsform, außer dass sie zwei Rahmenelemente **R** <20> umfasst, die die obere und untere Oberfläche der Trennwand **W** <16> begrenzen.

Abbildung 4 C (= "Fig. 4 C") zeigt eine weitere Ausführungsformen einer bevorzugten Trennwand **W** <16>. Die in Abbildung 4 C dargestellt Trennwand **W** <16> entspricht der in Abbildung 2 B dargestellten Trennwand **W** <16>, bis darauf, dass sie auch ein Rahmenelement **R** <20> umfasst. Dieses bedeckt alle Oberflächen der Trennwand **W** <16> außer $\mathbf{O_{KK}}$ <163> und $\mathbf{O_{A/MK}}$ <164> vollständig. Das Rahmenelement **R** <20> ist nicht einstückig mit dem Trennelement **T** <17> ausgeführt.

Abbildung 4 D (= "Fig. 4 D") zeigt eine weitere Ausführungsformen einer bevorzugten Trennwand **W** <16>. Diese entspricht der in Abbildung 4 C dargestellten Ausführungsform, außer dass sie zwei Rahmenelemente **R** <20> umfasst, die die obere und untere Oberfläche der Trennwand **W** <16> begrenzen.

**Abbildungen 5 A und 5 B**

Abbildung 5 A (= "Fig. 5 A") zeigt eine Elektrolysezelle **E** <1> in einer bevorzugten Ausführungsform des erfindungsgemäßen Verfahrens. Diese entspricht der in Abbildung 1 A dargestellten Elektrolysezelle mit dem Unterschied, dass eine Trennwand **W** <16> den Innenraum $\mathbf{I_{KK}}$ <122> der Kathodenkammer $\mathbf{K_K}$ <12> vom Innenraum $\mathbf{I_{KA}}$ <112> der Anodenkammer $\mathbf{K_A}$ <11 > abtrennt. Die Trennwand ist jene, die in Abbildungen 2 A und 2 B dargestellt ist.

Abbildung 5 B (= "Fig. 5 B") zeigt eine Elektrolysezelle **E** <1> in einer bevorzugten Ausführungsform des erfindungsgemäßen Verfahrens. Diese entspricht der in Abbildung 1 A dargestellten Elektrolysezelle mit dem Unterschied, dass eine Trennwand **W** <16> den Innenraum $\mathbf{I_{KK}}$ <122> der Kathodenkammer $\mathbf{K_K}$ <12> vom Innenraum $\mathbf{I_{KA}}$ <112> der Anodenkammer $\mathbf{K_A}$ <11 > abtrennt. Die Trennwand **W** <16> ist jene, die in Abbildungen 4 A bis 4 D dargestellt ist. Das Rahmenelement **R** <20> bildet einen Teil der Außenwand $\mathbf{W_A}$ <80>, so dass die von der Trennwand **W** <16> umfassten Festelektrolytkeramiken vor dem Druck, der durch die Trennwand **W** <16> auf sie wirken würde, wenn sie Teil der Trennwand **W** <16> wären, geschützt sind. Zusätzlich werden die Festelektrolytkeramiken so vollständig zur Trennung der Innenräume $I_{KK}$ <122> und $I_{KA}$ <112> innerhalb der Elektrolysezelle **E** <1> eingesetzt, da sie nicht teilweise durch die Außenwand verdeckt werden.

**Abbildungen 6 A und 6 B**

Die Abbildung 6 A (= Fig. 6 A) zeigt das erfindungsgemäße Verfahren anhand einer Elektrolysezelle **E** <1>, die der in der Fig. 1 B gezeigten mit dem Unterschied entspricht, dass die Verbindung $\mathbf{V_{AM}}$ <15> vom Innenraum $\mathbf{I_{KM}}$ <132> der Mittelkammer $\mathbf{K_M}$ <13> zum Innenraum $\mathbf{I_{KA}}$ <112> der Anodenkammer $\mathbf{K_A}$ <11> durch mehrere Perforationen in der Diffusionsbarriere **D** <14> gebildet wird. Diese Perforationen können nachträglich in die Diffusionsbarriere **D** <14> gestanzt werden oder schon aufgrund des Herstellungsprozesses der Diffusionsbarriere **D** <14> von Vorneherein in dieser vorliegen (z.B. bei textilen Geweben wie Filtertüchern oder Metallgeweben). Die Gesamtheit dieser Perforationen stellt in dieser Ausführungsform die Verbindung $\mathbf{V_{AM}}$ <15> dar, durch welche Elektrolyt aus dem Innenraum $\mathbf{I_{KM}}$ <132> in den Innenraum $\mathbf{I_{KA}}$ <112> geleitet werden kann.

Abbildung 6 B (= "Fig. 6 B") zeigt eine weitere Ausführungsform des erfindungsgemäßen Verfahrens anhand einer Elektrolysezelle **E** <1>. Diese entspricht der in Abbildung 1 B dargestellten Elektrolysezelle **E** <1> mit dem Unterschied, dass die Verbindung $\mathbf{V_{AM}}$ <15> vom Innenraum $\mathbf{I_{KM}}$ <132> der Mittelkammer $\mathbf{K_M}$ <13> zum Innenraum $\mathbf{I_{KA}}$ <112> der Anodenkammer $\mathbf{K_A}$ <11> durch einen Spalt gebildet wird, der sich zwischen der Diffusionsbarriere **D** <14> und der Außenwand $\mathbf{W_A}$ <80> ausbildet. Dieser Spalt kann dadurch eingerichtet werden, dass eine ansonsten dichte Diffusionsbarriere **D** <14> so in der Elektrolysezelle **E** <1> angeordnet wird, dass sie den Innenraum $\mathbf{I_{KM}}$ <132> der Mittelkammer $\mathbf{K_M}$ <13> nicht vollständig vom Innenraum $\mathbf{I_{KA}}$ <112> der Anodenkammer $\mathbf{K_A}$ <11> abtrennt, sondern ein Spalt als Verbindung $\mathbf{V_{AM}}$ <15> beibehalten wird.

**Abbildungen 7 A und 7 B**

Abbildung 7 A (= "Fig. 7 A") zeigt eine weitere Ausführungsform einer bevorzugten Trennwand **W** <16>. Diese umfasst vier NaSICON-Festelektrolytkeramiken **$F_A$** <18>, **$F_B$** <19>, **$F_C$** <28> und **$F_D$** <29>, die durch ein Trennelement **T** <17>, welches zwei Hälften <171> und <172> umfasst, voneinander getrennt sind. Die Trennwand **W** <16> umfasst auch ein Rahmenelement **R** <20>, welches ebenfalls aus zwei Hälften <201> und <202> besteht.

Die Trennwand **W** <16> besteht aus zwei zusammenklappbaren Teilen, in denen die Hälfte <171> des Trennelements **T** <17> mit der Hälfte <201> des Rahmenelements **R** <20> einstückig vorliegt und die Hälfte <172> des Trennelements **T** <17> mit der Hälfte <202> des Rahmenelements **R** <20> einstückig vorliegt. Diese beiden Teile können optional über ein Scharnier <50> miteinander verbunden und im zusammengeklappten Zustand über das Schloss <60> arretiert werden. Zwischen diesen Hälften werden die vier NaSICON-Festelektrolytkeramiken **$F_A$** <18>, **$F_B$** <19>, **$F_C$** <28> und **$F_D$** <29> eingeklemmt, wobei zur Abdichtung jeweils ein als Dichtung **Di** <40> fungierender Ring eingesetzt wird.

Die linke Seite der Abbildung 7 A zeigt die Frontalansicht auf die Seite **$S_{KK}$** <161> mit der Oberfläche **$O_{KK}$** <163> der Trennwand **W** <16>. Die als Dichtung **Di** <40> fungierenden Ringe sind mit gestrichelten Umrissen angegeben. Die rechte Seite der Abbildung zeigt die seitliche Ansicht der Trennwand **W** <16>.

Abbildung 7 B (= "Fig. 7 B") zeigt eine weitere Ausführungsform einer bevorzugten Trennwand **W** <16>. Diese entspricht der in Abbildung 7 A beschriebenen Ausführungsform, bis darauf dass sie neun NaSICON-Festelektrolytkeramiken **$F_A$** <18>, **$F_B$** <19>, **$F_C$** <28>, **$F_D$** <29>, **$F_E$** <30>, **$F_F$** <31>, **$F_G$** <32>, **$F_H$** <33>, **$F_I$** <34> umfasst.

**Abbildung 8**

Die Abbildung 8 (= Fig. 8) zeigt den Vergleich der Gehalte von **BHET** ("1"), 2-Hydroxyethylterephthalat **("MHET";** "2") und Terephthalat ("**TS";** "3") bei der Depolymerisierung mit gemäß dem erfindungsgemäßen Verfahren erhaltenem Natriumglykolat sowie gemäß mit herkömmlichen Verfahren erhaltenem Natriumglykolat.

Die Balken mit der engen Schraffierung "///////" zeigen den jeweiligen Gehalt von **BHET, MHET** und **TS** im Reaktoraustrag bei der Depolymerisierung von **PET** gemäß dem erfinderischen Beispiel **E1,** in dem das für die Depolymerisierung verwendete Natriumglykolat durch Elektrolyse erhalten wurde.

Die schwarzen Balken zeigen den jeweiligen Gehalt von **BHET, MHET** und **TS** im Reaktoraustrag bei der Depolymerisierung von **PET** gemäß dem Vergleichsbeispiel **V1,** in dem bei der Depolymerisierung lediglich Glykol eingesetzt wurde.

Die Balken mit der fetten Schraffierung "/////" zeigen den jeweiligen Gehalt von **BHET, MHET** und **TS** im Reaktoraustrag bei der Depolymerisierung von **PET** gemäß dem Vergleichsbeispiel **V2,** in dem das für die Depolymerisierung verwendete Natriumglykolat durch Mischen von NaOH und Glykol im Reaktor erhalten wurde.

**Detaillierte Beschreibung der Erfindung**

Es wurde nun überraschend gefunden, dass die Glykolyse von **PET** besonders effizient verläuft, wenn Alkalimetallglykolat, insbesondere Natrium- oder Kaliumglykolat eingesetzt wird, welches elektrolytisch erhalten wurde. Es wurde beobachtet, dass im erfindungsgemäßen Verfahren im Vergleich zu den Verfahren des Standes der Technik, in denen Glykolat eingesetzt wird, welches durch Auflösen der entsprechenden Alkalimetallhydroxide in Glykol erhalten wurde, ein höherer Anteil von **BHET** im Spaltprodukt erhalten wird.

**1. Schritt (a): Elektrolyse zum Erhalt der Lösung $L_1$ umfassend Glykol und $M_A$-Glykolat**

Die im erfindungsgemäßen Verfahren eingesetzte Lösung **$L_1$** umfassend Glykol und $M_A$-Glykolat wird erfindungsgemäß elektrolytisch in einer Elektrolysezelle **E** <1> erhalten.

Unter "Glykol" wird im Sinne der Erfindung 1,2-Ethylendiol mit der chemischen Formel $HO-CH_2-CH_2-OH$ (CAS-Nr. 107-21-1) verstanden.

Unter "$M_A$-Glykolat" wird im Sinne der Erfindung das Salz des Glykols mit $M_A$ verstanden. Der Begriff "$M_A$-Glykolat" umfasst mindestens einen von $M_AO-CH_2-CH_2-OH$ und $M_AO-CH_2-CH_2-OM_A$, bevorzugt mindestens $M_AO-CH_2-CH_2-OH$, am bevorzugtesten $M_AO-CH_2-CH_2-OH$ und $M_AO-CH_2-CH_2-OM_A$.

$M_A$ ist ein Alkalimetallkation, welches insbesondere aus Lithium, Natrium, Kalium ausgewählt ist, und bevorzugt aus Natrium, Kalium ausgewählt ist. Am bevorzugtesten ist das Alkalimetallkation Natrium.

<u>1.1 Elektrolysezelle **E**</u>

Die in Schritt (b) des erfindungsgemäßen Verfahrens eingesetzte Lösung **$L_1$** <21> von $M_A$-Glykolat in Glykol wird in Schritt (a) des erfindungsgemäßen Verfahrens in einer Elektrolysezelle **E** hergestellt.

Die Elektrolysezelle **E** umfasst mindestens eine Anodenkammer $K_A$ und mindestens eine Kathodenkammer $K_K$ und gegebenenfalls mindestens eine dazwischen liegende Mittelkammer $K_M$. Dies umfasst auch Elektrolysezellen **E,** welche mehr als eine Anodenkammer $K_A$ und/oder Kathodenkammer $K_K$ und/oder Mittelkammer $K_M$ aufweisen. Solche Elektrolysezellen, in denen diese Kammern modulartig aneinandergefügt werden, sind beispielsweise in der DD 258 143 A3 und der US 2006/0226022 A1 beschrieben.

Die Elektrolysezelle **E** umfasst in einer bevorzugten Ausführungsform der Erfindung eine Anodenkammer $K_A$ und eine Kathodenkammer $K_K$ und gegebenenfalls eine dazwischen liegende Mittelkammer $K_M$.

Die Elektrolysezelle **E** weist üblicherweise eine Außenwand $W_A$ auf. Die Außenwand $W_A$ ist insbesondere aus einem Material, welches aus der Gruppe bestehend aus Stahl, bevorzugt gummiertem Stahl, Kunststoff, der insbesondere aus Telene ® (duroplastischem Polydicyclopentadien), PVC (Polyvinylchlorid), PVC-C (nachchloriertes Polyvinylchlorid), PVDF (Polyvinylidenfluorid) ausgewählt ist. $W_A$ kann insbesondere für Zuläufe und Abläufe durchbrochen sein. Innerhalb von $W_A$ liegen dann die mindestens eine Anodenkammer $K_A$, die mindestens eine Kathodenkammer $K_K$ und in den Ausführungsformen, in denen die Elektrolysezelle **E** eine solche umfasst, die mindestens eine dazwischen liegende Mittelkammer $K_M$.

### *1.1.1 Kathodenkammer $K_K$*

Die mindestens eine Kathodenkammer $K_K$ weist mindestens einen Zulauf $Z_{KK}$, mindestens einen Ablauf $A_{KK}$ und einen Innenraum $I_{KK}$, der eine kathodische Elektrode $E_K$ umfasst, auf.

Der Innenraum $I_{KA}$ der Anodenkammer $K_A$ ist durch eine Trennwand **W,** vom Innenraum $I_{KK}$ der Kathodenkammer $K_K$ abgetrennt, falls die Elektrolysezelle **E** keine Mittelkammer $K_M$ umfasst. Der Innenraum $I_{KK}$ der Kathodenkammer $K_K$ ist durch eine Trennwand **W,** vom Innenraum $I_{KM}$ der Mittelkammer $K_M$ abgetrennt, falls die Elektrolysezelle **E** mindestens eine Mittelkammer $K_M$ umfasst.

Die Trennwand **W** und ihre Anordnung in der Elektrolysezelle **E** wird weiter unten (Abschnitt 1.1.4) beschrieben.

#### 1.1.1.1 Kathodische Elektrode $E_K$

Die Kathodenkammer $K_K$ umfasst einen Innenraum $I_{KK}$, der wiederum eine kathodische Elektrode $E_K$ umfasst. Als solche kathodische Elektrode $E_K$ kommt jede dem Fachmann geläufige Elektrode in Frage, die unter den Bedingungen des Schrittes (a) des erfindungsgemäßen Verfahrens stabil ist. Solche sind insbesondere in WO 2014/008410 A1, Absatz [025] oder DE 10360758 A1, Absatz [030] beschrieben. Diese Elektrode $E_K$ kann aus der Gruppe bestehend aus Maschenwolle, dreidimensionale Matrixstruktur oder "Kugeln" ausgewählt sein. Die kathodische Elektrode $E_K$ umfasst insbesondere ein Material, welches ausgewählt ist aus der Gruppe bestehend aus Stahl, Nickel, Kupfer, Platin, platinierte Metalle, Palladium, auf Kohle geträgertes Palladium, Titan, bevorzugter ausgewählt ist aus der Gruppe bestehend aus Stahl, Nickel. Bevorzugt umfasst $E_K$ Stahl, noch bevorzugter VA-Stahl (= rostfreien Stahl).

In den Ausführungsformen der Elektrolysezelle **E,** in denen diese eine Mittelkammer $K_M$ umfasst, befindet sich diese zwischen der Anodenkammer $K_A$ und der Kathodenkammer $K_K$.

#### 1.1.1.2 Zulauf $Z_{KK}$ und Ablauf $A_{KK}$

Die Kathodenkammer $K_K$ umfasst auch mindestens einen Zulauf $Z_{KK}$ und mindestens einen Ablauf $A_{KK}$. Dies ermöglicht es, dem Innenraum $I_{KK}$ der Kathodenkammer $K_K$ Flüssigkeit, wie zum Beispiel die Lösung $L_2$, zuzufügen und darin befindliche Flüssigkeit, wie zum Beispiel die Lösung $L_1$, zu entfernen. Der Zulauf $Z_{KK}$ und der Ablauf $A_{KK}$ sind dabei so an der Kathodenkammer $K_K$ angebracht, dass die Flüssigkeit beim Durchströmen des Innenraums $I_{KK}$ der Kathodenkammer $K_K$ die kathodische Elektrode $E_K$ kontaktiert. Dies ist die Voraussetzung dafür, dass bei der Durchführung von Schritt (a) des erfindungsgemäßen Verfahrens am Ablauf $A_{KK}$ die Lösung $L_1$ erhalten wird, wenn die Lösung $L_2$ von Glykol, die optional auch ein $M_A$-Glykolat umfasst, durch den Innenraum $I_{KK}$ der Kathodenkammer $K_K$ geleitet wird.

Der Zulauf $Z_{KK}$ und der Ablauf $A_{KK}$ können nach dem Fachmann bekannten Verfahren an der Elektrolysezelle **E** angebracht werden, z.B. durch Bohrungen in der Außenwand und entsprechenden Anschlüssen (Ventilen), die die Einbzw. Ausleitung von Flüssigkeit vereinfachen.

### *1.1.2 Anodenkammer $K_A$*

Die mindestens eine Anodenkammer $K_A$ weist mindestens einen Zulauf $Z_{KA}$, mindestens einen Ablauf $A_{KA}$ und einen Innenraum $I_{KA}$, der eine anodische Elektrode $E_A$ umfasst, auf.

Der Innenraum $I_{KA}$ der Anodenkammer $K_A$ ist, falls die Elektrolysezelle **E** eine Mittelkammer $K_M$ umfasst, durch eine Diffusionsbarriere **D** vom Innenraum $I_{KM}$ der Mittelkammer $K_M$ abgetrennt.

Falls die Elektrolysezelle **E** keine Mittelkammer $K_M$ umfasst, ist der Innenraum $I_{KA}$ der Anodenkammer **K** durch

die Trennwand **W** vom Innenraum $I_{KK}$ der Kathodenkammer $K_K$ abgetrennt.

1.1.2.1 Anodische Elektrode $E_A$

Die Anodenkammer $K_A$ umfasst einen Innenraum $I_{KA}$, der wiederum eine anodische Elektrode $E_A$ umfasst. Als solche anodische Elektrode $E_A$ kommt jede dem Fachmann geläufige Elektrode in Frage, die unter den Bedingungen des Schrittes (a) des erfindungsgemäßen Verfahrens stabil ist. Solche sind insbesondere in WO 2014/008410 A1, Absatz [024] oder DE 10360758 A1, Absatz [031] beschrieben. Diese Elektrode $E_A$ kann aus einer Schicht bestehen oder aus mehreren planen, zueinander parallelen Schichten bestehen, die jeweils perforiert oder expandiert sein können. Die anodische Elektrode $E_A$ umfasst insbesondere ein Material, welches ausgewählt ist aus der Gruppe bestehend aus Rutheniumoxid, Iridiumoxid, Nickel, Kobalt, Nickelwolframat, Nickeltitanat, Edelmetalle wie insbesondere Platin, welches auf einem Träger wie Titan oder Kovar ® (einer Eisen/Nickel/Kobalt-Legierung, in denen die einzelnen Anteile bevorzugt wie folgt sind: 54 Massen-% Eisen, 29 Massen-% Nickel, 17 Massen-% Cobalt) geträgert ist. Weitere mögliche Anodenmaterialien sind insbesondere Edelstahl, Blei, Graphit, Wolframcarbid, Titandiborid. Bevorzugt umfasst die anodische Elektrode $E_A$ eine mit Rutheniumoxid/ Iridiumoxid beschichtete Titananode ($RuO_2$ + $IrO_2$ / Ti).

1.1.2.2 Zulauf $Z_{KA}$ und Ablauf $A_{KA}$

Die Anodenkammer $K_A$ umfasst auch einen Zulauf $Z_{KA}$ und einen Ablauf $A_{KA}$. Dies ermöglicht es, dem Innenraum $I_{KA}$ der Anodenkammer $K_A$ Flüssigkeit, wie zum Beispiel die Lösung $L_3$, zuzufügen und darin befindliche Flüssigkeit, wie zum Beispiel die Lösung $L_4$, zu entfernen. Der Zulauf $Z_{KA}$ und der Ablauf $A_{KA}$ sind dabei so an der Anodenkammer $K_A$ angebracht, dass die Flüssigkeit beim Durchströmen des Innenraums $I_{KA}$ der Anodenkammer $K_A$ die anodische Elektrode $E_A$ kontaktiert. Dies ist die Voraussetzung dafür, dass bei der Durchführung des Schrittes (a) des erfindungsgemäßen Verfahrens am Ablauf $A_{KA}$ die Lösung $L_4$ erhalten wird, wenn die Lösung $L_3$ eines Salzes **S** durch den Innenraum $I_{KA}$ der Anodenkammer $K_A$ geleitet wird.

Der Zulauf $Z_{KA}$ und der Ablauf $A_{KA}$ können nach dem Fachmann bekannten Verfahren an der Elektrolysezelle **E** angebracht werden, z.B. durch Bohrungen in der Außenwand und entsprechenden Anschlüssen (Ventilen), die die Ein- bzw. Ausleitung von Flüssigkeit vereinfachen. Der Zulauf $Z_{KA}$ kann in bestimmten Ausführungsformen, in denen die Elektrolysezelle E eine Mittelkammer $K_M$ umfasst, auch innerhalb der Elektrolysezelle liegen, beispielsweise als Perforation in der Diffusionsbarriere **D.**

*1.1.3 Optionale Mittelkammer $K_M$*

Die in Schritt (a) des erfindungsgemäßen Verfahrens eingesetzte Elektrolysezelle **E** weist gegebenenfalls mindestens eine Mittelkammer $K_M$ auf. Die optionale Mittelkammer $K_M$ liegt zwischen Kathodenkammer $K_K$ und Anodenkammer $K_A$. Sie umfasst mindestens einen Zulauf $Z_{KM}$, mindestens einen Ablauf $A_{KM}$ und einen Innenraum $I_{KM}$.

Der Innenraum $I_{KA}$ der Anodenkammer $K_A$ ist, falls die Elektrolysezelle **E** eine Mittelkammer $K_M$ umfasst, durch eine Diffusionsbarriere **D** vom Innenraum $I_{KM}$ der Mittelkammer $K_M$ abgetrennt. $A_{KM}$ ist dann außerdem durch eine Verbindung $V_{AM}$ mit dem Zulauf $Z_{KA}$ verbunden, so dass durch die Verbindung $V_{AM}$ Flüssigkeit aus $I_{KM}$ in $I_{KA}$ geleitet werden kann.

1.1.3.1 Diffusionsbarriere **D**

Der Innenraum $I_{KM}$ der optionalen Mittelkammer $K_M$ ist durch eine Diffusionsbarriere **D** vom Innenraum $I_{KA}$ der Anodenkammer $K_A$ abgetrennt und durch die Trennwand **W** vom Innenraum $I_{KK}$ der Kathodenkammer $K_K$ abgetrennt.

Für die Diffusionsbarriere **D** kann jedes Material genutzt werden, welches unter den Bedingungen des Schrittes (a) des erfindungsgemäßen Verfahrens stabil ist und den Übergang von Protonen von der im Innenraum $I_{KA}$ der Anodenkammer $K_A$ befindlichen Flüssigkeit in den Innenraum $I_{KM}$ der optionalen Mittelkammer $K_M$ verhindert oder verlangsamt.

Als Diffusionsbarriere **D** wird insbesondere eine nicht ionenspezifische Trennwand oder eine für spezifische Ionen durchlässige Membran verwendet. Bevorzugt handelt es sich bei der Diffusionsbarriere **D** um eine nicht ionenspezifische Trennwand.

Das Material der nicht ionenspezifischen Trennwand ist insbesondere aus der Gruppe bestehend aus Gewebe, wobei es sich insbesondere um textiles Gewebe oder Metallgewebe handelt, Glas, wobei es sich insbesondere um gesintertes Glas oder Glasfritten handelt, Keramik, insbesondere keramische Fritten, Membrandiaphragmas ausgewählt, und ist besonders bevorzugt ein textiles Gewebe oder Metallgewebe, besonders bevorzugt ein textiles Gewebe. Das textile Gewebe umfasst bevorzugt Kunststoff, bevorzugter einen Kunststoff ausgewählt aus PVC, PVC-C, Polyvinylether ("PVE"), Polytetrafluorethylen ("PTFE").

Handelt es sich bei der Diffusionsbarriere **D** um eine "für spezifische Ionen durchlässige Membran", so bedeutet dies erfindungsgemäß, dass die jeweilige Membran die Diffusion bestimmter Ionen durch sie hindurch gegenüber anderen Ionen begünstigt. Insbesondere sind damit Membranen gemeint, die die Diffusion durch sie hindurch von Ionen einer bestimmten Ladungsart gegenüber entgegengesetzt geladenen Ionen begünstigt. Noch bevorzugter begünstigen für spezifische Ionen durchlässige Membranen außerdem die Diffusion bestimmter Ionen mit einer Ladungsart gegenüber anderen Ionen derselben Ladungsart durch sie hindurch.

Ist die Diffusionsbarriere **D** eine "für spezifische Ionen durchlässige Membran", handelt es sich insbesondere bei der Diffusionsbarriere **D** um eine anionenleitende Membran oder um eine kationenleitende Membran.

Anionenleitende Membranen sind erfindungsgemäß solche, die selektiv Anionen, bevorzugt selektiv bestimmte Anionen leiten. In anderen Worten begünstigen sie die Diffusion von Anionen durch sie hindurch gegenüber der von Kationen, insbesondere gegenüber Protonen, noch bevorzugter begünstigen sie zusätzlich die Diffusion von bestimmten Anionen durch sie hindurch gegenüber der Diffusion anderer Anionen durch sie hindurch.

Kationenleitende Membranen sind erfindungsgemäß solche, die selektiv Kationen, bevorzugt selektiv bestimmte Kationen leiten. In anderen Worten begünstigen sie die Diffusion von Kationen durch sie hindurch gegenüber der von Anionen, noch bevorzugter begünstigen sie zusätzlich die Diffusion von bestimmten Kationen, durch sie hindurch gegenüber der Diffusion anderer Kationen durch sie hindurch, noch viel mehr bevorzugter von Kationen, bei denen es sich nicht um Protonen handelt, noch bevorzugter um Natriumkationen handelt, gegenüber Protonen.

"Begünstigen die Diffusion bestimmter Ionen X gegenüber der Diffusion anderer Ionen Y" bedeutet insbesondere, dass der Diffusionskoeffizient (Einheit $m^2/s$) der Ionenart X bei einer gegebenen Temperatur für die betreffende Membran um den Faktor 10, bevorzugt 100, bevorzugt 1000 höher ist als der Diffusionskoeffizient der Ionenart Y für die betreffende Membran.

Handelt es sich bei der Diffusionsbarriere **D** um eine "für spezifische Ionen durchlässige Membran", so ist es bevorzugt eine anionenleitende Membran, denn diese verhindert besonders gut die Diffusion von Protonen aus der Anodenkammer $\mathbf{K_A}$ in die Mittelkammer $\mathbf{K_M}$.

Als anionenleitende Membran wird insbesondere eine solche eingesetzt, die für die vom Salz **S** umfassten Anionen selektiv ist. Solche Membranen sind dem Fachmann bekannt und können von ihm eingesetzt werden. Erfindungsgemäß umfasst das Salz **S** $M_A$ als Kation.

Das Salz **S** ist bevorzugt ein Halogenid, Sulfat, Sulfit, Nitrat, Hydrogencarbonat oder Carbonat von $M_A$, noch bevorzugter ein Halogenid.

Halogenide sind Fluoride, Chloride, Bromide, Jodide. Das bevorzugteste Halogenid ist Chlorid.

Bevorzugt wird als anionenleitende Membran eine für Halogenide, bevorzugt Chlorid, selektive Membran eingesetzt.

Anionenleitende Membranen sind beispielsweise von M.A. Hickner, A.M. Herring, E.B. Coughlin, Journal of Polymer Science, Part B: Polymer Physics 2013, 51, 1727-1735, von C.G. Arges, V. Ramani, P.N. Pintauro, Electrochemical Society Interface 2010, 19, 31-35, in WO 2007/048712 A2 sowie auf Seite 181 des Lehrbuchs von Volkmar M. Schmidt Elektrochemische Verfahrenstechnik: Grundlagen, Reaktionstechnik, Prozessoptimierung, 1. Auflage (8. Oktober 2003) beschrieben.

Noch bevorzugter werden demnach als anionenleitende Membran organische Polymere, welche insbesondere aus Polyethylen, Polybenzimidazolen, Polyetherketonen, Polystyrol, Polypropylen oder fluorierten Membranen wie Polyperfluorethylen, bevorzugt Polystyrol, ausgewählt sind, eingesetzt, wobei diese kovalent gebunden funktionelle Gruppen ausgewählt aus $-NH_3^+$, $-NRH_2^+$, $-NR_3^+$, $=NR^+$;$-PR_3^+$, wobei es sich bei R um Alkylgruppen mit bevorzugt 1 bis 20 Kohlenstoffatomen handelt, oder andere kationische Gruppen aufweisen. Bevorzugt weisen sie kovalent gebundene funktionelle Gruppen, ausgewählt aus $-NH_3^+$, $-NRH_2^+$, $-NR_3^+$, bevorzugter ausgewählt aus $-NH_3^+$, $-NR_3^+$, noch bevorzugter $-NR_3^+$, auf.

Wenn die Diffusionsbarriere **D** eine kationenleitende Membran ist, handelt es sich insbesondere um eine Membran, die für $M_A$, also dem vom Salz **S** umfassten Kation, selektiv ist. Noch bevorzugter ist die Diffusionsbarriere **D** eine alkalikationenleitende Membran, noch mehr bevorzugter eine kalium- und/oder natriumionenleitende Membran, am bevorzugtesten eine natriumionenleitende Membran.

Kationenleitende Membranen sind beispielsweise beschrieben auf Seite 181 des Lehrbuchs von Volkmar M. Schmidt Elektrochemische Verfahrenstechnik: Grundlagen, Reaktionstechnik, Prozessoptimierung, 1. Auflage (8. Oktober 2003).

Noch bevorzugter werden demnach als kationenleitende Membran organische Polymere, welche insbesondere aus Polyethylen, Polybenzimidazolen, Polyetherketonen, Polystyrol, Polypropylen oder fluorierten Membranen wie Polyperfluorethylen, bevorzugt Polystyrol, Polyperfluorethylen, ausgewählt sind, eingesetzt, wobei diese kovalent gebunden funktionelle Gruppen ausgewählt aus $-SO_3^-$, $-COO^-$, $-PO_3^{2-}$, $-PO_2H^-$, bevorzugt $-SO_3^-$, (beschrieben in DE 10 2010 062 804 A1, US 4,831,146) tragen.

Dies kann zum Beispiel ein sulfoniertes Polyperfluorethylen (Nafion ® mit CAS-Nummer: 31175-20-9) sein. Diese sind dem Fachmann beispielsweise aus der WO 2008/076327 A1, Absatz [058], US 2010/0044242 A1, Absatz

[0042] oder der US 2016/ 0204459 A1 bekannt und unter dem Handelsnamen Nafion ®, Aciplex ® F, Flemion ®, Neosepta ®, Ultrex ®, PC-SK ® erwerblich. Neosepta®-Membranen sind beispielsweise beschrieben von S.A. Mareev, D.Yu. Butylskii, N.D. Pismenskaya, C. Larchet, L. Dammak, V.V. Nikonenko, Journal of Membrane Science 2018, 563, 768-776.

Wird eine kationenleitende Membran als Diffusionsbarriere **D** eingesetzt, kann dies beispielsweise ein mit Sulfonsäuregruppen funktionalisiertes Polymer, insbesondere der folgenden Formel $\mathbf{P_{NAFION}}$, wobei n und m unabhängig voneinander eine ganze Zahl von 1 bis $10^6$, bevorzugter eine ganze Zahl von 10 bis $10^5$, noch bevorzugter eine ganze Zahl von $10^2$ bis $10^4$ ist, sein.

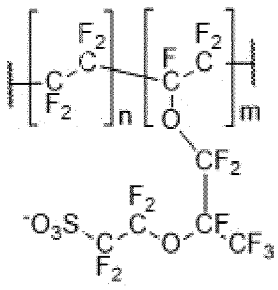

$\mathbf{P_{Nafion}}$

1.1.3.2 Zulauf $\mathbf{Z_{KM}}$ und Ablauf $\mathbf{A_{MK}}$

Die optionale Mittelkammer $\mathbf{K_M}$ umfasst auch einen Zulauf $\mathbf{Z_{KM}}$ und einen Ablauf $\mathbf{A_{KM}}$. Dies ermöglicht es, dem Innenraum $\mathbf{I_{KM}}$ der Mittelkammer $\mathbf{K_M}$ Flüssigkeit, wie zum Beispiel die Lösung $\mathbf{L_3}$, zuzufügen, und darin befindliche Flüssigkeit, wie zum Beispiel die Lösung $\mathbf{L_3}$, in den Innenraum $\mathbf{I_{KA}}$ der Anodenkammer $\mathbf{K_A}$ zu überführen.

Der Zulauf $\mathbf{Z_{KM}}$ und der Ablauf $\mathbf{A_{KM}}$ können nach dem Fachmann bekannten Verfahren an der Elektrolysezelle **E** angebracht werden, z.B. durch Bohrungen in der Außenwand und entsprechenden Anschlüssen (Ventilen), die die Ein- bzw. Ausleitung von Flüssigkeit vereinfachen. Der Ablauf $\mathbf{A_{KM}}$ kann auch innerhalb der Elektrolysezelle liegen, beispielsweise als Perforation in der Diffusionsbarriere **D.**

1.1.3.3 Verbindung $\mathbf{V_{AM}}$

In der in Schritt (a) des erfindungsgemäßen Verfahrens verwendeten Elektrolysezelle **E** ist der Ablauf $\mathbf{A_{KM}}$ durch eine Verbindung $\mathbf{V_{AM}}$ mit dem Zulauf $\mathbf{Z_{KA}}$ so verbunden, dass durch die Verbindung $\mathbf{V_{AM}}$ Flüssigkeit aus $\mathbf{I_{KM}}$ in $\mathbf{I_{KA}}$ geleitet werden kann.

Die Verbindung $\mathbf{V_{AM}}$ kann innerhalb der Elektrolysezelle **E** und/oder außerhalb der Elektrolysezelle **E** ausgebildet sein, und ist bevorzugt innerhalb der Elektrolysezelle ausgebildet.

**1)** Ist die Verbindung $\mathbf{V_{AM}}$ innerhalb der Elektrolysezelle **E** ausgebildet, wird sie bevorzugt durch mindestens eine Perforation in der Diffusionsbarriere **D** gebildet. Diese Ausführungsform ist insbesondere dann bevorzugt, wenn als Diffusionsbarriere **D** eine nicht ionenspezifische Trennwand, insbesondere ein Metallgewebe oder textiles Gewebe eingesetzt wird. Dieses fungiert als Diffusionsbarriere **D** und weist aufgrund der Webeigenschaften von vorneherein Perforationen und Lücken auf, die als Verbindung $\mathbf{V_{AM}}$ fungieren.

**2)** Die folgend beschriebene Ausführungsform ist insbesondere dann bevorzugt, wenn als Diffusionsbarriere **D** eine für spezifische Ionen durchlässige Membran eingesetzt wird: In dieser Ausführungsform ist die Verbindung $\mathbf{V_{AM}}$ außerhalb der Elektrolysezelle **E** ausgebildet, wobei sie bevorzugt durch eine außerhalb der Elektrolysezelle **E** verlaufende Verbindung von $\mathbf{A_{KM}}$ und $\mathbf{Z_{KA}}$ gebildet wird, insbesondere dadurch, dass vom Innenraum $\mathbf{I_{KM}}$ der Mittelkammer $\mathbf{K_M}$ ein Ablauf $\mathbf{A_{KM}}$ durch die Außenwand $\mathbf{W_A}$, bevorzugt am Boden der Mittelkammer $\mathbf{K_M}$, wobei noch bevorzugter der Zulauf $\mathbf{Z_{KM}}$ an der Oberseite der Mittelkammer $\mathbf{K_M}$ ist, gebildet wird, und ein Zulauf $\mathbf{Z_{KA}}$ in den Innenraum $\mathbf{I_{KA}}$ der Anodenkammer $\mathbf{K_A}$ durch die Außenwand $\mathbf{W_A}$, bevorzugt am Boden der Anodenkammer $\mathbf{K_A}$, gebildet wird, und diese durch eine Leitung, beispielsweise ein Rohr oder ein Schlauch, der bevorzugt ein Material ausgewählt aus Gummi, Kunststoff umfasst, verbunden sind. Der Ablauf $\mathbf{A_{KA}}$ ist dann noch bevorzugter an der Oberseite der Anodenkammer $\mathbf{K_A}$ ausgebildet.

"Ablauf $\mathbf{A_{KM}}$ am Boden der Mittelkammer $\mathbf{K_M}$**"** bedeutet, dass der Ablauf $\mathbf{A_{KM}}$ so an der Elektrolysezelle **E**

angebracht ist, dass die Lösung $\mathbf{L_3}$ die Mittelkammer $\mathbf{K_M}$ gleichgerichtet mit der Schwerkraft verlässt.

"Zulauf $\mathbf{Z_{KA}}$ am Boden der Anodenkammer $\mathbf{K_A}$" bedeutet, dass der Zulauf $\mathbf{Z_{KA}}$ so an der Elektrolysezelle **E** angebracht ist, dass die Lösung $\mathbf{L_3}$ in die Anodenkammer $\mathbf{K_A}$ entgegen der Schwerkraft eintritt.

"Zulauf $\mathbf{Z_{KM}}$ an der Oberseite der Mittelkammer $\mathbf{K_M}$" bedeutet, dass der Zulauf $\mathbf{Z_{KM}}$ so an der Elektrolysezelle **E** angebracht ist, dass die Lösung $\mathbf{L_3}$ in die Mittelkammer $K_M$ gleichgerichtet mit der Schwerkraft eintritt.

"Ablauf $\mathbf{A_{KA}}$ an der Oberseite der Anodenkammer $\mathbf{K_A}$" bedeutet, dass der Ablauf $\mathbf{A_{KA}}$ so an der Elektrolysezelle **E** angebracht ist, dass die Lösung $\mathbf{L_4}$ die Anodenkammer $\mathbf{K_A}$ entgegen der Schwerkraft verlässt.

Diese Ausführungsform ist dabei besonders vorteilhaft und deshalb bevorzugt, wenn der Ablauf $\mathbf{A_{KM}}$ durch die Außenwand $\mathbf{W_A}$ am Boden der Mittelkammer $\mathbf{K_M}$, und der Zulauf $\mathbf{Z_{KA}}$ durch die Außenwand $\mathbf{W_A}$ am Boden der Anodenkammer $\mathbf{K_A}$, gebildet wird. Durch diese Anordnung ist es besonders einfach möglich, in der Anodenkammer $\mathbf{K_A}$ gebildete Gase mit $\mathbf{L_4}$ aus der Anodenkammer $\mathbf{K_A}$ abzuleiten, um diese dann weiter abzutrennen. Fig. 1 B zeigt eine solche Ausführungsform.

Wenn die Verbindung $\mathbf{V_{AM}}$ außerhalb der Elektrolysezelle **E** ausgebildet ist, sind insbesondere $\mathbf{Z_{KM}}$ und $\mathbf{A_{KM}}$ an gegenüberliegenden Seiten der Außenwand $\mathbf{W_A}$ der Mittelkammer $\mathbf{K_M}$ angeordnet (also z.B. $\mathbf{Z_{KM}}$ am Boden und $\mathbf{A_{KM}}$ an der Oberseite der Elektrolysezelle **E** oder umgekehrt) und $\mathbf{Z_{KA}}$ und $\mathbf{A_{KA}}$ an gegenüberliegenden Seiten der Außenwand $\mathbf{W_A}$ der Anodenkammer $\mathbf{K_A}$ angeordnet (also $\mathbf{Z_{KA}}$ am Boden und $\mathbf{A_{KA}}$ an der Oberseite der Elektrolysezelle **E** oder umgekehrt), wie es insbesondere in Abbildung 1 B gezeigt ist. Durch diese Geometrie muss $\mathbf{L_3}$ die beiden Kammern $\mathbf{K_M}$ und $\mathbf{K_A}$ durchströmen. Dabei können $\mathbf{Z_{KA}}$ und $\mathbf{Z_{KM}}$ an derselben Seite der Elektrolysezelle **E** ausgebildet sein, wobei dann automatisch auch $\mathbf{A_{KM}}$ und $\mathbf{A_{KA}}$ an derselben Seite der Elektrolysezelle **E** ausgebildet sind. Alternativ können $\mathbf{Z_{KA}}$ und $\mathbf{Z_{KM}}$, wie bei der in Abbildung 1 B gezeigten Ausführungsform, an gegenüberliegenden Seiten der Elektrolysezelle **E** ausgebildet sein, wobei dann automatisch auch $\mathbf{A_{KM}}$ und $\mathbf{A_{KA}}$ an gegenüberliegenden Seiten der Elektrolysezelle **E** ausgebildet sind.

3) Wenn die Verbindung $\mathbf{V_{AM}}$ innerhalb der Elektrolysezelle **E** ausgebildet ist, kann dies insbesondere dadurch gewährleistet werden, dass eine Seite ("Seite A") der Elektrolysezelle **E,** bei der es sich um die Oberseite oder den Boden der Elektrolysezelle **E** handelt, bevorzugt wie in Abbildung 6 B gezeigt um die Oberseite handelt, den Zulauf $\mathbf{Z_{KM}}$ und den Ablauf $\mathbf{A_{KA}}$ umfasst und die Diffusionsbarriere **D** ausgehend von dieser Seite ("Seite A") sich in die Elektrolysezelle **E** erstreckt, aber nicht ganz bis zur der der Seite A gegenüberliegenden Seite ("Seite B") der Elektrolysezelle **E,** bei der es dann sich um den Boden bzw. die Oberseite der Elektrolysezelle **E** handelt, reicht und dabei 50 % oder mehr der Höhe der Dreikammerzelle **E,** bevorzugter 60 % bis 99 % der Höhe der Dreikammerzelle **E,** noch bevorzugter 70 % bis 95 % der Höhe der Dreikammerzelle **E,** noch mehr bevorzugter 80 % bis 90 % der Höhe der Dreikammerzelle **E,** noch viel mehr bevorzugter 85 % der Höhe der Dreikammerzelle **E** durchspannt. Dadurch dass die Diffusionsbarriere **D** die Seite B der Dreikammerzelle **E** nicht berührt, entsteht so ein Spalt zwischen Diffusionsbarriere **D** und der Außenwand $\mathbf{W_A}$ der Seite B der Dreikammerzelle **E.** Der Spalt ist dann die Verbindung $\mathbf{V_{AM}}$. Durch diese Geometrie muss $\mathbf{L_3}$ die beiden Kammern $\mathbf{K_M}$ und $\mathbf{K_A}$ vollständig durchströmen.

Diese Ausführungsformen gewährleisten am besten, dass am säureempfindlichen Festelektrolyten die wässrige Salzlösung $\mathbf{L_3}$ vorbeiströmt, bevor diese mit der anodischen Elektrode $\mathbf{E_A}$ in Kontakt kommt, wodurch es zur Bildung von Säuren kommt.

"Boden der Elektrolysezelle **E**" ist erfindungsgemäß die Seite der Elektrolysezelle **E,** durch die eine Lösung (z.B. $\mathbf{L_3}$ bei $\mathbf{A_{KM}}$ in Abbildung 1 B) gleichgerichtet mit der Schwerkraft aus der Elektrolysezelle **E** austritt bzw. die Seite der Elektrolysezelle **E,** durch die eine Lösung (z.B. $\mathbf{L_2}$ bei $\mathbf{Z_{KK}}$ in Abbildungen 1 A, 1 B, 6 A und 6 B und $\mathbf{L_3}$ bei $\mathbf{Z_{KA}}$ in Abbildungen 1 A und 1 B) der Elektrolysezelle **E** entgegen der Schwerkraft zugeführt wird.

"Oberseite der Elektrolysezelle **E**" ist erfindungsgemäß die Seite der Elektrolysezelle **E,** durch die eine Lösung (z.B. $\mathbf{L_4}$ bei $\mathbf{A_{KA}}$ und $\mathbf{L_1}$ bei $\mathbf{A_{KK}}$ in Abbildungen 1 A, 1 B, 6 A und 6 B) entgegen der Schwerkraft aus der Elektrolysezelle **E** austritt bzw. die Seite der Elektrolysezelle **E,** durch die eine Lösung (z.B. $\mathbf{L_3}$ bei $\mathbf{Z_{KM}}$ in Abbildungen 1 B, 6 A und 6 B) der Elektrolysezelle **E** gleichgerichtet mit der Schwerkraft zugeführt wird.

1.1.3.4 Weitere Ausführungsformen der Mittelkammer $\mathbf{K_M}$

In einer bevorzugten Ausführungsform der Elektrolysezelle **E** umfasst der Innenraum $\mathbf{I_{KM}}$ auch mindestens ein zusätzliches Merkmal ausgewählt aus:

1) Einbauten, die so eingerichtet sind, dass sie im Elektrolyten $\mathbf{L_3}$ zu Turbulenzen führen;
2) einer Rührvorrichtung;
3) einer zusätzlichen Einleitung eines Inertgases (z.B. Stickstoff oder Edelgas), durch einen zusätzlichen Zulauf an der Unterseite der Mittelkammer und einen zusätzlichen Ablauf an der Oberseite der Mittelkammer. Über diesen zusätzlichen Ablauf können auch eventuell entstehende Gase wie z.B. $CO_2$, wenn es sich beim Salz **S** um ein Carbonat oder Hydrogencarbonat handelt, aus $\mathbf{I_{KM}}$ abgeleitet werden.

Durch diese zusätzlichen bevorzugten Ausführungsformen **1), 2)** und **3)** bilden sich im Elektrolyten $\mathbf{L_3}$ beim Durchströmen der $\mathbf{I_{KM}}$ Verwirbelungen und Turbulenzen aus. Dadurch wird in der Mittelkammer die Ausbildung eines pH-Gradienten zusätzlich behindert und so die Schädigung der AFK durch einen zu niedrigen pH unterbunden. Dies erhöht die Lebensdauer der AFK.

*1.1.4 Trennwand **W***

Die in Schritt (a) des erfindungsgemäßen Verfahrens eingesetzte Elektrolysezelle **E** umfasst eine Trennwand **W.** Die Trennwand **W** umfasst mindestens eine alkalikationenleitende Festelektrolytkeramik $\mathbf{F_A}$. In einer bevorzugten Ausführungsform besteht die Trennwand **W aus** einer alkalikationenleitende Festelektrolytkeramik $\mathbf{F_A}$.

Die Trennwand **W** umfasst in einer alternativen bevorzugten Ausführungsform der vorliegenden Erfindung mindestens zwei, optional durch ein Trennelement **T** voneinander getrennte, alkalikationenleitende Festelektrolytkeramiken ("alkalikationenleitende Festelektrolytkeramik" wird im Folgenden durch "AFK" abgekürzt) $\mathbf{F_A}$ und $\mathbf{F_B}$.

Die Trennwand **W** weist zwei Seiten $\mathbf{S_{KK}}$ und $\mathbf{S_{A/MK}}$, die einander gegenüberliegen, auf, das heißt, die Seite $\mathbf{S_{A/MK}}$ liegt der Seite $\mathbf{S_{KK}}$ gegenüber (und umgekehrt). Die beiden Seiten $\mathbf{S_{KK}}$ und $\mathbf{s_{A/MK}}$ umfassen insbesondere im Wesentlichen zueinander parallele Ebenen.

Die Geometrie der Trennwand **W** ist ansonsten nicht weiter beschränkt und kann insbesondere an den Querschnitt der Elektrolysezelle **E** angepasst werden, in der sie zum Einsatz kommt. Beispielsweise kann sie die Geometrie eines Quaders aufweisen und somit einen rechteckigen Durchschnitt aufweisen, oder die Geometrie eines stumpfen Kegels oder Zylinders und demnach einen kreisförmigen Durchschnitt aufweisen.

Optional kann die Trennwand **W** auch die Geometrie eines Quaders mit abgerundeten Ecken und/oder Auswölbungen aufweisen, die wiederum Löcher aufweisen können. Die Trennwand **W** hat dann Auswölbungen ("Hasenohren"), mit denen die Trennwand **W** an Elektrolysezellen fixiert werden kann oder auch Rahmenteile der Trennwand **W** aneinander fixiert werden können.

Die Seite $\mathbf{S_{KK}}$ der Trennwand **W** weist die Oberfläche $\mathbf{O_{KK}}$ auf, und die Seite $\mathbf{S_{A/MK}}$ der Trennwand **W** weist die Oberfläche $\mathbf{O_{A/MK}}$ auf.

Das Merkmal "Trennwand" bedeutet, dass die Trennwand **W** flüssigkeitsdicht ist. Somit existieren keine Lücken, durch welche wässrige Lösung, alkoholische Lösung, Alkohol oder Wasser von der Seite $\mathbf{S_{KK}}$ auf die Seite $\mathbf{S_{A/MK}}$ oder umgekehrt fließen könnte. Dies bedeutet in den Fällen, in denen die Trennwand **W** mindestens zwei alkalikationenleitende Festelektrolytkeramiken $\mathbf{F_A}$ und $\mathbf{F_B}$ und gegebenenfalls ein Trennelement **T** umfasst, dass $\mathbf{F_A}$ und $\mathbf{F_B}$ und das gegebenenfalls vorhandene mindestens eine Trennelement **T** lückenlos aneinander anschließen.

Die in der Elektrolysezelle **E** gemäß Schritt (a) des erfindungsgemäßen Verfahrens einsetzbare Trennwand **W** umfasst auch Ausführungsformen, in denen die Trennwand **W** mehr als zwei AFKs, z.B. vier oder neun oder zwölf AFKs, umfasst, wobei die AFKs untereinander entweder direkt aneinandergrenzen oder durch ein Trennelement **T** voneinander getrennt sind.

Wenn die AFKs untereinander direkt aneinandergrenzen, erfordert dies jedoch eine genaue Passung der jeweils angrenzenden AFKs, um das Entstehen einer Lücke zwischen ihnen, durch welche wässrige Flüssigkeit oder Wasser oder Glykol oder glykolische Lösung von der Seite $\mathbf{S_{KK}}$ auf die Seite $\mathbf{s_{A/MK}}$ fließen könnte, auszuschließen. Es ist deshalb vorteilhaft und bevorzugt, dass, wenn die Trennwand **W** mehr als eine AFK umfasst, in der Trennwand **W** alle von der Trennwand **W** umfassten AFKs durch mindestens ein Trennelement **T** voneinander getrennt sind, das heißt kein AFK direkt, also ohne dass ein Trennelement **T** dazwischen wäre, an ein anderes AFK anschließt.

Die Trennwand **W** ist weiterhin dadurch gekennzeichnet, dass die von der Trennwand **W** umfasste AFK $\mathbf{F_A}$ sowohl über die Oberfläche $\mathbf{O_{KK}}$ als auch über die Oberfläche $\mathbf{O_{A/MK}}$ direkt kontaktierbar ist.

In der Ausführungsform, in der die Trennwand **W** mindestens zwei AFKs $\mathbf{F_A}$, $\mathbf{F_B}$ umfasst, ist es bevorzugt, dass alle von der Trennwand **W** umfassten AFKs sowohl über die Oberfläche $\mathbf{O_{KK}}$ als auch über die Oberfläche $\mathbf{O_{A/MK}}$ direkt kontaktierbar sind.

"Direkt kontaktierbar" bedeutet mit Bezug auf die von der Trennwand **W** umfassten AFKs, dass mindestens ein Teil der Oberflächen $\mathbf{O_{KK}}$ und $\mathbf{O_{A/MK}}$ durch die Oberfläche der von der Trennwand **W** umfassten AFKs gebildet wird, das heißt, dass die von der Trennwand **W** umfassten AFKs an den beiden Oberflächen $\mathbf{O_{KK}}$ und $\mathbf{O_{A/MK}}$ unmittelbar zugänglich sind, so dass sie an den beiden Oberflächen $\mathbf{O_{KK}}$ und $\mathbf{O_{A/MK}}$ zum Beispiel mit wässriger Lösung, glykolischer Lösung, Glykol oder Wasser benetzt werden können.

Für die Anordnung der AFKs in der Trennwand **W** bedeutet dies, dass es für jede der von der Trennwand **W** umfassten AFKs einen Weg von der Oberfläche $\mathbf{O_{KK}}$ auf der Seite $\mathbf{S_{KK}}$ auf die Oberfläche $\mathbf{O_{A/MK}}$ auf der Seite $\mathbf{S_{A/MK}}$ gibt, der vollständig durch die jeweilige AFK führt.

Wenn die Trennwand **W** mindestens ein Trennelement **T** aufweist, ist typischerweise auch das mindestens eine Trennelement **T** sowohl über mindestens einen Teil der Oberfläche $\mathbf{O_{KK}}$ als auch über mindestens einen Teil der Oberfläche $\mathbf{O_{A/MK}}$ direkt kontaktierbar.

"Direkt kontaktierbar" bedeutet mit Bezug auf das von der Trennwand **W** optional umfasste mindestens eine

Trennelement **T,** dass ein Teil der Oberflächen $O_{KK}$ und $O_{A/MK}$ durch die

Oberfläche des Trennelements **T** gebildet wird, das heißt dass das Trennelement **T** an den beiden Oberflächen $O_{KK}$ und $O_{A/MK}$ unmittelbar zugänglich ist, so dass das Trennelement **T** an den beiden Oberflächen $O_{KK}$ und $O_{A/MK}$ zum Beispiel mit wässriger Lösung, alkoholischer Lösung, Alkohol oder Wasser benetzt werden kann.

Für die Anordnung des optionalen Trennelements **T** in der Trennwand **W** bedeutet dies insbesondere, dass es für das von der Trennwand **W** optional umfasste Trennelement **T** einen Weg von der Oberfläche $O_{KK}$ auf der Seite $S_{KK}$ zur Oberfläche $O_{A/MK}$ auf der Seite $S_{A/MK}$ gibt, der durch das Trennelement **T,** und gegebenenfalls durch eine Dichtung **Di,** aber nicht durch eine AFK, führt.

In einer bevorzugten Ausführungsform der Trennwand **W** werden mindestens 50 %, bevorzugter mindestens 60 %, noch bevorzugter mindestens 70 %, noch bevorzugter mindestens 85 % der Oberfläche $O_{A/MK}$ durch die von der Trennwand **W** umfassten AFKs gebildet.

In einer bevorzugten Ausführungsform der Trennwand **W** werden mindestens 50 %, bevorzugter mindestens 60 %, noch bevorzugter mindestens 70 %, noch bevorzugter mindestens 85 % der Oberfläche $O_{KK}$ durch die von der Trennwand **W** umfassten AFKs gebildet.

In der Ausführungsform, in der die Trennwand **W** mehr als ein AFK aufweist, werden insbesondere 50 % bis 99 %, bevorzugter mindestens 60 % bis 96 %, noch bevorzugter 70 % bis 92 %, noch bevorzugter 85 % bis 90 % der Oberfläche $O_{KK}$ durch die von der Trennwand **W** umfassten AFKs gebildet wobei noch bevorzugter der Rest der Oberfläche $O_{KK}$ durch das Trennelement **T** und gegebenenfalls das Rahmenelement **R** gebildet werden. Gleichzeitig werden in der Ausführungsform, in der die Trennwand **W** mehr als ein AFK aufweist, insbesondere 50 % bis 99 %, bevorzugter mindestens 60 % bis 96 %, noch bevorzugter 70 % bis 92 %, noch bevorzugter 85 % bis 90 % der Oberfläche $O_{A/MK}$ durch die von der Trennwand **W** umfassten AFKs gebildet wobei noch bevorzugter der Rest der Oberfläche $O_{A/MK}$ durch das Trennelement **T** und gegebenenfalls das Rahmenelement **R** gebildet werden.

In der bevorzugten Ausführungsform umfasst die Trennwand **W** <16> eine alkalikationenleitende Festelektrolytkeramik $F_A$ und gegebenenfalls ein Rahmenelement **R.** Noch bevorzugter besteht die die Trennwand **W** <16> aus einer alkalikationenleitende Festelektrolytkeramik $F_A$.

In einer anderen bevorzugten Ausführungsform umfasst die Trennwand **W** mindestens vier AFKs $F_A$, $F_B$, $F_C$ und $F_D$, wobei sie dann noch bevorzugter genau vier AFKs $F_A$, $F_B$, $F_B$ und $F_D$ umfasst.

In einer weiteren bevorzugten Ausführungsform umfasst die Trennwand **W** mindestens neun AFKs $F_A$, $F_B$, $F_C$, $F_D$, $F_E$, $F_F$, $F_G$, $F_H$ und $F_I$ wobei sie dann noch bevorzugter genau neun AFKs $F_A$, $F_B$, $F_C$, $F_D$, $F_E$, $F_F$, $F_G$, $F_H$ und $F_I$ umfasst.

In einer weiteren bevorzugten Ausführungsform umfasst die Trennwand **W** mindestens zwölf AFKs $F_A$, $F_B$, $F_C$, $F_D$, $F_E$, $F_F$, $F_G$, $F_H$, $F_I$, $F_J$, $F_K$ und $F_L$, wobei sie dann noch bevorzugter genau zwölf AFKs $F_A$, $F_B$, $F_C$, $F_D$, $F_E$, $F_F$, $F_G$, $F_H$, $F_I$, $F_J$, $F_K$ und $F_L$ umfasst.

Durch die Anordnung von mindestens zwei AFKs nebeneinander in der Trennwand **W** ergibt sich gegenüber der Anordnung nur einer AFK ein Vorteil, nämlich eine weitere Ausbreitungsrichtung für die AFKs bei den Temperaturschwankungen, die sich bei dem Betrieb der Elektrolysezelle ergeben. NaSICON-Scheiben, die als Trennwände fungieren, sind in Elektrolysezellen durch die Außenwände der Elektrolysezelle oder von massiven Kunststoffrahmen eingegrenzt. Die bei der Ausdehnung auftretenden mechanischen Spannungen innerhalb der NaSICON können damit nicht abgeleitet werden, was zum Bruch der Keramik führen kann.

Demgegenüber grenzen die einzelnen AFKs innerhalb der Trennwand **W** bevorzugt an das Trennelement **T,** was zu zwei vorteilhaften Effekten führt, die beide die Langzeitstabilität der AFK erhöhen:

- jedem AFK steht mindestens ein weiterer Freiheitsgrad zur Verfügung, das heißt eine Dimension, in die es sich ausdehnen kann. Neben der Ausdehnung in z-Richtung (= das heißt über die Dicke der Keramikscheibe im rechten Winkel zur Ebene der Trennwand **W**) ist nun auch eine Ausdehnung in x- und/oder y-Richtung möglich, d.h. in waagerechter und senkrechter Richtung innerhalb der Ebene der Trennwand **W.** Diese Ausdehnungsrichtung ist nicht gegeben oder zumindest stark eingeschränkt, wenn die AFKs zum Beispiel als massive Scheibe den Querschnitt der Elektrolysezelle durchspannen und an die massive Wand der Elektrolysezelle grenzen;

- gegenüber einer gleich großen Trennwand, die nur aus einem AFK besteht, resultiert die Aufteilung in mehrere kleine AFKs darin, dass die Spannungen, die innerhalb der kleineren AFKs auftreten, auch absolut kleiner sind, schneller abgeführt werden können und sich dadurch nicht so schnell zu einer Spannung aufbauen können, die zum Bruch der AFK führt.

Dadurch ist die Tendenz zu brechen, für die "aufgeteilten" AFKs in der Trennwand **W** gegenüber dem Einsatz einer Scheibe deutlich reduziert.

1.1.4.1 Alkalikationenleitender Festelektrolytkeramik "AFK"

Als von der Trennwand **W** umfassten alkalikationenleitende Festelektrolytkeramiken $\mathbf{F_A}$, $\mathbf{F_B}$ etc. kommt jeder Festelektrolyt in Frage, durch welchen Kationen, insbesondere Alkalikationen, noch bevorzugter Natriumkationen, von der Seite $\mathbf{S_{A/MK}}$ auf die Seite $\mathbf{S_{KK}}$ transportiert werden können. Solche Festelektrolyten sind dem Fachmann bekannt und beispielsweise in der DE 10 2015 013 155 A1, in der WO 2012/048032 A2, Absätze [0035], [0039], [0040], in der US 2010/0044242 A1, Absätze [0040], [0041], in der DE 10360758 A1, Absätze [014] bis [025] beschrieben. Sie werden kommerziell unter dem Namen NaSICON, LiSICON, KSICON vertrieben. Ein natriumionenleitender Festelektrolyt ist bevorzugt, wobei dieser noch bevorzugter eine NaSICON-Struktur aufweist. Erfindungsgemäß einsetzbare NaSICON-Strukturen sind außerdem beispielsweise beschrieben von N. Anantharamulu, K. Koteswara Rao, G. Rambabu, B. Vijaya Kumar, Velchuri Radha, M. Vithal, J Mater Sci 2011, 46, 2821-2837.

In einer bevorzugten Ausführungsform der Trennwand W weisen die von der Trennwand **W** umfassten alkalikationenleitende Festelektrolytkeramiken, und insbesondere die AFK $\mathbf{F_A}$, unabhängig voneinander eine NaSICON-Struktur der Formel $M^{I}_{1+2w+x-y+z}\ M^{II}_{w}\ M^{III}_{x}\ Zr^{IV}_{2-w-x-y}\ M^{V}_{y}\ (SiO_4)_z\ (PO_4)_{3-z}$ auf.

$M^{I}$ ist dabei ausgewählt aus $Na^+$, $Li^+$, bevorzugt $Na^+$.

$M^{II}$ ist dabei ein zweiwertiges Metallkation, bevorzugt ausgewählt aus $Mg^{2+}$, $Ca^{2+}$, $Sr^{2+}$, $Ba^{2+}$, $Co^{2+}$, $Ni^{2+}$, bevorzugter ausgewählt aus $Co^{2+}$, $Ni^{2+}$.

$M^{III}$ ist dabei ein dreiwertiges Metallkation, bevorzugt ausgewählt aus $Al^{3+}$, $Ga^{3+}$, $Sc^{3+}$, $La^{3+}$, $Y^{3+}$, $Gd^{3+}$, $Sm^{3+}$, $Lu^{3+}$, $Fe^{3+}$, $Cr^{3+}$, bevorzugter ausgewählt aus $Sc^{3+}$, $La^{3+}$, $Y^{3+}$, $Gd^{3+}$, $Sm^{3+}$, besonders bevorzugt ausgewählt aus $Sc^{3+}$, $Y^{3+}$, $La^{3+}$.

$M^{V}$ ist dabei ein fünfwertiges Metallkation, bevorzugt ausgewählt aus $V^{5+}$, $Nb^{5+}$, $Ta^{5+}$.

Die römischen Indizes I, II, III, IV, V geben die Oxidationszahlen an, in der die jeweiligen Metallkationen vorliegen.

w, x, y, z sind reelle Zahlen, wobei gilt, dass $0 \leq x < 2$, $0 \leq y < 2$, $0 \leq w < 2$, $0 \leq z < 3$, und wobei w, x, y, z so gewählt werden, dass gilt $1 + 2w + x - y + z \geq 0$ und $2 - w - x - y \geq 0$.

Die NaSICON-Struktur hat dabei erfindungsgemäß noch bevorzugter eine Struktur der Formel $Na_{(1+v)}Zr_2Si_vP_{(3-v)}O_{12}$, wobei v eine reelle Zahl ist, für die $0 \leq v \leq 3$ gilt. Am bevorzugtesten gilt v = 2.4

In einer bevorzugten Ausführungsform der Trennwand **W,** in der diese mindestens zwei AFKs $\mathbf{F_A}$, $\mathbf{F_B}$ umfasst, weisen alle von der Trennwand **W** umfassten AFKs die gleiche Struktur auf.

1.1.4.2 Trennelement **T**

In den Ausführungsformen der erfindungsgemäßen Trennwand **W,** in denen diese mindestens zwei AFKs $\mathbf{F_A}$, $\mathbf{F_B}$ umfasst, umfasst die Trennwand **W** bevorzugt ein Trennelement **T.** Das Trennelement **T** trennt dann erfindungsgemäß mindestens zwei von der Trennwand **W** umfasste alkalikationenleitende Festelektrolytkeramiken $\mathbf{F_A}$ und $\mathbf{F_B}$, das heißt es ist zwischen mindestens zwei von der Trennwand **W** umfassten alkalikationenleitende Festelektrolytkeramiken $\mathbf{F_A}$ und $\mathbf{F_B}$ angeordnet.

Als Trennelement **T,** welches bevorzugt von der Trennwand **W** umfasst ist, eignet sich jeder Körper, durch den die jeweiligen AFKs voneinander getrennt angeordnet werden können. Die AFKs schließen dabei lückenlos an das Trennelement **T** an, um die Funktion der Trennwand nicht zu beeinträchtigen, die in der Elektrolysezelle **E** die Kathodenkammer flüssigkeitsdicht von der benachbarten Mittel- bzw. Anodenkammer abtrennen soll.

Die Form des Trennelements **T** kann vom Fachmann in Abhängigkeit von der Anzahl der AFKs, die die Trennwand **W** in der bevorzugten Ausführungsform umfasst, gewählt werden.

Umfasst die Trennwand **W** beispielsweise zwei oder drei AFKs, können diese jeweils durch ein zwischen den AFKs angeordneter Steg als Trennelement **T** getrennt werden.

Umfasst die Trennwand **W** vier oder mehr AFKs, können diese durch ein Trennelement **T,** welches die Form eines Kreuzes oder Gitters hat, getrennt werden.

In den Ausführungsformen der erfindungsgemäßen Trennwand **W,** in denen diese mindestens zwei AFKs $\mathbf{F_A}$, $\mathbf{F_B}$ umfasst, ist es besonders bevorzugt, dass die Trennwand **W** mindestens vier AFKs umfasst und noch bevorzugter, dass das Trennelement **T** dann kreuzförmig oder gitterförmig ist, da dann gewährleistet ist, dass den AFKs alle drei Dimensionen vollständig für die thermische Ausdehnung/Schrumpfung zur Verfügung stehen.

Das Trennelement **T** kann dabei aus einem Stück bestehen. Dann wird der AFK zum Beispiel mit einem dem Fachmann bekannten Mittel lückenlos an das Trennelement **T** befestigt, zum Beispiel über einen Klebstoff, wobei bevorzugt Epoxidharze, Phenolharze genutzt werden Alternativ oder zusätzlich kann das Trennelement **T** auch so ausgeformt sein, dass das jeweilige AFK in das Trennelement eingepasst oder eingeklemmt werden kann. Dies kann schon bei der Herstellung der Trennwand **W** entsprechend durchgeführt werden.

In einer bevorzugten Ausführungsform, in denen die Trennwand **W** ein Trennelement umfasst, umfasst diese insbesondere zwischen Trennelement **T** und den AFKs, eine Dichtung **Di** (Abbildungen 3 B, 3 C). Dadurch wird besonders gut gewährleistet, dass die Trennwand **W** flüssigkeitsdicht ist. Die Dichtung **Di** kann vom Fachmann für die jeweilige AFK

bzw. das jeweilige Trennelement **T** ausgewählt werden.

Die Dichtung **Di** umfasst insbesondere ein Material, welches aus der Gruppe bestehend aus Elastomeren, Klebstoffe, bevorzugt Elastomeren, ausgewählt ist.

Als Elastomer kommt insbesondere Kautschuk in Betracht, bevorzugt Ethylen-Propylen-DienKautschuk ("EPDM"), Fluor-Polymer-Kautschuk ("FPM"), Perfluorpolymerkautschuk ("FFPM"), Acrylnitrilbutadienkautschuk ("NBR").

In einer weiteren bevorzugten Ausführungsform umfasst das Trennelement **T** mindestens zwei Teile $\mathbf{T_1}$ und $\mathbf{T_2}$, die aneinander befestigt werden können und so die AFKs zwischen sich einklemmen.

In dieser Ausführungsform ist es besonders bevorzugt, dann zwischen Trennelement **T** und AFK noch eine Dichtung **Di** anzubringen, um die Flüssigkeitsdichte zu gewährleisten.

Das Trennelement **T** umfasst bevorzugt ein Material, welches aus der Gruppe bestehend aus Kunststoff, Glas, Holz ausgewählt ist. Besonders bevorzugt ist das Trennelement **T** aus Kunststoff. Noch bevorzugter handelt es sich bei dem Kunststoff um einen, der aus der Gruppe bestehend aus Polypropylen, Polystyren, Polyvinylchlorid, nachchloriertes Polyvinylchlorid ("PVC-C") ausgewählt ist.

### 1.1.4.3 Rahmenelement **R**

In einer weiteren bevorzugten Ausführungsform umfasst die Trennwand **W** auch ein Rahmenelement **R.** Das Rahmenelement **R** unterscheidet sich dadurch vom Trennelement **T,** dass es nicht zwischen den von der Trennwand **W** umfassten alkalikationenleitenden Festelektrolytkeramiken angeordnet ist, diese also nicht voneinander trennt. Das Rahmenelement **R** begrenzt insbesondere die Oberflächen $\mathbf{O_{KK}}$ und $\mathbf{O_{A/MK}}$ mindestens teilweise, bevorzugt vollständig. Das bedeutet insbesondere: Das Rahmenelement **R** umschließt die Oberflächen $\mathbf{O}_{KK}$ und $\mathbf{O_{A/MK}}$ mindestens teilweise, bevorzugt vollständig.

Das Rahmenelement **R** kann dabei als ein Teil der Oberflächen $\mathbf{O_{KK}}$ und $\mathbf{O_{A/MK}}$ ausgebildet sein oder nicht. Bevorzugt ist das Rahmenelement **R** als ein Teil der Oberflächen $\mathbf{O}_{KK}$ und $\mathbf{O_{A/MK}}$ ausgebildet.

Das Rahmenelement **R** ist insbesondere über die Oberflächen $\mathbf{O_{KK}}$ und $\mathbf{O_{A/MK}}$ direkt kontaktierbar oder nicht direkt kontaktierbar, bevorzugt direkt kontaktierbar.

"Nicht direkt kontaktierbar" bedeutet mit Bezug auf das von der Trennwand **W** optional umfasste Rahmenelement **R,** dass das Rahmenelement **R** ausschließlich als mindestens ein Teil der Oberflächen jener Seiten der Trennwand **W** ausgebildet ist, bei denen es sich nicht um die Seiten $\mathbf{S_{KK}}$ und $\mathbf{S_{A/MK}}$ handelt. Insbesondere bildet das Rahmenelement **R** dann mindestens 1 %, bevorzugter mindestens 25 %, bevorzugter mindestens 50 %, noch bevorzugter 100 % der Oberflächen der Seiten der Trennwand **W aus,** bei denen es sich nicht um die Seiten $\mathbf{S_{KK}}$ und $\mathbf{S_{A/MK}}$ handelt.

"Direkt kontaktierbar" bedeutet mit Bezug auf das von der Trennwand **W** optional umfasste Rahmenelement **R,** dass ein Teil der Oberflächen $\mathbf{O_{KK}}$ und $\mathbf{O_{A/MK}}$ durch die Oberfläche des Rahmenelements **R** gebildet wird, das heißt, dass das von der Trennwand **W** umfasste Rahmenelement **R** an den beiden Oberflächen $\mathbf{O_{KK}}$ und $\mathbf{O_{A/MK}}$ unmittelbar zugänglich sind, so dass es an den beiden Oberflächen $\mathbf{O_{KK}}$ und $\mathbf{O_{A/MK}}$ zum Beispiel mit wässriger Lösung, alkoholischer Lösung, Alkohol oder Wasser benetzt werden kann.

Für die Anordnung des Rahmenelements **R** in der Trennwand **W** bedeutet dies, dass es dann einen Weg von der Oberfläche $\mathbf{O_{KK}}$ auf der Seite $\mathbf{S_{KK}}$ auf die Oberfläche $\mathbf{O_{A/MK}}$ auf der Seite $\mathbf{S_{A/MK}}$ gibt, der vollständig durch das Rahmenelement **R** führt.

Dies schließt die folgenden Ausführungsformen ein:

- ein Teil des Randes der Oberflächen $\mathbf{O_{KK}}$ und $\mathbf{O_{A/MK}}$ wird durch das Rahmenelement **R** gebildet (wie in den Abbildungen 4 B, 4 D gezeigt);
- der Rand der Oberflächen $\mathbf{O_{KK}}$ und $\mathbf{O_{A/MK}}$ wird vollständig durch das Rahmenelement **R** gebildet (wie in den Abbildungen 4 A, 4 C, 7 A, 7 B gezeigt);

Dabei kann das Rahmenelement **R** zusätzlich auch als mindestens ein Teil der Oberflächen jener Seiten der Trennwand **W** ausgebildet sein, bei denen es sich nicht um die Seiten $\mathbf{S_{KK}}$ und $\mathbf{S_{A/MK}}$ handelt. Insbesondere bildet das Rahmenelement **R** mindestens 1 %, bevorzugter mindestens 25 %, bevorzugter mindestens 50 %, noch bevorzugter 100 % der Oberflächen der Seiten der Trennwand **W** aus, bei denen es sich nicht um die Seiten $\mathbf{S_{KK}}$ und $\mathbf{S_{A/MK}}$ handelt.

In Fig. 4 B und Fig. 4 D sind beispielsweise Ausführungsformen gezeigt, in denen das Rahmenelement **R** einen Teil der Oberflächen jener Seiten der Trennwand **W** ausbildet, bei denen es sich nicht um die Seiten $\mathbf{S_{KK}}$ und $\mathbf{S_{A/MK}}$ handelt.

In Fig. 4 A und Fig. 4 C sind beispielsweise Ausführungsformen gezeigt, in denen das Rahmenelement **R** die Oberflächen jener Seiten der Trennwand **W,** bei denen es sich nicht um die Seiten $\mathbf{S_{KK}}$ und $\mathbf{S_{A/MK}}$ handelt, vollständig ausbildet.

Das Rahmenelement **R** ist insbesondere aus einem Material, welches ausgewählt ist aus der Gruppe bestehend aus Kunststoff, Glas, Holz ausgewählt ist. Besonders bevorzugt ist das Rahmenelement **R** aus Kunststoff.

Noch bevorzugter handelt es sich bei dem Kunststoff um einen, der aus der Gruppe bestehend aus Polypropylen, Polystyren, Polyvinylchlorid, PVC-C ausgewählt ist.

In einer weiteren bevorzugten Ausführungsform sind, wenn die Trennwand **W** ein Trennelement **T** und ein Rahmenelement **R** umfasst, das Rahmenelement **R** und das Trennelement **T** aus dem gleichen Material, noch bevorzugter sind beide aus Kunststoff, der noch bevorzugter ausgewählt ist aus Polypropylen, Polystyren, Polyvinylchlorid, PVC-C.

Das Rahmenelement **R** kann dabei aus einem Stück bestehen. Dann wird der AFK zum Beispiel mit einem dem Fachmann bekannten Mittel lückenlos an das Rahmenelement **R** befestigt, zum Beispiel über einen Klebstoff, wobei sich besonders Epoxidharze und Phenolharze eignen. Alternativ oder zusätzlich kann das Rahmenelement **R** auch so ausgeformt sein, dass das jeweilige AFK in das Rahmenelement **R** eingepasst oder eingeklemmt werden kann.

Dies bedeutet auch, dass in der bevorzugten Ausführungsform, in der die Trennwand **W** mindestens zwei AFKs $\mathbf{F_A}$, $\mathbf{F_B}$, mindestens ein Trennelement **T** und ein Rahmenelement **R** umfasst, die AFKs, das mindestens eine Trennelement **T** und das Rahmenelement **R** lückenlos aneinander anschließen.

Somit existieren dann keine Lücken, zwischen Trennelement **T,** Rahmenelement **R** und den von der Trennwand W umfassten AFKs, durch welche Glykol, glykolische Lösung, wässrige Lösung oder Wasser von der Seite $\mathbf{S_{KK}}$ auf die Seite $\mathbf{S_{A/MK}}$ oder umgekehrt fließen könnte.

Daneben kann, insbesondere wenn die Trennwand **W** mindestens zwei AFKs $\mathbf{F_A}$, $\mathbf{F_B}$, ein Rahmenelement **R** und mindestens ein Trennelement **T** umfasst, und das Rahmenelement **R** und das mindestens eine Trennelement **T** mindestens teilweise einstückig miteinander ausgebildet sind, das Rahmenelement **R** aus mindestens zwei Teilen bestehen, die aneinander befestigt sind und dabei die AFKs zwischen sich einklemmen. Beispielsweise kann die Trennwand **W** dann ein Scharnier aufweisen, an dem sich die beiden Teile des Rahmenelements **R** auf- und zuklappen lassen. Weiterhin kann die die Trennwand **W** dann ein Schloss aufweisen, an dem sich die beiden Teile des Rahmenelements **R** im zugeklappten Zustand arretieren lassen (Abbildung 7 A).

Im zugeklappten Zustand können dann die AFKs und, falls dieses nicht schon einstückig mit dem Rahmenelement **R** ausgebildet ist, das Trennelement **T** zwischen den beiden Teilen des Rahmenelements **R** eingeklemmt werden. In dieser Ausführungsform kann dann zwischen Trennelement **T** und AFK bzw. Rahmenelement **R und** AFK noch eine Dichtung angebracht sein, um die Flüssigkeitsdichte zu gewährleisten.

In einer bevorzugten Ausführungsform ist, wenn die Trennwand **W** mindestens zwei AFKs $\mathbf{F_A}$, $\mathbf{F_B}$, ein Rahmenelement **R** und mindestens ein Trennelement **T** umfasst, mindestens ein Teil des Trennelements **T** einstückig mit mindestens einem Teil des Rahmenelements **R** ausgebildet. Dies bedeutet insbesondere, dass dann mindestens ein Teil des Trennelements **T** in das Rahmenelement **R** übergeht.

Bevorzugt liegen das mindestens eine Trennelement **T** und das Rahmenelement **R** dann einstückig vor.

Die Ausführungsform eines Rahmenelements **R** hat den Vorteil, dass es beim Zusammenbau der Elektrolysezelle **E** als Teil der Außenwand fungieren kann. Dieser Teil der Trennwand **W** kontaktiert die Lösungen im jeweiligen Innenraum $\mathbf{I_{KK}}$, $\mathbf{I_{KA}}$ bzw. $\mathbf{I_{KM}}$ nicht, weshalb es Verschwendung wäre, für diesen Teil die mindestens eine Festelektrolytkeramik $\mathbf{F_A}$ zu nehmen. Daneben ist der Teil der Trennwand **W,** welcher zwischen die Außenwand geklemmt oder ein Teil davon bildet, Drücken ausgesetzt, was die brüchige Festelektrolytkeramik $\mathbf{F_A}$ ungeeignet macht. Stattdessen wird somit ein bruchsicheres und billigeres Material für den Rahmen **R** ausgewählt.

#### 1.1.4.4 Herstellung der Trennwand **W**

Die Trennwand **W** kann nach dem Fachmann bekannten Verfahren hergestellt werden.

Als Trennwand **W** kann in einer Ausführungsform des erfindungsgemäßen Verfahrens eine AFK $\mathbf{F_A}$ genutzt werden, die nach dem Fachmann bekannten Verfahren geschnitten oder geformt wird.

Umfasst die Trennwand **W** ein Rahmenelement **R** oder mindestens ein Trennelement **T,** können die von der Trennwand umfassten AFKs, gegebenenfalls mit Dichtungen, in eine Gießform eingelegt werden und das Trennelement über flüssigen Kunststoff gegossen und dann erstarren gelassen werden (Spritzgußverfahren). Beim Erstarren umschließt dieser dann die AFKs.

Alternativ wird das Trennelement **T** separat gegossen (bzw. in Teilen) und dann lückenlos an die mindestens zwei AFKs befestigt (zum Beispiel geklebt).

#### 1.1.4.5 Anordnung der Trennwand **W** in der Elektrolysezelle **E**

**1)** Die Trennwand **W** ist in der Elektrolysezelle **E** so angeordnet, dass die von der Trennwand **W** umfasste

alkalikationenleitende Festelektrolytkeramik $\mathbf{F_A}$ den Innenraum $\mathbf{I_{KK}}$ auf der Seite $\mathbf{S_{KK}}$ über die Oberfläche $\mathbf{O_{KK}}$ direkt kontaktiert.

Wenn die Trennwand **W** mindestens zwei AFKs $\mathbf{F_A}$, $\mathbf{F_B}$, mindestens ein Trennelement **T** und gegebenenfalls ein Rahmenelement **R** umfasst, ist die Trennwand **W** in der Elektrolysezelle **E** so angeordnet, dass die von der Trennwand **W** umfassten alkalikationenleitenden Festelektrolytkeramiken $\mathbf{F_A}$ und $\mathbf{F_B}$ und bevorzugt auch das Trennelement **T,** den Innenraum $\mathbf{I_{KK}}$ auf der Seite $\mathbf{S_{KK}}$ über die Oberfläche $\mathbf{O_{KK}}$ direkt kontaktieren.

Dies bedeutet, dass die Trennwand **W** in der Elektrolysezelle **E** so angeordnet ist, dass, wenn der Innenraum $\mathbf{I_{KK}}$ auf der Seite $\mathbf{S_{KK}}$ mit Lösung $\mathbf{L_2}$ vollständig gefüllt ist, dass die Lösung $\mathbf{L_2}$ dann über die Oberfläche $\mathbf{O_{KK}}$ mindestens die von der Trennwand **W** umfasste alkalikationenleitende Festelektrolytkeramik $\mathbf{F_A}$ kontaktiert, so dass Ionen (z.B. Alkalimetallionen wie Natrium, Lithium) aus $\mathbf{F_A}$ in die Lösung $\mathbf{L_2}$ eintreten können.

Wenn die Trennwand **W** mindestens zwei AFKs $\mathbf{F_A}$, $\mathbf{F_B}$, mindestens ein Trennelement **T** und gegebenenfalls ein Rahmenelement **R** umfasst, bedeutet dies, dass die Trennwand **W** in der Elektrolysezelle **E** so angeordnet ist, dass, wenn der Innenraum $\mathbf{I_{KK}}$ auf der Seite $\mathbf{S_{KK}}$ mit Lösung $\mathbf{L_2}$ vollständig gefüllt ist, dass die Lösung $\mathbf{L_2}$ dann über die Oberfläche $\mathbf{O_{KK}}$ mindestens die zwei von der Trennwand **W** umfassten alkalikationenleitenden Festelektrolytkeramiken $\mathbf{F_A}$ und $\mathbf{F_B}$ und bevorzugt auch das Trennelement **T** so kontaktiert, dass Ionen (z.B. Alkalimetallionen wie Natrium, Lithium) aus $\mathbf{F_A}$ und $\mathbf{F_B}$, in die Lösung $\mathbf{L_2}$ eintreten können.

**2)** Zusätzlich ist die Trennwand **W** in den Ausführungsformen, in denen die Elektrolysezelle **E** keine Mittelkammer $\mathbf{K_M}$ umfasst, so in der Elektrolysezelle **E** angeordnet, dass die von der Trennwand **W** umfasste alkalikationenleitende Festelektrolytkeramik $\mathbf{F_A}$ den Innenraum $I_{KA}$ auf der Seite $\mathbf{S_{A/MK}}$ über die Oberfläche $\mathbf{O_{A/MK}}$ direkt kontaktiert.

Wenn die Trennwand **W** mindestens zwei AFKs $\mathbf{F_A}$, $\mathbf{F_B}$, mindestens ein Trennelement **T** und gegebenenfalls ein Rahmenelement **R** umfasst, und wenn die Elektrolysezelle **E** keine Mittelkammer $\mathbf{K_M}$ umfasst, bedeutet dies, dass die Trennwand **W** so in der Elektrolysezelle **E** angeordnet ist, dass die von der Trennwand **W** umfassten alkalikationenleitenden Festelektrolytkeramiken, und bevorzugt auch das Trennelement **T,** den Innenraum $\mathbf{I_{KA}}$ auf der Seite $\mathbf{S_{A/MK}}$ über die Oberfläche $\mathbf{O_{A/MK}}$ direkt kontaktieren.

Dies bedeutet Folgendes: in den Ausführungsformen, in denen die Elektrolysezelle **E** keine Mittelkammer $\mathbf{K_M}$ umfasst, grenzt die Trennwand **W** an den Innenraum $\mathbf{I_{KA}}$ der Anodenkammer $\mathbf{K_A}$**.**

In diesen Ausführungsformen ist die Trennwand **W** in der Elektrolysezelle E dann so angeordnet, dass, wenn der Innenraum $\mathbf{I_{KA}}$ auf der Seite $\mathbf{S_{A/MK}}$ mit Lösung $L_3$ vollständig gefüllt ist, dass die Lösung $\mathbf{L_3}$ dann über die Oberfläche $\mathbf{O_{A/MK}}$ mindestens die von der Trennwand W umfasste alkalikationenleitende Festelektrolytkeramik $\mathbf{F_A}$ kontaktiert, so dass Ionen (z.B. Alkalimetallionen wie Natrium, Lithium) aus der Lösung $\mathbf{L_4}$ in die AFK $\mathbf{F_A}$ eintreten können.

Wenn die Trennwand **W** mindestens zwei AFKs $\mathbf{F_A}$, $\mathbf{F_B}$, mindestens ein Trennelement **T** und gegebenenfalls ein Rahmenelement **R** umfasst, bedeutet dies dann, dass die Trennwand **W** in der Elektrolysezelle **E** so angeordnet ist, dass, wenn der Innenraum $\mathbf{I_{KA}}$ auf der Seite $\mathbf{S_{A/MK}}$ mit Lösung $\mathbf{L_3}$ vollständig gefüllt ist, dass die Lösung $\mathbf{L_3}$ dann über die Oberfläche $\mathbf{O_{A/MK}}$ mindestens die zwei von der Trennwand **W** umfassten alkalikationenleitenden Festelektrolytkeramiken $\mathbf{F_A}$ und $\mathbf{F_B}$ und bevorzugt auch das Trennelement **T** so kontaktiert, dass Ionen (z.B. Alkalimetallionen wie Natrium, Lithium) aus der Lösung $\mathbf{L_3}$ in die AFK $\mathbf{F_A}$ und $\mathbf{F_B}$ eintreten können.

**3)** Zusätzlich ist die Trennwand **W** in den Fällen, in denen die Elektrolysezelle **E** mindestens eine Mittelkammer $\mathbf{K_M}$ umfasst, so in der Elektrolysezelle E angeordnet, dass die von der Trennwand **W** umfassten alkalikationenleitende Festelektrolytkeramik $\mathbf{F_A}$ den Innenraum $\mathbf{I_{KM}}$ auf der Seite $\mathbf{S_{A/MK}}$ über die Oberfläche $\mathbf{O_{A/MK}}$ direkt kontaktiert.

Wenn die Trennwand **W** mindestens zwei AFKs $\mathbf{F_A}$, $\mathbf{F_B}$, mindestens ein Trennelement **T** und gegebenenfalls ein Rahmenelement **R** umfasst, und wenn die Elektrolysezelle **E** mindestene eine Mittelkammer $\mathbf{K_M}$ umfasst, bedeutet dies, dass die Trennwand **W** so in der Elektrolysezelle **E** angeordnet ist, dass die von der Trennwand **W** umfassten alkalikationenleitenden Festelektrolytkeramiken, und bevorzugt auch das Trennelement **T,** den Innenraum $\mathbf{I_{KM}}$ auf der Seite $\mathbf{S_{A/MK}}$ über die Oberfläche $\mathbf{O_{A/MK}}$ direkt kontaktieren.

Dies bedeutet Folgendes: in den Ausführungsformen, in denen die Elektrolysezelle **E** mindestens eine Mittelkammer $\mathbf{K_M}$ umfasst, grenzt die Trennwand **W** an den Innenraum $\mathbf{I_{KM}}$ der Mittelkammer $\mathbf{K_M}$**.**

In diesen Ausführungsformen ist die Trennwand **W** in der Elektrolysezelle **E** dann so angeordnet, dass, wenn der Innenraum $I_{KM}$ auf der Seite $\mathbf{S_{A/MK}}$ mit Lösung $\mathbf{L_3}$ vollständig gefüllt ist, dass die Lösung $\mathbf{L_3}$ dann über die Oberfläche $\mathbf{O_{A/MK}}$ mindestens die von der Trennwand **W** umfasste alkalikationenleitende Festelektrolytkeramik $\mathbf{F_A}$ kontaktiert, so dass Ionen (z.B. Alkalimetallionen wie Natrium, Lithium) aus der Lösung $\mathbf{L_3}$ in die AFK $\mathbf{F_A}$ eintreten können.

Wenn die Trennwand **W** mindestens zwei AFKs $\mathbf{F_A}$, $\mathbf{F_B}$, mindestens ein Trennelement **T** und gegebenenfalls ein Rahmenelement **R** umfasst, bedeutet dies dann, dass die Trennwand **W** in der Elektrolysezelle **E** so angeordnet ist, dass, wenn der Innenraum $\mathbf{I_{KM}}$ auf der Seite $\mathbf{S_{A/MK}}$ mit Lösung $\mathbf{L_3}$ vollständig gefüllt ist, dass die Lösung $\mathbf{L_3}$ dann über die Oberfläche $\mathbf{O_{A/MK}}$ mindestens die zwei von der Trennwand **W** umfassten alkalikationenleitenden Festelektrolytkeramiken $\mathbf{F_A}$ und $\mathbf{F_B}$ und bevorzugt auch das Trennelement **T** so kontaktiert, dass Ionen (z.B. Alkalimetallionen wie Natrium, Lithium) aus der Lösung $\mathbf{L_3}$ in die AFK $\mathbf{F_A}$ und $\mathbf{F_B}$ eintreten können.

In einer bevorzugten Ausführungsform der Elektrolysezelle **E** kontaktieren mindestens 50 %, insbesondere

mindestens 70 %, bevorzugt mindestes 90 %, am bevorzugtesten 100 % des Teils der Oberfläche $\mathbf{O_{KK}}$, der durch AFKs gebildet wird, den Innenraum $\mathbf{I_{KK}}$.

In einer bevorzugten Ausführungsform der Elektrolysezelle **E** ohne Mittelkammer kontaktieren mindestens 50 %, insbesondere mindestens 70 %, bevorzugt mindestes 90 %, am bevorzugtesten 100 % des Teils der Oberfläche $\mathbf{O_{A/MK}}$, der durch AFKs gebildet wird, den Innenraum $I_{KA}$.

In einer bevorzugten Ausführungsform der Elektrolysezelle **E** mit mindestens einer Mittelkammer kontaktieren mindestens 50 %, insbesondere mindestens 70 %, bevorzugt mindestes 90 %, am bevorzugtesten 100 % des Teils der Oberfläche $\mathbf{O_{A/MK}}$, der durch AFKs gebildet wird, den Innenraum $\mathbf{I_{KM}}$.

<u>1.2 Schritt (a) des erfindungsgemäßen Verfahrens</u>

Schritt (a) des erfindungsgemäßen Verfahrens betrifft die Herstellung einer Lösung $L_1$ von $M_A$-Glykolat in Glykol, wobei $M_A$ ein Alkalimetallkation ist. Das Verfahren wird in einer Elektrolysezelle E durchgeführt.

Bevorzugt ist $M_A$ aus der Gruppe bestehend aus $Li^+$, $K^+$, $Na^+$, bevorzugter aus der Gruppe bestehend aus $K^+$, $Na^+$ ausgewählt. Am bevorzugtesten ist $M_A = Na^+$.

*1.2.1 Erfindungsgemäßes Verfahren in einer Elektrolysezelle **E** ohne Mittelkammer $\mathbf{K_M}$*

In den Fällen, in denen die Elektrolysezelle E keine Mittelkammer $\mathbf{K_M}$ umfasst, werden die gleichzeitig ablaufenden Schritt ($\alpha$1), ($\alpha$2), ($\alpha$3) durchgeführt.

<u>1.2.1.1 Schritt ($\alpha$1)</u>

Im Schritt ($\alpha$1) wird eine Lösung $\mathbf{L_2}$ umfassend Glykol, bevorzugt umfassend ein Alkalimetallglykolat $M_A$-Glykolat und Glykol, durch $I_{KK}$ geleitet.

Die Lösung $\mathbf{L_2}$ ist bevorzugt frei von Wasser. "Frei von Wasser" bedeutet erfindungsgemäß, dass das Gewicht des Wassers in der Lösung $\mathbf{L_2}$ bezogen auf das Gewichts des Glykols in der Lösung $\mathbf{L_2}$ (Massenverhältnis) $\leq 1 : 10$, bevorzugter $\leq 1 : 20$, noch bevorzugter $\leq 1 : 100$, noch bevorzugter $\leq 0.5 : 100$, noch bevorzugter $\leq 1 : 1000$, noch bevorzugter $\leq 1 : 10000$ ist.

Umfasst die Lösung $L_2$ $M_A$-Glykolat, so liegt der Massenanteil von $M_A$-Glykolat in der Lösung $L_2$, bezogen auf die gesamte Lösung $\mathbf{L_2}$, insbesondere bei > 0 bis 30 Gew.-%, bevorzugt bei 0.1 bis 20 Gew.-%, noch bevorzugter bei 0.2 bis 10 Gew.-%, noch bevorzugter bei 0.5 bis 5 Gew.-%, am bevorzugtesten bei 0.7 bis 2 Gew.-%, am allerbevorzugtesten bei 1 Gew.-%.

Umfasst die Lösung $\mathbf{L_2}$ $M_A$-Glykolat, so liegt in der Lösung $\mathbf{L_2}$ insbesondere das Massenverhältnis von $M_A$-Glykolat zu Glykol im Bereich 1 : 1000 bis 1 : 5, bevorzugter im Bereich 1 : 250 bis 3 : 20, noch bevorzugter im Bereich 1 : 120 bis 1 : 8, noch bevorzugter bei 1 : 100.

<u>1.2.1.2 Schritt ($\alpha$2)</u>

In Schritt ($\alpha$2) wird eine neutrale oder alkalische, wässrige Lösung $\mathbf{L_3}$ eines Salzes **S** umfassend $M_A$ als Kation durch $\mathbf{I_{KA}}$ geleitet.

Das Salz **S** ist bevorzugt ein Halogenid, Sulfat, Sulfit, Nitrat, Hydrogencarbonat oder Carbonat von $M_A$, noch bevorzugter ein Halogenid.

Halogenide sind Fluoride, Chloride, Bromide, Jodide. Das bevorzugteste Halogenid ist Chlorid.

Der pH der wässrigen Lösung $\mathbf{L_3}$ ist dabei $\geq 7.0$, bevorzugt im Bereich 7 bis 12, bevorzugter im Bereich 8 bis 11, noch bevorzugter 10 bis 11, am bevorzugtesten bei 10.5.

Der Massenanteil des Salzes **S** in der Lösung $\mathbf{L_3}$ liegt dabei bevorzugt im Bereich > 0 bis 20 Gew.-%, bevorzugt 1 bis 20 Gew.-%, bevorzugter bei 5 bis 20 Gew.-%, noch bevorzugter bei 10 bis 20 Gew.-%, am bevorzugtesten bei 20 Gew.-%, bezogen auf die gesamte Lösung $\mathbf{L_3}$.

<u>1.2.1.3 Schritt ($\alpha$3)</u>

Im Schritt ($\alpha$3) wird dann eine Spannung zwischen $\mathbf{E_A}$ und $\mathbf{E_K}$ angelegt.

Dadurch kommt es zu einem Stromtransport von der Ladungsquelle zur Anode, zu einem Ladungstransport über Ionen zur Kathode und schließlich zu einem Stromtransport zurück zur Ladungsquelle. Die Ladungsquelle ist dem Fachmann bekannt und ist typischerweise ein Gleichrichter, der Wechselstrom in Gleichstrom umwandelt und über Spannungsumwandler bestimmte Spannungen erzeugen kann.

Dies hat wiederum folgende Konsequenzen:

am Ablauf $\mathbf{A_{KK}}$ wird die Lösung $\mathbf{L_1}$ erhalten, wobei die Konzentration von $M_A$-Glykolat in $\mathbf{L_1}$ höher ist als in $\mathbf{L_2}$,

am Ablauf $\mathbf{A_{KA}}$ wird eine wässrige Lösung $L_4$ von S erhalten, wobei die Konzentration von **S** in $\mathbf{L_4}$ geringer ist als in $\mathbf{L_3}$.

Im Schritt ($\alpha$3) des erfindungsgemäßen Verfahrens wird insbesondere eine solche Spannung angelegt, dass so ein Strom fließt, so dass die Stromdichte (= Verhältnis des Stroms, der zur Elektrolysezelle fließt, zur Fläche des Festelektrolyten, die den in $\mathbf{I_{KA}}$ befindlichen Anolyten kontaktiert) im Bereich von 10 bis 8000 A/ $m^2$ liegt, bevorzugter im Bereich von 100 bis 2000 A/ $m^2$ liegt, noch bevorzugter im Bereich von 300 bis 800 A/ $m^2$, noch bevorzugter bei 494 A/ $m^2$ liegt. Dies kann vom Fachmann standardmäßig bestimmt werden. Die Fläche des Festelektrolyten, die den im Innenraum $\mathbf{I_{KA}}$ der Anodenkammer $\mathbf{K_A}$ befindlichen Anolyten kontaktiert, beträgt insbesondere 0.00001 bis 10 $m^2$, bevorzugt 0.0001 bis 2.5 $m^2$, bevorzugter 0.0002 bis 0.15 $m^2$, noch bevorzugter 2.83 $cm^2$.

Es versteht sich von selbst, dass der Schritt ($\alpha$3) des erfindungsgemäßen Verfahrens dann durchgeführt wird, wenn der Innenraum $\mathbf{I_{KA}}$ der Anodenkammer $\mathbf{K_A}$ mindestens teilweise mit $\mathbf{L_3}$ beladen ist und der Innenraum $\mathbf{I_{KK}}$ der Kathodenkammer $\mathbf{K_K}$ mit $\mathbf{L_2}$ mindestens teilweise beladen ist, so dass sowohl $\mathbf{L_3}$ als auch $\mathbf{L_2}$ die von der Trennwand **W** umfassten AFKs kontaktieren und insbesondere auch das Trennelement **T,** wenn die Trennwand **W ein** solches umfasst, kontaktieren.

Die Tatsache, dass in Schritt ($\alpha$3) ein Ladungstransport zwischen $\mathbf{E_A}$ und $\mathbf{E_K}$ stattfindet, impliziert, dass $\mathbf{I_{KK}}$ und $I_{KA}$ gleichzeitig mit $\mathbf{L_2}$ bzw. $\mathbf{L_3}$ so beladen sind, dass sie die Elektroden $\mathbf{E_K}$ bzw. $\mathbf{E_A}$ so weit bedecken, dass der Stromkreislauf geschlossen ist.

Das ist insbesondere dann der Fall, wenn kontinuierlich ein Flüssigkeitsstrom von $\mathbf{L_3}$ durch $I_{KA}$ und ein Flüssigkeitsstrom von $\mathbf{L_2}$ durch $\mathbf{I_{KK}}$ geleitet wird und der Flüssigkeitsstrom von $\mathbf{L_3}$ die Elektrode $\mathbf{E_A}$ und der Flüssigkeitsstrom von $\mathbf{L_2}$ die Elektrode $\mathbf{E_K}$ mindestens teilweise, bevorzugt vollständig bedeckt.

In einer weiteren bevorzugten Ausführungsform wird das Verfahren gemäß der Erfindung kontinuierlich durchgeführt, also Schritt ($\alpha$1) und Schritt ($\alpha$2) kontinuierlich durchgeführt und dabei gemäß Schritt ($\alpha$3) Spannung angelegt.

Nach Durchführung des Schrittes ($\alpha$3) wird am Ablauf $\mathbf{A_{KK}}$ die Lösung $\mathbf{L_1}$ erhalten, wobei die Konzentration von $M_A$-Glykolat in $\mathbf{L_1}$ höher ist als in $\mathbf{L_2}$. Wenn $\mathbf{L_2}$ schon $M_A$-Glykolat umfasste, ist die Konzentration von $M_A$-Glykolat in $\mathbf{L_1}$ bevorzugt um das 1.01 bis 200.2-fache, bevorzugter um das 5.04 bis 100.8-fache, noch bevorzugter um das 10.077 bis 50.4-fache, noch mehr bevorzugter um das 18.077 bis 20.08-fache höher als in $\mathbf{L_2}$, am bevorzugtesten um das 20.00-fache höher als in $\mathbf{L_2}$, wobei noch bevorzugter dabei der Massenanteil von $M_A$-Glykolat in $\mathbf{L_1}$ und in $\mathbf{L_2}$ im Bereich 0.1 bis 50 Gew.-%, noch mehr bevorzugter 1 bis 20 Gew.-% liegt.

Am Ablauf $\mathbf{A_{KA}}$ wird eine wässrige Lösung $\mathbf{L_4}$ von **S** erhalten, wobei die Konzentration von **S** in $\mathbf{L_4}$ geringer ist als in $\mathbf{L_3}$.

Die Konzentration des Kations $M_A$ in der wässrigen Lösung $L_3$ liegt bevorzugt im Bereich 0.5 bis 5 mol/l, bevorzugter 1 mol/l. Die Konzentration des Kations $M_A$ in der wässrigen Lösung $L_4$ ist bevorzugter 0.5 mol/l geringer als jene der jeweils eingesetzten wässrigen Lösung $\mathbf{L_3}$.

Insbesondere werden die Schritte ($\alpha$1) bis ($\alpha$3) des erfindungsgemäßen Verfahrens bei einer Temperatur von 20 °C bis 110 °C, bevorzugt 50 °C bis 105 °C, bevorzugter 80 °C bis 99 °C, noch bevorzugter 90 °C bis 95 °C und einem Druck von 0.5 bar bis 1.5 bar, bevorzugt 0.9 bar bis 1.1 bar, bevorzugter 1.0 bar durchgeführt.

Bei der Durchführung der Schritte ($\alpha$1) bis ($\alpha$3) des erfindungsgemäßen Verfahrens entsteht in $\mathbf{I_{KK}}$ typischerweise Wasserstoff, der über den Ablauf $\mathbf{A_{KK}}$ aus der Zelle zusammen mit der Lösung $\mathbf{L_1}$ abgeführt werden kann. Die Mischung aus Wasserstoff und Lösung $\mathbf{L_1}$ kann dann in einer besonderen Ausführungsform der vorliegenden Erfindung nach dem Fachmann bekannten Verfahren aufgetrennt werden. In $\mathbf{I_{KA}}$, wenn es sich bei der eingesetzten Alkalimetallverbindung um ein Halogenid, insbesondere Chlorid handelt, kann Chlor oder ein anderes Halogengas entstehen, welches über den Ablauf $\mathbf{A_{KK}}$ aus der Zelle zusammen mit der Lösung $\mathbf{L_4}$ abgeführt werden kann. Daneben kann auch Sauerstoff oder/und Kohlendioxid entstehen, was ebenso abgeführt werden kann. Die Mischung aus Chlor, Sauerstoff und/oder $CO_2$ und Lösung $\mathbf{L_4}$ kann dann in einer besonderen Ausführungsform der vorliegenden Erfindung nach dem Fachmann bekannten Verfahren aufgetrennt werden. Genauso kann dann nach Abtrennung der Gase Chlor, Sauerstoff und/oder $CO_2$ von der Lösung $\mathbf{L_4}$ diese nach dem Fachmann bekannten Verfahren voneinander abgetrennt werden.

### *1.2.2 Erfindungsgemäßes Verfahren in einer Elektrolysezelle **E** mit Mittelkammer $\mathbf{K_M}$*

In den Fällen, in denen die Elektrolysezelle **E** mindestens eine Mittelkammer $\mathbf{K_M}$ umfasst, werden die gleichzeitig ablaufenden Schritte ($\beta$1), ($\beta$2), ($\beta$3) durchgeführt.

Es ist bevorzugt, dass die Elektrolysezelle **E** mindestens eine Mittelkammer $\mathbf{K_M}$ umfasst, und dann die gleichzeitig ablaufenden Schritte ($\beta$1), ($\beta$2), ($\beta$3) durchgeführt werden.

1.2.2.1 Schritt (β1)

Im Schritt (β1) wird eine Lösung $\mathbf{L_2}$ umfassend Glykol, bevorzugt umfassend ein Alkalimetallglykolat $M_A$-Glykolat und Glykol, durch $I_{KK}$ geleitet.
Die Lösung $\mathbf{L_2}$ ist bevorzugt frei von Wasser. "Frei von Wasser" bedeutet erfindungsgemäß, dass das Gewicht des Wassers in der Lösung $\mathbf{L_2}$ bezogen auf das Gewichts des Glykols in der Lösung $\mathbf{L_2}$ (Massenverhältnis) ≤ 1 : 10, bevorzugter ≤ 1 : 20, noch bevorzugter ≤ 1 : 100, noch bevorzugter ≤ 0.5 : 100 ist.
Umfasst die Lösung $\mathbf{L_2}$ $M_A$-Glykolat, so liegt der Massenanteil von $M_A$-Glykolat in der Lösung $\mathbf{L_2}$, bezogen auf die gesamte Lösung $\mathbf{L_2}$, insbesondere bei > 0 bis 30 Gew.-%, bevorzugt bei 0.1 bis 20 Gew.-%, noch bevorzugter bei 0.2 bis 10 Gew.-%, noch bevorzugter bei 0.5 bis 5 Gew.-%, am bevorzugtesten bei 0.7 bis 2 Gew.-%, am allerbevorzugtesten bei 1 Gew.-%.
Umfasst die Lösung $\mathbf{L_2}$ $M_A$-Glykolat, so liegt in der Lösung $\mathbf{L_2}$ insbesondere das Massenverhältnis von $M_A$-Glykolat zu Glykol im Bereich 1 : 1000 bis 1 : 5, bevorzugter im Bereich 1 : 250 bis 3 : 20, noch bevorzugter im Bereich 1 : 120 bis 1 : 8, noch bevorzugter bei 1 : 100.

1.2.2.2 Schritt (β2)

In Schritt (β2) wird eine neutrale oder alkalische, wässrige Lösung $\mathbf{L_3}$ eines Salzes **S** umfassend $M_A$ als Kation durch $\mathbf{I_{KM}}$, dann über $\mathbf{V_{AM}}$, dann durch $I_{KA}$ geleitet.
Das Salz **S** ist bevorzugt ein Halogenid, Sulfat, Sulfit, Nitrat, Hydrogencarbonat oder Carbonat von $M_A$, noch bevorzugter ein Halogenid.
Halogenide sind Fluoride, Chloride, Bromide, Jodide. Das bevorzugteste Halogenid ist Chlorid.
Der pH der wässrigen Lösung $\mathbf{L_3}$ ist dabei ≥ 7.0, bevorzugt im Bereich 7 bis 12, bevorzugter im Bereich 8 bis 11, noch bevorzugter 10 bis 11, am bevorzugtesten bei 10.5.
Der Massenanteil des Salzes **S** in der Lösung $\mathbf{L_3}$ liegt dabei bevorzugt im Bereich > 0 bis 20 Gew.-%, bevorzugt 1 bis 20 Gew.-%, bevorzugter bei 5 bis 20 Gew.-%, noch bevorzugter bei 10 bis 20 Gew.-%, am bevorzugtesten bei 20 Gew.-%, bezogen auf die gesamte Lösung $\mathbf{L_3}$.

1.2.2.3 Schritt (β3)

Im Schritt (β3) wird dann eine Spannung zwischen $\mathbf{E_A}$ und $\mathbf{E_K}$ angelegt.
Dadurch kommt es zu einem Stromtransport von der Ladungsquelle zur Anode, zu einem Ladungstransport über Ionen zur Kathode und schließlich zu einem Stromtransport zurück zur Ladungsquelle. Die Ladungsquelle ist dem Fachmann bekannt und ist typischerweise ein Gleichrichter, der Wechselstrom in Gleichstrom umwandelt und über Spannungsumwandler bestimmte Spannungen erzeugen kann.
Dies hat wiederum folgende Konsequenzen:

am Ablauf $\mathbf{A_{KK}}$ wird die Lösung $\mathbf{L_1}$ erhalten, wobei die Konzentration von $M_A$-Glykolat in $\mathbf{L_1}$ höher ist als in $\mathbf{L_2}$,

am Ablauf $\mathbf{A_{KA}}$ wird eine wässrige Lösung $\mathbf{L_4}$ von **S** erhalten, wobei die Konzentration von **S** in $\mathbf{L_4}$ geringer ist als in $\mathbf{L_3}$.

Im Schritt (β3) des erfindungsgemäßen Verfahrens wird insbesondere eine solche Spannung angelegt, dass so ein Strom fließt, so dass die Stromdichte (= Verhältnis des Stroms, der zur Elektrolysezelle fließt, zur Fläche des Festelektrolyten, die den in $\mathbf{I_{KM}}$ befindlichen Anolyten kontaktiert) im Bereich von 10 bis 8000 A/ $m^2$ liegt, bevorzugter im Bereich von 100 bis 2000 A/ $m^2$ liegt, noch bevorzugter im Bereich von 300 bis 800 A/ $m^2$, noch bevorzugter bei 494 A/ $m^2$ liegt. Dies kann vom Fachmann standardmäßig bestimmt werden. Die Fläche des Festelektrolyten, die den in der Mittelkammer $\mathbf{K_M}$ befindlichen Anolyten kontaktiert beträgt insbesondere 0.00001 bis 10 $m^2$, bevorzugt 0.0001 bis 2.5 $m^2$, bevorzugter 0.0002 bis 0.15 $m^2$, noch bevorzugter 2.83 $cm^2$.
Es versteht sich von selbst, dass der Schritt (β3) des erfindungsgemäßen Verfahrens dann durchgeführt wird, wenn die Innenräume $\mathbf{I_{KA}}$ und $\mathbf{I_{KM}}$ beider Kammern $\mathbf{K_M}$ und $\mathbf{K_A}$ mindestens teilweise mit $\mathbf{L_3}$ beladen sind und der Innenraum $\mathbf{I_{KK}}$ mit $\mathbf{L_2}$ mindestens teilweise beladen ist, so dass sowohl $\mathbf{L_3}$ als auch $\mathbf{L_2}$ die von der Trennwand **W** umfassten Festelektrolyten kontaktieren und insbesondere auch das Trennelement **T,** wenn die Trennwand **W ein** solches umfasst, kontaktieren.
Die Tatsache, dass in Schritt (β3) ein Ladungstransport zwischen $\mathbf{E_A}$ und $\mathbf{E_K}$ stattfindet, impliziert, dass $\mathbf{I_{KK}}$, $\mathbf{I_{KM}}$ und $\mathbf{I_{KA}}$ gleichzeitig mit $\mathbf{L_2}$ bzw. $\mathbf{L_3}$ so beladen sind, dass sie die Elektroden $\mathbf{E_K}$ bzw. $\mathbf{E_A}$ so weit bedecken, dass der Stromkreislauf geschlossen ist.
Das ist insbesondere dann der Fall, wenn kontinuierlich ein Flüssigkeitsstrom von $\mathbf{L_3}$ durch $\mathbf{I_{KM}}$, $\mathbf{V_{AM}}$ und $\mathbf{I_{KP}}$, und ein Flüssigkeitsstrom von $\mathbf{L_2}$ durch $\mathbf{I_{KK}}$ geleitet wird und der Flüssigkeitsstrom von $L_3$ die Elektrode $\mathbf{E_A}$ und der

Flüssigkeitsstrom von $L_2$ die Elektrode $\mathbf{E_K}$ mindestens teilweise, bevorzugt vollständig bedeckt.

In einer weiteren bevorzugten Ausführungsform wird das Verfahren gemäß der Erfindung kontinuierlich durchgeführt, also Schritt (β1) und Schritt (β2) kontinuierlich durchgeführt und dabei gemäß Schritt (β3) Spannung angelegt.

Nach Durchführung des Schrittes (β3) wird am Ablauf $\mathbf{A_{KK}}$ die Lösung $\mathbf{L_1}$ erhalten, wobei die Konzentration von $M_A$-Glykolat in $\mathbf{L_1}$ höher ist als in $\mathbf{L_2}$. Wenn $\mathbf{L_2}$ schon $M_A$-Glykolat umfasste, ist die Konzentration von $M_A$-Glykolat in $\mathbf{L_1}$ bevorzugt um das 1.01 bis 200.2-fache, bevorzugter um das 5.04 bis 100.80-fache, noch bevorzugter um das 10.077 bis 50.40-fache, noch mehr bevorzugter um das 18.077 bis 20.08-fache höher als in $\mathbf{L_2}$, am bevorzugtesten um das 20.00-fache höher als in $\mathbf{L_2}$, wobei noch bevorzugter dabei der Massenanteil von $M_A$-Glykolat in $\mathbf{L_1}$ und in $\mathbf{L_2}$ im Bereich 0.1 bis 50 Gew.-%, noch mehr bevorzugter 1 bis 20 Gew.-% liegt.

Am Ablauf $\mathbf{A_{KA}}$ wird eine wässrige Lösung $\mathbf{L_4}$ von **S** erhalten, wobei die Konzentration von **S** in $\mathbf{L_4}$ geringer ist als in $\mathbf{L_3}$.

Die Konzentration des Kations $M_A$ in der wässrigen Lösung $\mathbf{L_3}$ liegt bevorzugt im Bereich 0.5 bis 5 mol/l, bevorzugter 1 mol/l. Die Konzentration des Kations $M_A$ in der wässrigen Lösung $\mathbf{L_4}$ ist bevorzugter 0.5 mol/l geringer als jene der jeweils eingesetzten wässrigen Lösung $\mathbf{L_3}$.

Insbesondere werden die Schritte (β1) bis (β3) des erfindungsgemäßen Verfahrens bei einer Temperatur von 20 °C bis 110 °C, bevorzugt 50 °C bis 105 °C, bevorzugter 80 °C bis 99 °C, noch bevorzugter 90 °C bis 95 °C und einem Druck von 0.5 bar bis 1.5 bar, bevorzugt 0.9 bar bis 1.1 bar, bevorzugter 1.0 bar durchgeführt.

Bei der Durchführung der Schritte (β1) bis (β3) des erfindungsgemäßen Verfahrens entsteht in der Kathodenkammer $I_{KK}$ typischerweise Wasserstoff, der über den Ablauf $\mathbf{A_{KK}}$ aus der Zelle zusammen mit der Lösung $\mathbf{L_1}$ abgeführt werden kann. Die Mischung aus Wasserstoff und Lösung $\mathbf{L_1}$ kann dann in einer besonderen Ausführungsform der vorliegenden Erfindung nach dem Fachmann bekannten Verfahren aufgetrennt werden. In $\mathbf{I_{KA}}$, wenn es sich bei der eingesetzten Alkalimetallverbindung um ein Halogenid, insbesondere Chlorid handelt, kann Chlor oder ein anderes Halogengas entstehen, welches über den Ablauf $\mathbf{A_{KK}}$ aus der Zelle zusammen mit der Lösung $\mathbf{L_4}$ abgeführt werden kann. Daneben kann auch Sauerstoff oder/und Kohlendioxid entstehen, was ebenso abgeführt werden kann. Die Mischung aus Chlor, Sauerstoff und/oder $CO_2$ und Lösung $\mathbf{L_4}$ kann dann in einer besonderen Ausführungsform der vorliegenden Erfindung nach dem Fachmann bekannten Verfahren aufgetrennt werden. Genauso kann dann nach Abtrennung der Gase Chlor, Sauerstoff und/oder $CO_2$ von der Lösung $\mathbf{L_4}$ diese nach dem Fachmann bekannten Verfahren voneinander abgetrennt werden.

### 1.2.2.4 Zusätzliche Vorteile der Schritte (β1) bis (β3)

Diese Durchführung der Schritte (β1) bis (β3) bringen noch weitere überraschende Vorteile, die im Lichte des Standes der Technik nicht zu erwarten waren. Durch die Schritte (β1) bis (β3) des erfindungsgemäßen Verfahrens wird der säurelabile Feststoffelektrolyt vor Korrosion geschützt, ohne dass dabei wie im Stand der Technik Alkoholatlösung aus dem Kathodenraum als Pufferlösung geopfert werden muss. Damit ist das erfindungsgemäße Verfahren effizienter als die in WO 2008/076327 A1 beschriebene Vorgehensweise, in der die Produktlösung für die Mittelkammer verwendet wird, was den Gesamtumsatz schmälert.

## 2. Schritt (b): Umsetzung von PET mit der Lösung $L_1$ <21>

In Schritt (b) des erfindungsgemäßen Verfahrens wird die in Schritt (a) erhaltene Lösung $L_1$ <21> umfassend Glykol und $M_A$-Glykolat mit **PET** zu einer Mischung $\mathbf{M_1}$ umfassend **BHET** umgesetzt.

### *2.1 PET-Ausgangsmaterial*

Als **PET,** welches im Schritt (b) des erfindungsgemäßen Verfahrens eingesetzt wird, kann jegliches **PET** eingesetzt werden, was depolymerisiert werden muss. Typischerweise fällt solches **PET** als Abfall an, insbesondere im Haushalt, in der Industrie oder in der Landwirtschaft.

In einer Ausführungsform des erfindungsgemäßen Verfahrens liegt das damit zu depolymerisierende **PET** in Mischung mit anderen Kunststoffen, insbesondere mindestens einem Kunststoff ausgewählt aus Polyethylen ("PE"), Polyvinylchlorid ("PVC") vor. Dies ist typischerweise der Fall, wenn im erfindungsgemäßen Verfahrens **PET** aus Kunststoffabfällen depolymerisiert werden soll. In dieser Ausführungsform wird das **PET** mindestens teilweise von den anderen Kunststoffen, bevorzugt durch Assortieren, abgetrennt, bevor es dem Schritt (b) des erfindungsgemäßen Verfahrens unterworfen wird.

In einer Ausführungsform des erfindungsgemäßen Verfahrens wird das **PET** mindestens einem Vorbehandlungsschritt ausgesetzt.

Solche Vorbehandlungsschritte sind beispielsweise beschrieben in DE 10032899 C2.

Erfindungsgemäß wird das **PET** mindestens einem Vorbehandlungsschritt ausgewählt aus chemischen Vorbehandlungsschritt, Zerkleinerungsschritt unterworfen, bevor es in Schritt (b) eingesetzt wird.

In den Fällen, in denen das **PET** in Mischung mit anderen Kunststoffen vorliegt, wird das **PET** bevorzugt mindestens einem Vorbehandlungsschritt ausgewählt aus mindestens teilweiser Abtrennung von anderen Kunststoffen, bevorzugt durch Aussortierung, chemischen Vorbehandlungsschritt, Zerkleinerungsschritt unterworfen, bevor es in Schritt (b) eingesetzt wird.

In den Fällen, in denen das **PET** in Mischung mit anderen Kunststoffen vorliegt, wird das **PET** bevorzugter erst mindestens teilweise von anderen Kunststoffen abgetrennt, dann mindestens einmal chemisch vorbehandelt und schließlich zerkleinert.

Bei dem chemischen Vorbehandlungsschritt handelt es sich insbesondere um einen Waschschritt. Ein solcher Waschschritt hat den Vorteil, dass vor der Durchführung des Schrittes (b) eventuelle Verunreinigungen insbesondere Speisereste, Reste von Kosmetika und/oder Körpersekrete (z.B. Blut, Sperma, Fäkalien), entfernt werden. Solche Verunreinigungen könnten die Effizienz der Umsetzung in Schritt (b) herabsetzen und/oder die Reinheit des damit erhaltenen **BHET** verschlechtern.

Beim chemischen Vorbehandlungsschritt, insbesondere dem Waschschritt, wird der Abfall insbesondere in einer Waschlösung bei einer Temperatur von 30 °C bis 99 °C, bevorzugt 50 C° bis 90 °C, noch bevorzugter 70 °C bis 85 °C, erhitzt.

Typische Waschlösungen sind dem Fachmann geläufig und bevorzugt ausgewählt aus:

- wässrige Lösung eines Tensids, bevorzugt eines nicht-ionischen Tensids;
- wässrige Lösung eines Alkalimetallhydroxids oder Erdalkalimetallhydroxids; bevorzugt wässrige NaOH.

Die Behandlungszeit des chemischen Vorbehandlungsschritts, insbesondere des Waschschritts, beträgt dabei insbesondere 1 min bis 12 h, bevorzugt 10 min bis 6 h, bevorzugter 30 min bis 2 h, noch bevorzugter 45 bis 90 min, am bevorzugtesten 60 min.

Nach der Behandlung des **PET** mit dem chemischen Vorbehandlungsschritt, insbesondere dem Waschschritt, wird die wässrige Lösung abgetrennt, z.B. durch Filtration, und das gesäuberte **PET** bevorzugt mindestens einmal mit Wasser gewaschen, um Reste der Waschlösung zu entfernen.

Der so erhaltene PET-Abfall wird dann getrocknet, insbesondere in einem Trockenschrank. Die zum Trocknen eingesetzt Temperatur liegt dabei insbesondere im Bereich 30 bis 120 °C, bevorzugt 50 °C bis 100 °C, bevorzugter 60 °C bis 90 °C, am bevorzugtesten bei 80 °C.

Der Zerkleinerungsschritt hat den Vorteil, dass die in Schritt (b) für die Reaktion zur Verfügung stehende Oberfläche des **PET** erhöht wird. Dadurch erhöht sich die Reaktionsgeschwindigkeit der Umsetzung in Schritt (b) erhöht. Die Zerkleinerung kann in den Fachmann bekannten Apparaturen erfolgen, beispielsweise einem Shredder oder einer Schneidmühle.

In einer weiteren Ausführungsform des erfindungsgemäßen Verfahrens wird das **PET,** bevor es dem Schritt (b) unterworfen wird, entfärbt oder gezielt gefärbt. Dies kann mit dem Fachmann bekannten Verfahren durchgeführt werden, z.B. Entfärbung mit Wasserstoffperoxid oder Färbung mit einem Farbstoff.

*2.2 Umsetzungsbedingungen*

Die Umsetzung des **PET** mit einer Lösung $L_1$ <21> umfassend Glykol und $M_A$-Glykolat zu einer Mischung $\mathbf{M_1}$ kann dann unter dem Fachmann geläufigen Bedingungen erfolgen.

Bevorzugt wird die Umsetzung in Schritt (b) so lange, d.h. bis zu einem Zeitpunkt $t_b$, durchgeführt bis sich mindestens **P** = 10 %, bevorzugt mindestens **P** = 20 %, bevorzugter mindestens **P** = 25 %, bevorzugter mindestens **P** = 30 %, bevorzugter mindestens **P** = 40 %, bevorzugter mindestens **P** = 50 %, bevorzugter mindestens **P** = 60 %, bevorzugter mindestens **P** = 70 %, bevorzugter mindestens **P** = 80 %, bevorzugter mindestens **P** = 90 %, bevorzugter mindestens **P** = 95 %, noch bevorzugter mindestens **P** = 99 % des in Schritt (b) eingesetzten **PETs** umgesetzt haben.

Dieser prozentuale Anteil **P** wird nach der folgenden Formel berechnet:

$$\mathbf{P} = (n_{\mathbf{TS}} + n_{\mathbf{MHET}} + n_{\mathbf{BHET}}) / n_{\mathbf{PET}}.$$

Dabei ist $n_{PET}$ die Stoffmenge an Wiederholungseinheiten der folgenden Struktur (Ξ) in dem in Schritt (b) eingesetzten **PET:**

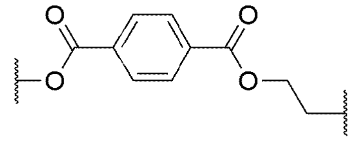

(Ξ)

$n_{TS}$ ist die Stoffmenge an **TS,** die sich von Beginn des Schrittes (b) bis zum Zeitpunkt $t_b$ in Schritt (b) gebildet haben.

$n_{MHET}$ ist die Stoffmenge an **MHET,** die sich von Beginn des Schrittes (b) bis zum Zeitpunkt $t_b$ in Schritt (b) gebildet haben.

$n_{BHET}$ ist die Stoffmenge an **BHET,** die sich von Beginn des Schrittes (b) bis zum Zeitpunkt $t_b$ in Schritt (b) gebildet haben.

Die Strukturen der Verbindungen **BHET, MHET, TS** sind wie folgt:

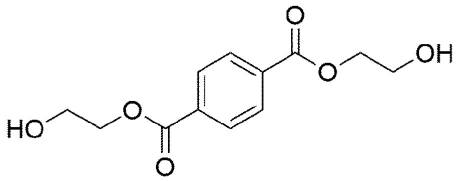

**BHET**

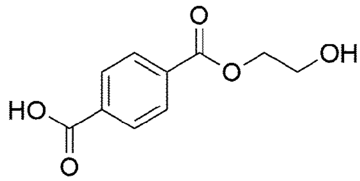

**MHET**

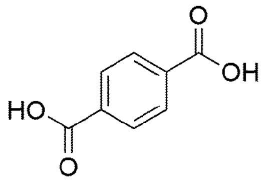

**TS**

**"MHET"** umfasst auch das entsprechende Carboxylat der gezeigten Struktur.

"**TS**" umfasst auch das entsprechende Mono- und Dicarboxylat der gezeigten Struktur.

Die Umsetzung in Schritt (b) wird dabei insbesondere bei einer Temperatur von mindestens 100 °C, bevorzugt bei einer Temperatur im Bereich von ≥ 100 °C bis ≤ 197 °C, bevorzugter bei einer Temperatur im Bereich von ≥ 130 °C bis ≤ 197 °C, bevorzugter bei einer Temperatur im Bereich von ≥ 150 °C bis ≤ 197 °C, bevorzugter bei einer Temperatur im Bereich von ≥ 175 °C bis ≤ 197 °C durchgeführt.

Die Umsetzung in Schritt (b) wird bevorzugt bei der Siedetemperatur des Glykols, durchgeführt. Noch bevorzugter wird Glykol dabei refluxiert, das heißt Glykol wird aus der Umsetzung verdampft, kondensiert und dann wieder in die Umsetzung rückgeführt. Diese Refluxierung kann mit dem Fachmann geläufigen Mitteln eingestellt werden, beispielsweise in einer Destillationsapparatur.

Das Gesamtgewicht des im Verfahren eingesetzten $M_A$-Glykolat bezogen auf das Gesamtgewicht des im Verfahren eingesetzten **PETs** liegt insbesondere im Bereich von 0.1 bis 100 Gew.-%, bevorzugt im Bereich von 0.5 bis 80 Gew.-%, bevorzugter im Bereich von 1.0 bis 50 Gew.-%, bevorzugter im Bereich von 1.5 bis 25 Gew.-%, bevorzugter im Bereich von 2.0 bis 10 Gew.-%, bevorzugter im Bereich von 2.5 bis 6.0 Gew.-%, besonders bevorzugt bei 3.5 bis 5.0 Gew-%, am bevorzugtesten bei 3.9 Gew.-%.

Die Umsetzung kann mit dem Fachmann geläufigen Geräten erfolgen.

Nach Beendigung des Schrittes (b) des erfindungsgemäßen Verfahrens wird eine Mischung $M_1$ erhalten, in der das molare Verhältnis η der Stoffmenge von **BHET** ($n_{BHET}$) zur Summe der Stoffmengen von **MHET** und **TS** ($n_{MHET} + n_{TS}$) im Bereich 1 : 1 bis 1000 : 1, bevorzugt 2 : 1 bis 500 : 100, bevorzugter 4 : 1 bis 300 : 1, noch bevorzugter 10 : 1 bis 100 : 1, noch mehr bevorzugter 13 : 1 bis 60 : 1, noch mehr bevorzugter 13 : 1 bis 24 : 1 liegt.

$$\eta = n_{BHET} / (n_{MHET} + n_{TS})$$

*2.3 Bevorzugter Schritt (c)*

In einem bevorzugten, weiteren Schritt (c) wird **BHET** mindestens teilweise aus $\mathbf{M_1}$ abgetrennt. Dies erfolgt noch bevorzugter durch Kristallisation und/oder Destillation. Noch bevorzugter wird **BHET** in Schritt (c) aus $\mathbf{M_1}$ abfiltriert und

dann auskristallisiert.

3. Verfahren zum Recycling von **PET**

Das im erfindungsgemäßen Verfahren in der Mischung $\mathbf{M_1}$ erhaltene **BHET** wird bevorzugt in einem Verfahren zum Recycling von Polyethylenterephthalat in einem Schritt (ζ) zu **PET** polymerisiert.

Diese Polymerisierung ist dem Fachmann als "Polykondensation" bekannt und z.B. in EP 0 723 951 A1 und von Th. Rieckmann und S. Völker in Kapitel 2 "Poly(Ethylen Terephthalate) Polymerization - Mechanism, Catalysis, Kinetics, Mass Transfer and Reactor Design" auf Seite 92 des Buches " Modern Polyesters: Chemistry and Technology of Polyesters and Copolyesters. Edited by J. Scheirs and T. E. Long, 2003, John Wiley & Sons, Ltd ISBN: 0-471-49856-4" beschrieben.

Insbesondere wird dazu **BHET** im Schritt (ζ) in Gegenwart von Katalysatoren, bei denen es sich insbesondere um Katalysatoren, die aus der Gruppe bestehend aus Antimonverbindungen, bevorzugt $Sb_2O_3$ ausgewählt sind, handelt, wieder zu **PET** polymerisiert.

Bevorzugt wird die Polymerisierung von **BHET** zu **PET** in Schritt (ζ) mindestens bei der Siedetemperatur des Glykols durchgeführt. Insbesondere wird während der Polymerisierung in Schritt (ζ) Glykol aus dem Reaktionsgemisch entfernt, um das Reaktionsgleichgewicht auf die Seite des Polymers **PET** zu verschieben.

Bevorzugter wird die Polymerisierung von **BHET** zu **PET** in Schritt (ζ) bei der Siedetemperatur des Glykols durchgeführt. Noch bevorzugter wird dann während der Polymerisierung in Schritt (ζ) Glykol aus dem Reaktionsgemisch entfernt, um das Reaktionsgleichgewicht auf die Seite des Polymers **PET** zu verschieben.

Dies wird insbesondere durch Destillation bei einem Druck < 1 bar, bevorzugt 0.1 mbar bei der gleichzeitigen Siedetemperatur des Glykols beim jeweiligen Druck erreicht.

**Beispiele**

1. Erfinderisches Beispiel **E1:**

*1.1 Herstellung der glykolischen Natriumglykolatösung durch Elektrolyse*

1.1.1 Versuchsaufbau

Die elektrolytische Gewinnung von Natriumglykolat wurde in einer Dreikammer-Elektrolysezelle durchgeführt.

Die Mittelkammer war durch ein Filtertuch von der Anodenkammer und durch eine 15 x 15 cm Nasicon-Keramik von der Kathodenkammer abgetrennt.

Als Anode wurde eine DSA-Anode ["*dimensionally stable anode";* mit Rutheniumoxid/ Iridiumoxid beschichtete Titananode ($RuO_2$+ $IrO_2$ / Ti)] verwendet, die Kathode bestand aus VA-Stahl (VA bedeutet "rostfrei"; Edelstahl).

Es wurden ein Potentiostat Gamry Reference 3000 (AE) und Reference 30K Booster als Spannungsquelle eingesetzt.

Die Kathodenkammer der Elektrolysezelle war mit einem 250 ml-beheizbaren Doppelmantelgefäß mit Magnetrührer verbunden, aus dem über eine Perestaltikpumpe Elektrolyt über eine Leitfähigkeitsmessstelle und einen weiteren 100 ml-Wärmetauscher aus Glas in die Kathodenkammer der Elektrolysezelle gepumpt werden konnte. Von dort konnte der Katholyt wieder in das Doppelmantelgefäß zurückgepumpt werden. Der Katholyt wurde demnach im Kreis gefahren.

Über einen Thermostaten mit PT 100 Sensor (Platinsensor, der bei einer Temperatur von 0 °C einen Nennwiderstand von 100 Ω aufweist) und einen Wärmetauscher konnte die Temperatur des kathodenseitigen Elektrolyten gemessen bzw. eingestellt werden.

Die Mittelkammer der Elektrolysezelle war mit einem Vorratsgefäß verbunden, aus dem über eine Perestaltikpumpe Elektrolyt in die Mittelkammer, dann über das Filtertuch in die Anodenkammer, und von dort über eine pH-Messstelle in ein Sammelgefäß gepumpt werden konnte.

Über einen Thermostaten mit PT 100 Sensor bzw. Wärmetauscher konnte die Temperatur des anodenseitigen Elektrolyten gemessen bzw. eingestellt werden. Der Anolyt wurde nicht im Kreis gefahren.

1.1.2 Versuchsdurchführung:

Der Thermostat für die Kathodenseite wurde auf 90°C eingestellt und gestartet. Es wurden 650 g 1 Gew.-%ige Natriumglykolatlösung in das beheizbare Doppelmantelgefäß eingefüllt und die Peristaltik Pumpe gestartet (Fördermenge 1000 ml/h), dadurch wurde die Natriumglykolatlösung durch eine Leitfähigkeitsmessstelle und einen weiteren Wärmetauscher in die Kathodenkammer der Elektrolysezelle gepumpt. Aus der Kathodenkammer floss dann das Glykolat wieder in den Doppelmantel. Das Glykolat und die Kammer wurden so auf 90 °C temperiert. Der Thermostat

für die NaCl-Seite wurde auf 105 °C gestellt und die Peristaltik Pumpe für die NaCl-Sole gestartet (Fördermenge 4000 ml/h). Die 20 Gew.-%-ige NaCl-Sole mit pH 11 aus einem Vorratsgefäß wurde durch einen Wärmetauscher in die Mittelkammer der Elektrolysezelle gepumpt. Von da floss sie durch das Filtertuch in die Anodenkammer und dann aus der Zelle in eine pH Meßstelle und dann in das Sammelgefäß. Die NaCl-Sole wurde nicht im Kreis gefahren. Als die Kathodenkammer auf 90 °C erhitzt war und die Anodenseite an der pH-Messstelle eine Temperatur von 80°C aufwies, wurde der Strom der Elektrolysezelle angeschaltet. Dafür wurde der Potentiostat in den galvanostatischen Betrieb geschaltet. Die Stromstärke wurde auf 10 Ampere fest eingestellt und die Spannung entsprechend geregelt. Dann wurden die Leitfähigkeitsmessung für das Glykolat und die pH Messung der NaCl Sole aufgezeichnet.

Alle 20 Sekunden wurden Strom, Spannung, Temperatur des Glykolats, Leitfähigkeit des Glykolats, pH der Sole und Temperatur der Sole aufgezeichnet. Der entstehende Wasserstoff und das entstehende Chlor wurden abgesaugt. Das Chlor wurdr in Gaswaschflaschen mit NaOH neutralisiert.

Die Elektrolyse wurde 4 h durchgeführt. Dann wurde der Strom abgeschaltet und die Zelle komplett geleert.

Die Natriumglykolatlösung wies eine Konzentration ~ 20 Gew.-% auf.

*1.2 Depolymerisierung PET mit glykolischer Natriumglykolatösung aus der Elektrolyse*

Im erfindungsgemäßen Verfahren wurden 100 g **PET** in einem Autoklaven mit 800 g Ethylenglykol vorgelegt. Die Lösung wurde anschließend unter Rühren auf 150 °C erhitzt. Sobald die Temperatur von 150 °C erreicht war, wurden 19.5 g 20%-ige Natriumglykolat-Lösung in Ethylenglykol (entsprechend 0.046 mol) aus der Elektrolyse hinzugefügt. Die Reaktion wurde über fünf Stunden durchgeführt und der Reaktoraustrag nach dem Abkühlen untersucht. Der erhaltene Umsatz an **BHET** (1) und Mono-2-hydroxyethylterephthalsäure (= "**MHET**") (2) sowie Terephthalsäure (= "**TS**") (3) ist in der Abbildung 8 dargestellt (bestimmt durch Gaschromatographie; in % Umsatz bezogen auf die Wiederholungseinheit der Struktur (Ξ) des eingesetzten **PETs;** dünne Schraffierung "////").

2. Vergleichsbeispiel **V1:**

In einem Vergleichsversuch wurden 100 g **PET** in einem Autoklaven mit 800 g Ethylenglykol vorgelegt. Die Lösung wurd anschließend unter Rühren auf 150 °C erhitzt. Die Reaktion wird über fünf Stunden durchgeführt und der Reaktoraustrag nach dem Abkühlen untersucht. Der erhaltene Umsatz an **BHET** (1) und **MHET** (2) sowie **TS** (3) ist in der Abbildung 8 dargestellt (schwarz, "■").

3. Vergleichsbeispiel **V2:**

In einem Vergleichsversuch werden 100 g **PET** in einem Autoklaven mit 800 g Ethylenglykol vorgelegt. Die Lösung wurde anschließend unter Rühren auf 150 °C erhitzt. Sobald die Temperatur von 150 °C erreicht war, wurden 3.7 g 50%-ige NaOH-Lösung in Wasser (entsprechend 0.046 mol) hinzugefügt. Die Reaktion wurde über fünf Stunden durchgeführt und der Reaktoraustrag nach dem Abkühlen untersucht. Der erhaltene Umsatz an **BHET** (1) und **MHET** (2) sowie **TS** (3) ist in der Abbildung 8 dargestellt (fette Schraffierung: "////").

4. Ergebnis

Der Vergleich des Gehalts an **BHET, MHET** und **TS** im depolymerisierten Produkt im erfinderischen Beispiel **E1** und den Vergleichsbeispielen **V1, V2** zeigt (siehe Fig. 8), dass bei der Depolymerisierung unter Einsatz der elektrolytisch erhaltenen glykolischen Natriumglykolatlösung ein höherer Anteil an **BHET** erhalten wird. Dies ist vorteilhaft, da dadurch mehr Produkt zur Verfügung steht, welches direkt in einer Polykondensation zu neuem Produkt **PET** umgesetzt werden kann.

**6. Referenzzeichen in den Abbildungen**

| | | |
|---|---|---|
| Elektrolysezelle | **E** | <1> |
| Anodenkammer | $\mathbf{K_A}$ | <11> |
| Zulauf | $\mathbf{Z_{KA}}$ | <110> |
| Ablauf | $\mathbf{A_{KA}}$ | <111> |
| Innenraum | $\mathbf{I_{KA}}$ | <112> |

(fortgesetzt)

| | | |
|---|---|---|
| anodische Elektrode | $E_A$ | <113> |
| Kathodenkammer | $K_K$ | <12> |
| Zulauf | $Z_{KK}$ | <120> |
| Ablauf | $A_{KK}$ | <121> |
| Innenraum | $I_{KK}$ | <122> |
| kathodische Elektrode | $E_K$ | <123> |
| Mittelkammer | $K_M$ | <13> |
| Zulauf | $Z_{KM}$ | <130> |
| Ablauf | $A_{KM}$ | <131> |
| Innenraum | $I_{KM}$ | <132> |
| Diffusionsbarriere | **D** | <14> |
| Verbindung | $V_{AM}$ | <15> |
| Trennwand | **W** | <16> |
| Seite | $S_{KK}$ | <161> |
| Seite | $S_{A/MK}$ | <162> |
| Oberfläche | $O_{KK}$ | <163> |
| Oberfläche | $O_{A/MK}$ | <164> |
| Trennelement | **T** | <17> |
| Teil eines Trennelements | | <171> |
| Teil eines Trennelements | | <172> |
| Festelektrolytkeramik | $F_A$ | <18> |
| Festelektrolytkeramik | $F_B$ | <19> |
| Festelektrolytkeramik | $F_C$ | <28> |
| Festelektrolytkeramik | $F_D$ | <29> |
| Festelektrolytkeramiken | $F_E$, $F_F$, $F_G$, $F_H$, $F_I$ | <30>, <31>, <32>, <33>, <34> |
| Rahmenelement | **R** | <20> |
| Rahmenteil | | <201> |
| Rahmenteil | | <202> |
| Dichtung | **Di** | <40> |
| Scharnier | | <50> |
| Schloss | | <60> |
| Außenwand | $W_A$ | <80> |
| Lösung umfassend $M_A$-Glykolat in Glykol | $L_1$ | <21> |
| Lösung umfassend Glykol | $L_2$ | <22> |
| neutrale oder alkalische, wässrige Lösung eines Salzes **S** umfassend $M_A$ als Kation | $L_3$ | <23> |
| wässrige Lösung von **S,** wobei $[S]_{L4} < [S]_{L3}$. | $L_4$ | <24> |

**Patentansprüche**

1. Verfahren zur Depolymerisierung von Polyethylenterephthalat **PET,** umfassend die folgenden Schritte:

(a) Herstellung einer Lösung $\mathbf{L_1}$ <21> von $M_A$-Glykolat in Glykol, wobei $M_A$ ein Alkalimetallkation ist, in einer Elektrolysezelle **E** <1>, umfassend

- mindestens eine Anodenkammer $\mathbf{K_A}$ <11> mit mindestens einem Zulauf $\mathbf{Z_{KA}}$ <110>, mindestens einem Ablauf $\mathbf{A_{KA}}$ <111> und einem Innenraum $\mathbf{I_{KA}}$ <112>, der eine anodische Elektrode $\mathbf{E_A}$ <113> umfasst,
- mindestens eine Kathodenkammer $\mathbf{K_K}$ <12> mit mindestens einem Zulauf $Z_{KK}$ <120>, mindestens einem Ablauf $\mathbf{A_{KK}}$ <121> und einem Innenraum $\mathbf{I_{KK}}$ <122>, der eine kathodische Elektrode $\mathbf{E_K}$ <123> umfasst,
- und gegebenenfalls mindestens eine dazwischen liegende Mittelkammer $\mathbf{K_M}$ <13> mit mindestens einem Zulauf $\mathbf{Z_{KM}}$ <130>, mindestens einem Ablauf $\mathbf{A_{KM}}$ <131> und einem Innenraum $\mathbf{I_{KM}}$ <132>, wobei dann $\mathbf{I_{KA}}$ <112> und $\mathbf{I_{KM}}$ <132> durch eine Diffusionsbarriere **D** <14> voneinander abgetrennt sind, und $\mathbf{A_{KM}}$ <131> durch eine Verbindung $\mathbf{V_{AM}}$ <15> mit dem Zulauf $\mathbf{Z_{KA}}$ <110> verbunden ist, so dass durch die Verbindung $\mathbf{V_{AM}}$ <15> Flüssigkeit aus $\mathbf{I_{KM}}$ <132> in $\mathbf{I_{KA}}$ <112> geleitet werden kann,

wobei
- in den Fällen, in denen die Elektrolysezelle **E** <1> keine Mittelkammer $\mathbf{K_M}$ <13> umfasst, $\mathbf{I_{KA}}$ <112> und $\mathbf{I_{KK}}$ <122> durch eine Trennwand **W** <16> voneinander abgetrennt sind,
- in den Fällen, in denen die Elektrolysezelle **E** <1> mindestens eine Mittelkammer $\mathbf{K_M}$ <13> umfasst, $\mathbf{I_{KK}}$ <122> und $\mathbf{I_{KM}}$ <132> durch eine Trennwand **W** <16> voneinander abgetrennt sind,

wobei die Trennwand **W** <16> eine Seite $S_{KK}$ <161> mit der Oberfläche $\mathbf{O_{KK}}$ <163> und eine der Seite $\mathbf{S_{KK}}$ <161> gegenüberliegende Seite $\mathbf{S_{A/MK}}$ <162> mit der Oberfläche $\mathbf{O_{A/MK}}$ <164> aufweist, wobei die Trennwand **W** <16> mindestens eine alkalikationenleitende Festelektrolytkeramik $\mathbf{F_A}$ <18> dergestalt umfasst, dass die von der Trennwand **W** <16> umfasste alkalikationen leitende Festelektrolytkeramik $\mathbf{F_A}$ <18> den Innenraum $\mathbf{I_{KK}}$ <122> auf der Seite $\mathbf{S_{KK}}$ <161> über die Oberfläche $\mathbf{O_{KK}}$ <163> direkt kontaktiert,
und wobei

- in den Fällen, in denen die Elektrolysezelle **E** <1> keine Mittelkammer $\mathbf{K_M}$ <13> umfasst, die von der Trennwand **W** <16> umfasste alkalikationenleitende Festelektrolytkeramik $\mathbf{F_A}$ <18> den Innenraum $\mathbf{I_{KA}}$ <112> auf der Seite $\mathbf{S_{A/MK}}$ <162> über die Oberfläche $\mathbf{O_{A/MK}}$ <164> direkt kontaktiert,
- in den Fällen, in denen die Elektrolysezelle **E** <1> mindestens eine Mittelkammer $\mathbf{K_M}$ <13> umfasst, die von der Trennwand **W** <16> umfasste alkalikationenleitende Festelektrolytkeramik $\mathbf{F_A}$ <18> den Innenraum $\mathbf{I_{KM}}$ <132> auf der Seite $\mathbf{S_{A/MK}}$ <162> über die Oberfläche $\mathbf{O_{A/MK}}$ <164> direkt kontaktiert,

($\alpha$) wobei in der Elektrolysezelle **E** <1>, wenn diese keine Mittelkammer $\mathbf{K_M}$ <13> umfasst, die folgenden, gleichzeitig ablaufenden Schritte ($\alpha$1), ($\alpha$2), ($\alpha$3) durchgeführt werden:

($\alpha$1) eine Lösung $\mathbf{L_2}$ <22> umfassend Glykol wird durch $\mathbf{I_{KK}}$ <122> geleitet,
($\alpha$2) eine neutrale oder alkalische, wässrige Lösung $\mathbf{L_3}$ <23> eines Salzes **S** umfassend $M_A$ als Kation wird durch $I_{KA}$ <112> geleitet,
($\alpha$3) zwischen $\mathbf{E_A}$ <113> und $\mathbf{E_K}$ <123> wird Spannung angelegt,

oder
($\beta$) wobei in der Elektrolysezelle E <1>, wenn diese mindestens eine Mittelkammer $\mathbf{K_M}$ <13> umfasst, die folgenden, gleichzeitig ablaufenden Schritte ($\beta$1), ($\beta$2), ($\beta$3) durchgeführt werden:

($\beta$1) eine Lösung $\mathbf{L_2}$ <22> umfassend Glykol wird durch $I_{KK}$ <122> geleitet,
($\beta$2) eine neutrale oder alkalische, wässrige Lösung $\mathbf{L_3}$ <23> eines Salzes **S** umfassend $M_A$ als Kation wird durch $\mathbf{I_{KM}}$ <132>, dann über $\mathbf{V_{AM}}$ <15>, dann durch $I_{KA}$ <112> geleitet,
($\beta$3) zwischen $\mathbf{E_A}$ <113> und $\mathbf{E_K}$ <123> wird Spannung angelegt,

wodurch am Ablauf $\mathbf{A_{KK}}$ <121> die Lösung $\mathbf{L_1}$ <21> erhalten wird, wobei die Konzentration von $M_A$-Glykolat in $\mathbf{L_1}$ <21> höher ist als in $L_2$ <22>,
und wodurch am Ablauf $\mathbf{A_{KA}}$ <111> eine wässrige Lösung $\mathbf{L_4}$ <24> von **S** erhalten wird, wobei die Konzentration von **S** in $\mathbf{L_4}$ <24> geringer ist als in $\mathbf{L_3}$ <23>;

(b) Umsetzung der Lösung $L_1$ <21> mit **PET** zu einer Mischung $\mathbf{M_1}$ umfassend Bis-2-hydroxyethylterephthalat **BHET.**

**2.** Verfahren nach Anspruch 1, wobei die alkalikationenleitende Festelektrolytkeramik $\mathbf{F_A}$ <18> eine Struktur der Formel $M^{I}_{1+2w+x-y+z}\ M^{II}_{w}\ M^{III}_{x}\ Zr^{IV}_{2-w-x-y}\ M^{V}_{y}\ (SiO_4)_z\ (PO_4)_{3-z}$ aufweist,

wobei $M^I$ ausgewählt aus $Na^+$, $Li^+$ ist,
$M^{II}$ ein zweiwertiges Metallkation ist,
$M^{III}$ ein dreiwertiges Metallkation ist,
$M^V$ ein fünfwertiges Metallkation ist,
die römischen Indizes I, II, III, IV, V die Oxidationszahlen angeben, in der die jeweiligen Metallkationen vorliegen,
und w, x, y, z reelle Zahlen sind, wobei gilt, dass $0 \leq x < 2$, $0 \leq y < 2$, $0 \leq w < 2$, $0 \leq z < 3$,
und wobei w, x, y, z so gewählt werden, dass $1 + 2w + x - y + z \geq 0$ und $2 - w - x - y \geq 0$ gilt.

3. Verfahren nach Anspruch 1 oder 2, wobei die Elektrolysezelle **E** <1> keine Mittelkammer $\mathbf{K_M}$ <13> umfasst.

4. Verfahren nach Anspruch 1 oder 2, wobei die Elektrolysezelle **E** <1> mindestens eine Mittelkammer $\mathbf{K_M}$ <13> umfasst.

5. Verfahren nach einem der Ansprüche 1 bis 4, wobei $M_A$ aus der Gruppe bestehend aus Kalium, Natrium ausgewählt ist.

6. Verfahren nach einem der Ansprüche 1 bis 5, wobei Schritt (b) so lange durchgeführt wird, bis sich mindestens **P** = 10 % des in Schritt (b) eingesetzten **PET**s umgesetzt haben.

7. Verfahren nach einem der Ansprüche 1 bis 6, wobei Schritt (b) bei der Siedetemperatur des Glykols durchgeführt wird.

8. Verfahren nach einem der Ansprüche 1 bis 7, wobei in Schritt (b) so viel Lösung $L_1$ <21> eingesetzt wird, dass das Gesamtgewicht des in Schritt (b) eingesetzten $M_A$-Glykolats bezogen auf das Gesamtgewicht des in Schritt (b) eingesetzten **PET**s im Bereich von 0.1 bis 100 Gew.-% liegt.

9. Verfahren nach einem der Ansprüche 1 bis 8, wobei **BHET** in einem weiteren Schritt (c) mindestens teilweise aus $\mathbf{M_1}$ abgetrennt wird.

10. Verfahren nach Anspruch 9, wobei die mindestens teilweise Abtrennung von **BHET** aus $M_1$ in Schritt (c) durch Kristallisation und/oder Destillation erfolgt.

11. Verfahren nach einem der Ansprüche 1 bis 10, wobei das **PET** mindestens einem Vorbehandlungsschritt ausgewählt aus chemischen Vorbehandlungsschritt, Zerkleinerungsschritt unterworfen wird, bevor es in Schritt (b) eingesetzt wird.

12. Verfahren zum Recycling von Polyethylenterephthalat, in welchem mit einem Verfahren nach einem der Ansprüche 1 bis 11 **BHET** erhalten wird und in einem Schritt (ζ) das so erhaltene **BHET zu PET** polymerisiert wird.

13. Verfahren nach Anspruch 12, wobei die Polymerisierung von **BHET zu PET** in Schritt (ζ) bei mindestens der Siedetemperatur des Glykols durchgeführt wird.

14. Verfahren nach Anspruch 12 oder 13, wobei die Polymerisierung in Schritt (ζ) in Gegenwart eines Katalysators durchgeführt wird.

15. Verfahren nach Anspruch 14, wobei der Katalysator aus der Gruppe bestehend aus Antimonverbindungen ausgewählt ist.

## Claims

1. Method of depolymerization of polyethylene terephthalate PET, comprising the following steps:

(a) producing a solution $L_1$ <21> of $M_A$ glycolate in glycol, where $M_A$ is an alkali metal cation, in an electrolysis cell E <1> comprising

- at least one anode chamber $K_A$ <11> having at least one inlet $Z_{KA}$ <110>, at least one outlet $A_{KA}$ <111>, and an interior $I_{KA}$ <112> comprising an anodic electrode $E_A$ <113>,
- at least one cathode chamber $K_K$ <12> having at least one inlet $Z_{KK}$ <120>, at least one outlet $A_{KK}$ <121>,

and an interior $I_{KK}$ <122> comprising a cathodic electrode $E_K$ <123>,
- and optionally at least one interposed middle chamber $K_M$ <13> having at least one inlet $Z_{KM}$ <130>, at least one outlet $A_{KM}$ <131> and an interior $I_{KM}$ <132>, wherein $I_{KA}$ <112> and $I_{KM}$ <132> are then divided from one another by a diffusion barrier D <14>, and $A_{KM}$ <131> is connected by a connection $V_{AM}$ <15> to the inlet $Z_{KA}$ <110>, such that liquid can be passed from $I_{KM}$ <132> into $I_{KA}$ <112> via the connection $V_{AM}$ <15>, wherein
- in the cases in which the electrolysis cell E <1> does not comprise a middle chamber $K_M$ <13>, $I_{KA}$ <112> and $I_{KK}$ <122> are divided from one another by a dividing wall W <16>,
- in the cases in which the electrolysis cell E <1> comprises at least one middle chamber $K_M$ <13>, $I_{KK}$ <122> and $I_{KM}$ <132> are divided from one another by a dividing wall W <16>,
wherein the dividing wall W <16> has one side $S_{KK}$ <161> having the surface $O_{KK}$ <163> and a side $S_{A/MK}$ <162> which is opposite the side $S_{KK}$ <161> and has the surface $O_{A/MK}$ <164>, wherein the dividing wall W <16> comprises at least one alkali metal cation-conducting solid-state electrolyte ceramic $F_A$ <18> in such a way that the alkali metal cation-conducting solid-state electrolyte ceramic $F_A$ <18> encompassed by the dividing wall W <16> makes direct contact with the interior $I_{KK}$ <122> on the side $S_{KK}$ <161> via the surface $O_{KK}$ <163>,
and wherein

- in the cases in which the electrolysis cell E <1> does not comprise a middle chamber $K_M$ <13>, the alkali metal cation-conducting solid-state electrolyte ceramic $F_A$ <18> encompassed by the dividing wall W <16> makes direct contact with the interior $I_{KA}$ <112> on the side $S_{A/MK}$ <162> via the surface $O_{A/MK}$ <164>,
- in the cases in which the electrolysis cell E <1> comprises at least one middle chamber $K_M$ <13>, the alkali metal cation-conducting solid-state electrolyte ceramic $F_A$ <18> encompassed by the dividing wall W <16> makes direct contact with the interior $I_{KM}$ <132> on the side $S_{A/MK}$ <162> via the surface $O_{A/MK}$ <164>,

($\alpha$) wherein, in the electrolysis cell E <1>, when it does not comprise a middle chamber $K_M$ <13>, the following steps ($\alpha$1), ($\alpha$2), ($\alpha$3) that proceed simultaneously are performed:

($\alpha$1) a solution $L_2$ <22> comprising glycol is directed through $I_{KK}$ <122>,
($\alpha$2) a neutral or alkaline, aqueous solution $L_3$ <23> of a salt S comprising $M_A$ as cation is directed through $I_{KA}$ <112>,
($\alpha$3) voltage is applied between $E_A$ <113> and $E_K$ <123>, or

($\beta$) wherein, in the electrolysis cell E <1>, when it comprises at least one middle chamber $K_M$ <13>, the following steps ($\beta$1), ($\beta$2), ($\beta$3) that proceed simultaneously are performed:

($\beta$1) a solution $L_2$ <22> comprising glycol is directed through $I_{KK}$ <122>,
($\beta$2) a neutral or alkaline, aqueous solution $L_3$ <23> of a salt S comprising $M_A$ as cation is directed through $I_{KM}$ <132>, then through $V_{AM}$ <15>, then through $I_{KA}$ <112>,
($\beta$3) voltage is applied between $E_A$ <113> and $E_K$ <123>, which affords the solution $L_1$ <21> at the outlet $A_{KK}$ <121>, the concentration of $M_A$ glycolate being higher in $L_1$ <21> than in $L_2$ <22>,

and which affords an aqueous solution $L_4$ <24> of S at the outlet $A_{KA}$ <111>, the concentration of S being lower in $L_4$ <24> than in $L_3$ <23>;

(b) reacting the solution $L_1$ <21> with PET to give a mixture $M_1$ comprising bis-2-hydroxyethyl terephthalate BHET.

**2.** Method according to Claim 1, wherein the alkali metal cation-conducting solid-state electrolyte ceramic $F_A$ <18> has a structure of the formula $M^{I}_{1+2w+x-y+z}\ M^{II}_{w}\ M^{III}_{x}\ Zr^{IV}_{2-w-x-y}\ M^{V}_{y}\ (SiO_4)_z\ (PO_4)_{3-z}$,

where $M^I$ is selected from $Na^+$ and $Li^+$,
$M^{II}$ is a divalent metal cation,
$M^{III}$ is a trivalent metal cation,
$M^V$ is a pentavalent metal cation,
the Roman indices I, II, III, IV, V indicate the oxidation numbers in which the respective metal cations exist,

and w, x, y, z are real numbers, where $0 \leq x < 2$, $0 \leq y < 2$, $0 \leq w < 2$, $0 \leq z < 3$,
and where w, x, y, z are chosen such that $1 + 2w + x - y + z \geq 0$ and $2 - w - x - y \geq 0$.

**3.** Method according to Claim 1 or 2, wherein the electrolysis cell E <1> does not comprise a middle chamber $K_M$ <13>.

**4.** Method according to Claim 1 or 2, wherein the electrolysis cell E <1> comprises at least one middle chamber $K_M$ <13>.

**5.** Method according to any of Claims 1 to 4, wherein $M_A$ is selected from the group consisting of potassium, sodium.

**6.** Method according to any of Claims 1 to 5, wherein step (b) is conducted until at least P = 10% of the PET used in step (b) has been converted.

**7.** Method according to any of Claims 1 to 6, wherein step (b) is performed at the boiling temperature of the glycol.

**8.** Method according to any of Claims 1 to 7, wherein a sufficient amount of solution $L_1$ <21> is used in step (b) that the total weight of the $M_A$ glycolate used in step (b), based on the total weight of the PET used in step (b), is in the range from 0.1% to 100% by weight.

**9.** Method according to any of Claims 1 to 8, wherein BHET is at least partly separated from $M_1$ in a further step (c).

**10.** Method according to Claim 9, wherein the at least partial separation of BHET from $M_1$ in step (c) is effected by crystallization and/or distillation.

**11.** Method according to any of Claims 1 to 10, wherein the PET is subjected to at least one pretreatment step selected from chemical pretreatment step, comminution step, before being used in step (b).

**12.** Method of recycling polyethylene terephthalate, in which BHET is obtained by a method according to any of Claims 1 to 11 and the BHET thus obtained is polymerized to PET in a step ($\zeta$).

**13.** Method according to Claim 12, wherein the polymerization of BHET to PET in step ($\zeta$) is conducted at at least the boiling temperature of the glycol.

**14.** Method according to Claim 12 or 13, wherein the polymerization in step ($\zeta$) is performed in the presence of a catalyst.

**15.** Method according to Claim 14, wherein the catalyst is selected from the group consisting of antimony compounds.

## Revendications

**1.** Procédé de dépolymérisation de poly(téréphtalate d'éthylène) PET, comprenant les étapes suivantes :

(a) préparation d'une solution $L_1$ <21> de glycolate de $M_A$ dans du glycol, $M_A$ représentant un cation de métal alcalin, dans une cellule d'électrolyse E <1>, comprenant

- au moins une chambre anodique $K_A$ <11> présentant au moins une entrée $Z_{KA}$ <110>, au moins une sortie $A_{KA}$ <111> et une chambre interne $I_{KA}$ <112>, qui comprend l'électrode anodique $E_A$ <113>,
- au moins une chambre cathodique $K_K$ <12> présentant au moins une entrée $Z_{KK}$ <120>, au moins une sortie $A_{KK}$ <121> et une chambre interne $I_{KK}$ <122>, qui comprend l'électrode cathodique $E_K$ <123>,
- et le cas échéant au moins une chambre centrale $K_M$ <13>, située entre celles-ci, présentant au moins une entrée $Z_{KM}$ <130>, au moins une sortie $A_{KM}$ <131> et une chambre interne $I_{KM}$ <132>,
$I_{KA}$ <112> et $I_{KM}$ <132> étant alors séparées l'une de l'autre par une barrière de diffusion D <14>, et $A_{KM}$ <131> étant reliée à l'entrée $Z_{KA}$ <110> par une liaison $V_{AM}$ <15>, de telle sorte que du liquide peut être guidé à partir de $I_{KM}$ <132> dans $I_{KA}$ <112> à travers la liaison $V_{AM}$ <15>, dans lequel
- dans les cas où la cellule d'électrolyse E <1> ne comprend pas de chambre centrale $K_M$ <13>, $I_{KA}$ <112> et $I_{KK}$ <122> sont séparées l'une de l'autre par une paroi de séparation W <16>,
- dans les cas où la cellule d'électrolyse E <1> comprend au moins une chambre centrale $K_M$ <13>, $I_{KK}$ <122> et $I_{KM}$ <132> sont séparées l'une de l'autre par une paroi de séparation W <16>,
la paroi de séparation W <16> présentant une face $S_{KK}$ <161> de surface $O_{KK}$ <163> et une face $S_{A/MK}$

<162> de surface $O_{A/MK}$ <164> opposée à la face $S_{KK}$ <161>, la paroi de séparation W <16> comprenant au moins une céramique électrolytique solide $F_A$ <18> guidant les cations alcalins de telle sorte que la céramique électrolytique solide $F_A$ <18> guidant les cations alcalins incluse dans la paroi de séparation W <16> est en contact direct avec la chambre interne $I_{KK}$ <122> sur la face $S_{KK}$ <161 > par l'intermédiaire de la surface $O_{KK}$ <163>,
et
- dans les cas où la cellule d'électrolyse E <1> ne comprend pas de chambre centrale $K_M$ <13>, la céramique électrolytique solide $F_A$ <18> guidant les cations alcalins incluse dans la paroi de séparation W <16> étant en contact direct avec la chambre interne $I_{KA}$ <112> sur la face $S_{A/MK}$ <162> par l'intermédiaire de la surface $O_{A/MK}$ <164>,
- dans les cas où la cellule d'électrolyse E <1> comprend au moins une chambre centrale $K_M$ <13>, la céramique électrolytique solide $F_A$ <18> guidant les cations alcalins incluse dans la paroi de séparation W <16> étant en contact direct avec la chambre interne $I_{KM}$ <132> sur la face $S_{A/MK}$ <162> par l'intermédiaire de la surface $O_{A/MK}$ <164>,

(α) les étapes (α1), (α2), (α3) suivantes, se déroulant simultanément étant effectuées dans la cellule d'électrolyse E <1>, lorsque celle-ci ne contient pas de chambre centrale $K_M$ <13> :

(α1) une solution $L_2$ <22> comprenant du glycol est guidée à travers $I_{KK}$ <122>,
(α2) une solution aqueuse, neutre ou alcaline $L_3$ <23> d'un sel S comprenant $M_A$ en tant que cation est guidée à travers $I_{KA}$ <112>,
(α3) une tension est appliquée entre $E_A$ <113> et $E_K$ <123>, ou

(β) les étapes (β1), (β2), (β3) suivantes, se déroulant simultanément étant effectuées dans la cellule d'électrolyse E <1>, lorsque celle-ci contient au moins une chambre centrale $K_M$ <13> :

(β1) une solution $L_2$ <22> comprenant du glycol est guidée à travers $I_{KK}$ <122>,
(β2) une solution aqueuse, neutre ou alcaline $L_3$ <23> d'un sel S comprenant $M_A$ en tant que cation est guidée à travers $I_{KM}$ <132>, puis via $V_{AM}$ <15>, ensuite à travers $I_{KA}$ <112>,
(β3) une tension est appliquée entre $E_A$ <113> et $E_K$ <123>, suite à quoi, à la sortie $A_{KK}$ <121>, la solution $L_1$ <21> est obtenue, la concentration en glycolate de $M_A$ dans $L_1$ <21> étant plus élevée que dans $L_2$ <22>,
et suite à quoi, à la sortie $A_{KA}$ <111>, une solution aqueuse $L_4$ <24> de S est obtenue, la concentration en S dans $L_4$ <24> étant plus faible que dans $L_3$ <23>,

(b) transformation de la solution $L_1$ <21> avec du PET en un mélange $M_1$ comprenant du téréphtalate de bis-2-hydroxyéthyle BHET.

2. Procédé selon la revendication 1, dans lequel la céramique électrolytique solide $F_A$ <18> guidant les cations alcalins présente une structure de formule $M^{I}_{1+2w+x-y+z}\, M^{II}_{w}\, M^{III}_{x}\, Zr^{IV}_{2-w-x-y}\, M^{V}_{y}\, (SiO_4)_z\, (PO_4)_{3-z}$,

dans laquelle $M^I$ est choisi parmi $Na^+$, $Li^+$,
$M^{II}$ représente un cation métallique bivalent,
$M^{III}$ représente un cation métallique trivalent,
$M^V$ représentant un cation métallique pentavalent,
les exposants en chiffres romains I, II, III, IV, V indiquent les nombres d'oxydation dans lesquels sont présents les cations métalliques respectifs,
et w, x, y, z sont des nombres réels, à condition que $0 \leq x < 2$, $0 \leq y < 2$, $0 \leq w < 2$, $0 \leq z < 3$,
et w, x, y, z étant choisis de telle sorte que $1 + 2w + x-y + z \geq 0$ et $2 - w - x - y \geq 0$.

3. Procédé selon la revendication 1 ou 2, dans lequel la cellule d'électrolyse E <1> ne comprend pas de chambre centrale $K_M$ <13>.

4. Procédé selon la revendication 1 ou 2, dans lequel la cellule d'électrolyse E <1> comprend au moins une chambre centrale $K_M$ <13>.

5. Procédé selon l'une des revendications 1 à 4, dans lequel $M_A$ est choisi dans le groupe constitué par potassium, sodium.

6. Procédé selon l'une des revendications 1 à 5, dans lequel l'étape (b) est réalisée jusqu'à ce qu'au moins P = 10% du PET utilisé dans l'étape (b) soient transformés.

7. Procédé selon l'une des revendications 1 à 6, dans lequel l'étape (b) est réalisée à la température d'ébullition du glycol.

8. Procédé selon l'une des revendications 1 à 7, dans lequel, dans l'étape (b), on utilise une quantité de solution $L_1$ <21> telle que le poids total du glycolate de $M_A$ utilisé dans l'étape (b), par rapport au poids total du PET utilisé dans l'étape (b), se situe dans la plage de 0,1 à 100% en poids.

9. Procédé selon l'une des revendications 1 à 8, dans lequel le BHET est séparé au moins partiellement à partir de $M_1$ dans une autre étape (c).

10. Procédé selon la revendication 9, dans lequel la séparation au moins partielle du BHET à partir de $M_1$ dans l'étape (c) est effectuée par cristallisation et/ou distillation.

11. Procédé selon l'une des revendications 1 à 10, dans lequel le PET est soumis à au moins une étape de prétraitement choisie parmi une étape de prétraitement chimique et une étape de broyage avant d'être utilisé dans l'étape (b).

12. Procédé de recyclage de poly(téréphtalate d'éthylène), dans lequel, à l'aide d'un procédé selon l'une des revendications 1 à 11, du BHET est obtenu et, dans une étape (ζ), le BHET ainsi obtenu est polymérisé en PET.

13. Procédé selon la revendication 12, dans lequel la polymérisation de BHET en PET dans l'étape (ζ) est réalisée à au moins la température d'ébullition du glycol.

14. Procédé selon la revendication 12 ou 13, dans lequel la polymérisation dans l'étape (ζ) est réalisée en présence d'un catalyseur.

15. Procédé selon la revendication 14, dans lequel le catalyseur est choisi dans le groupe constitué par les composés d'antimoine.

Abbildungen

Fig. 1 A

Fig. 1 B

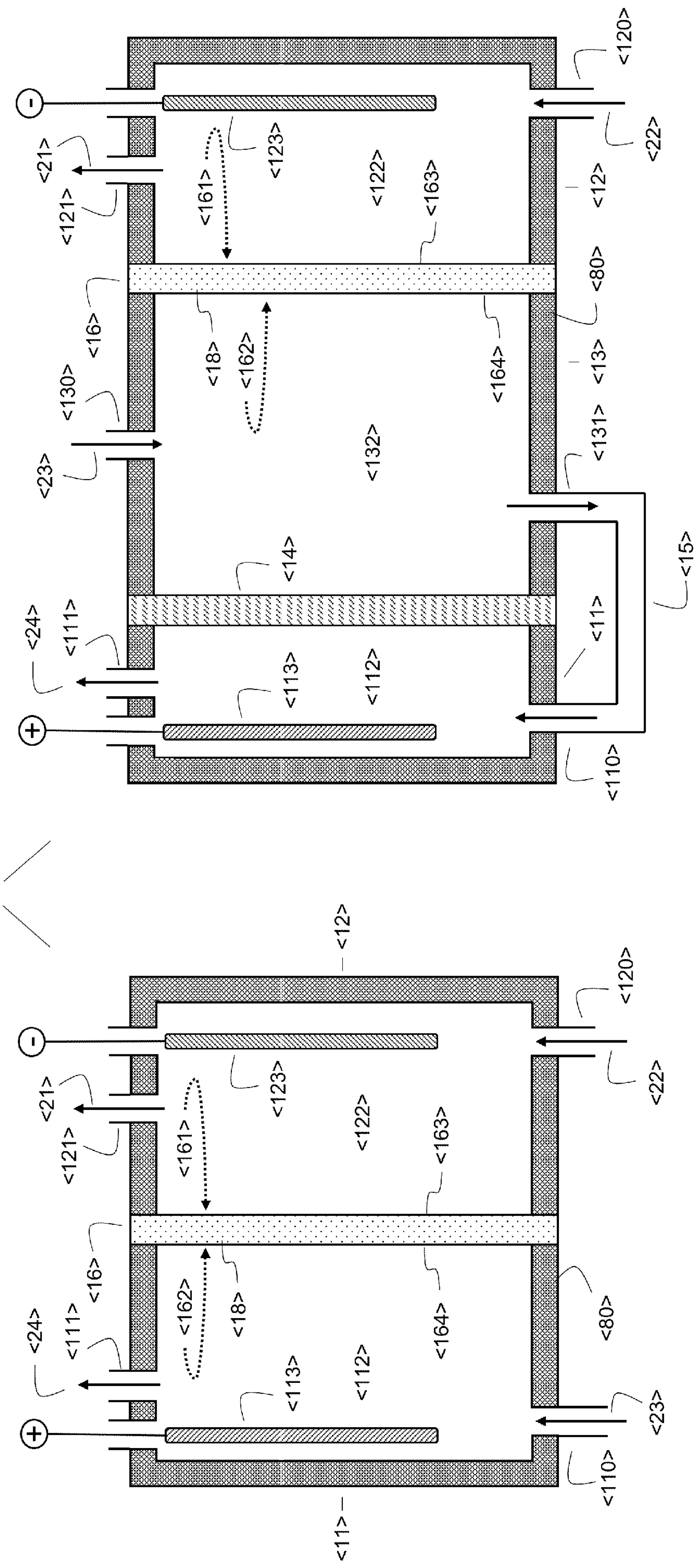

Fig. 3 B

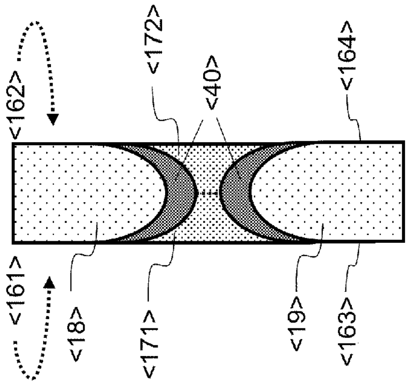


Fig. 3 A

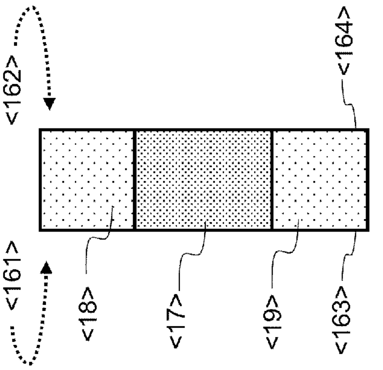


Fig. 3 C

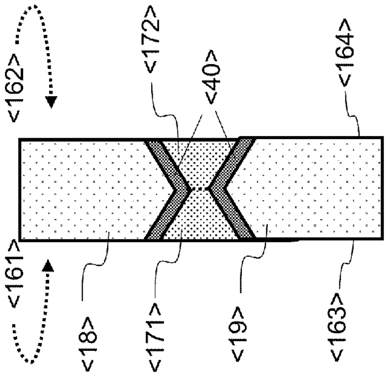


Fig. 2 B

Fig. 2 A

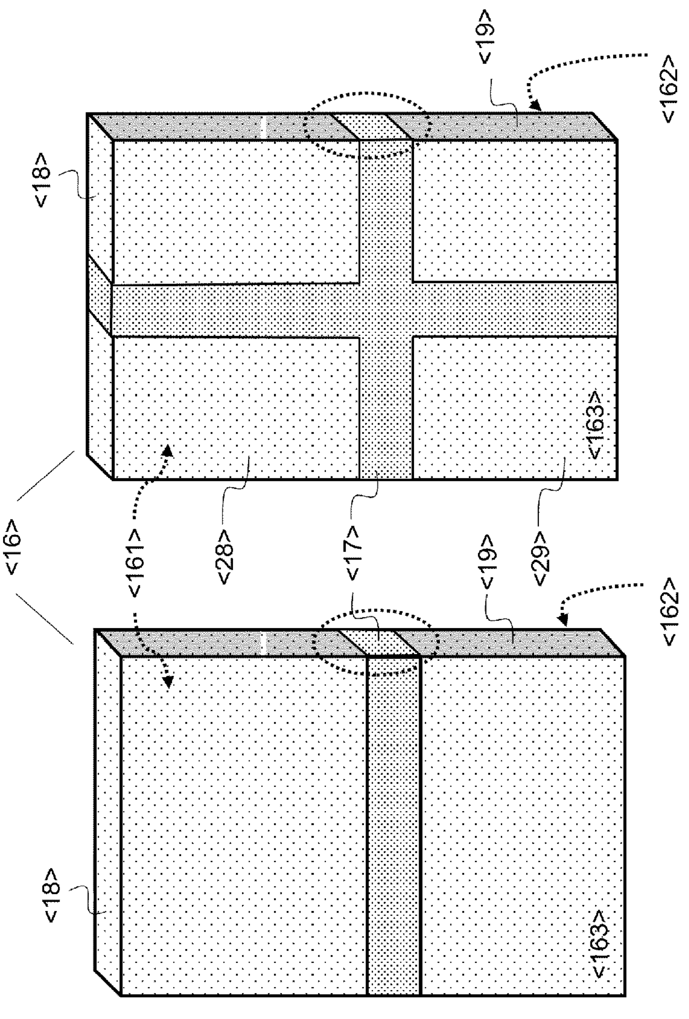

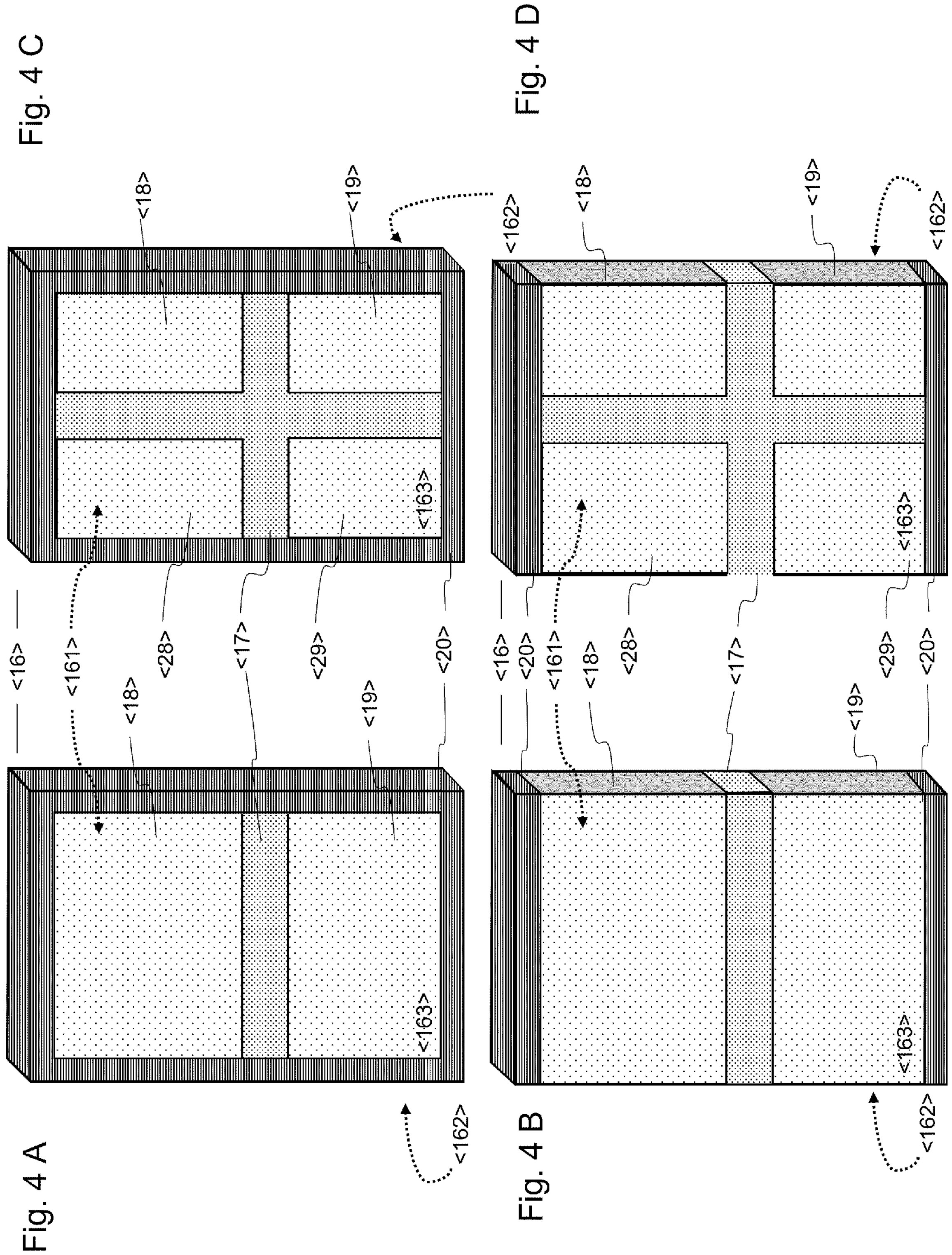


Fig. 4 A

Fig. 4 B

Fig. 4 C

Fig. 4 D

Fig. 5 A

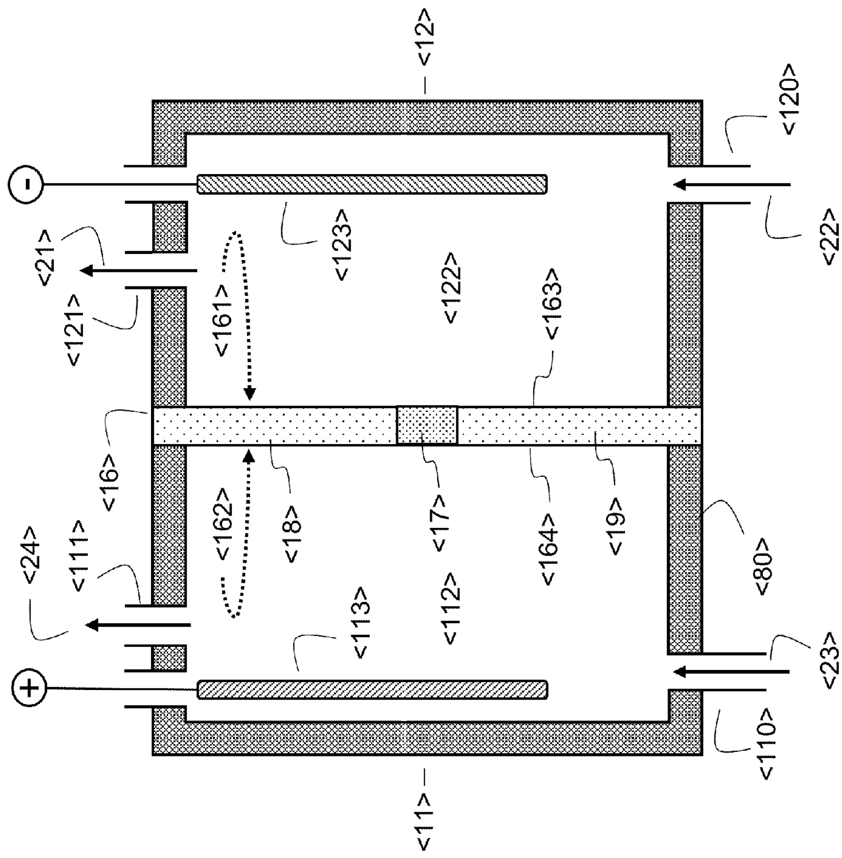
<11>
<12>
<110>
<111>
<112>
<113>
<120>
<121>
<122>
<123>
<16>
<161>
<162>
<163>
<164>
<17>
<18>
<19>
<21>
<22>
<23>
<24>
<80>
+
-

Fig. 5 B

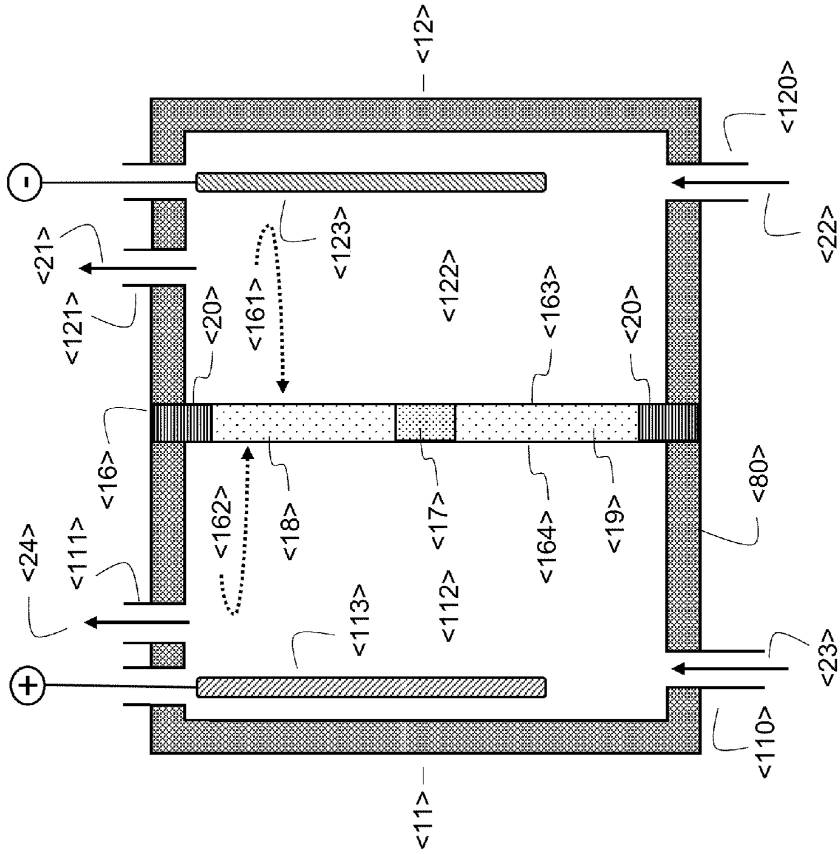
<11>
<12>
<110>
<111>
<112>
<113>
<120>
<121>
<122>
<123>
<16>
<161>
<162>
<163>
<164>
<17>
<18>
<19>
<20>
<20>
<21>
<22>
<23>
<24>
<80>
+
-

Fig. 6 B

Fig. 6 A

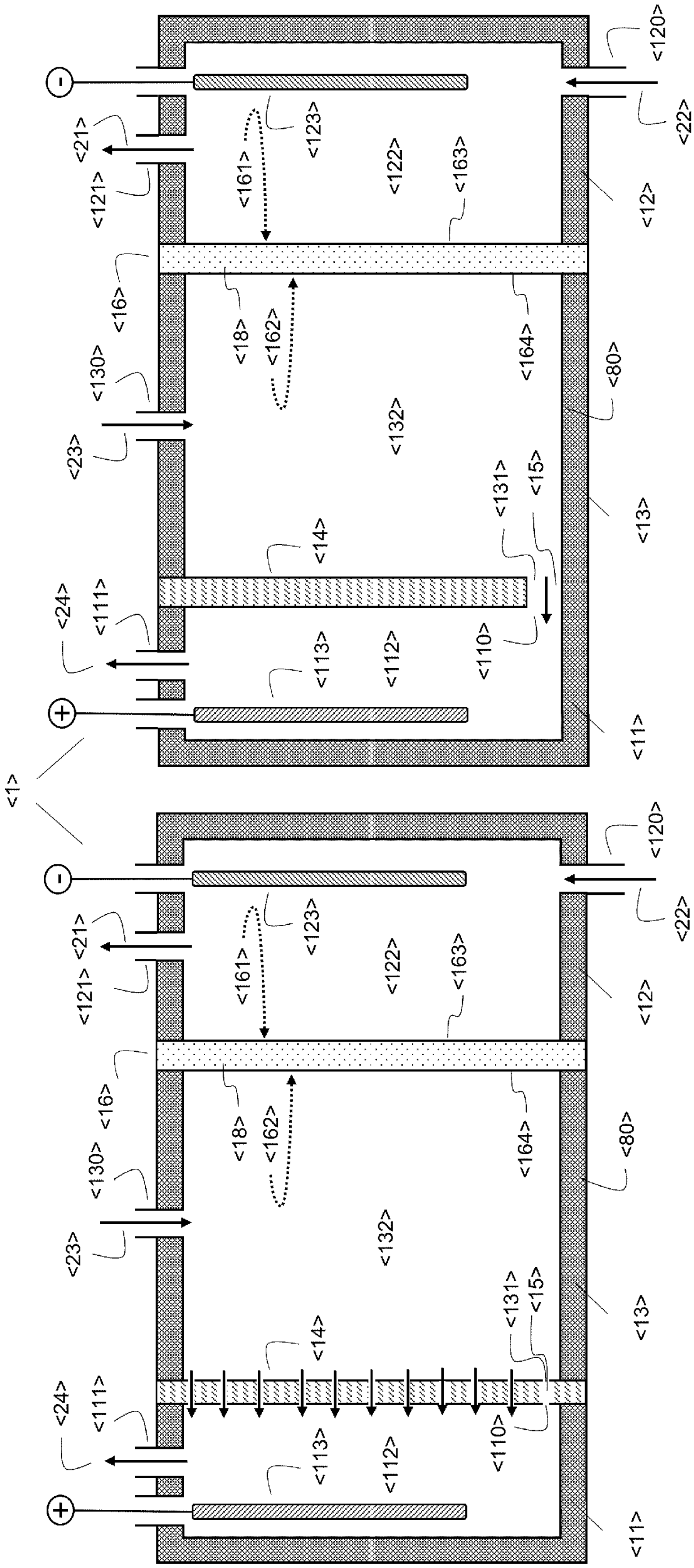
<1>
<11>
<12>
<13>
<14>
<15>
<16>
<18>
<21>
<22>
<23>
<24>
<80>
<110>
<111>
<112>
<113>
<120>
<121>
<122>
<123>
<130>
<131>
<132>
<161>
<162>
<163>
<164>
+
-

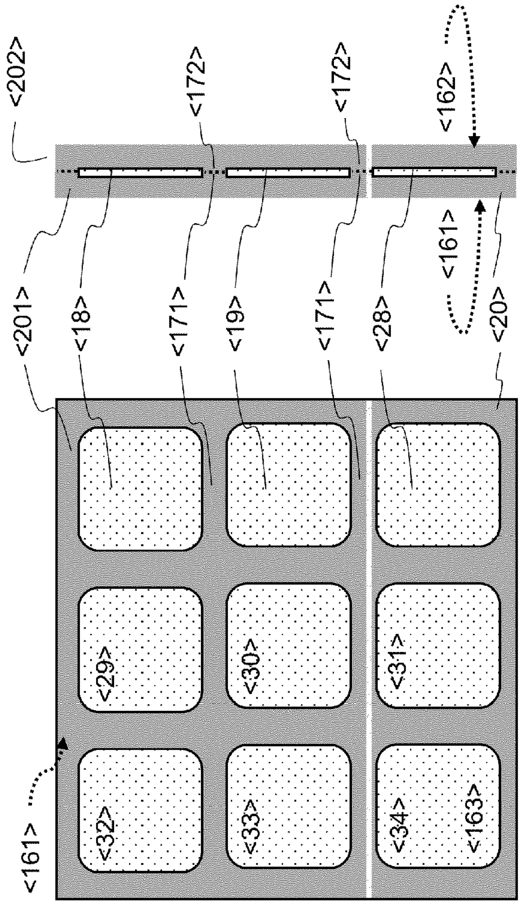
<202>
<172>
<172>
<162>
<201>
<18>
<171>
<19>
<171>
<28>
<161>
<20>
<29>
<30>
<31>
<161>
<32>
<33>
<34>
<163>

Fig. 7 B

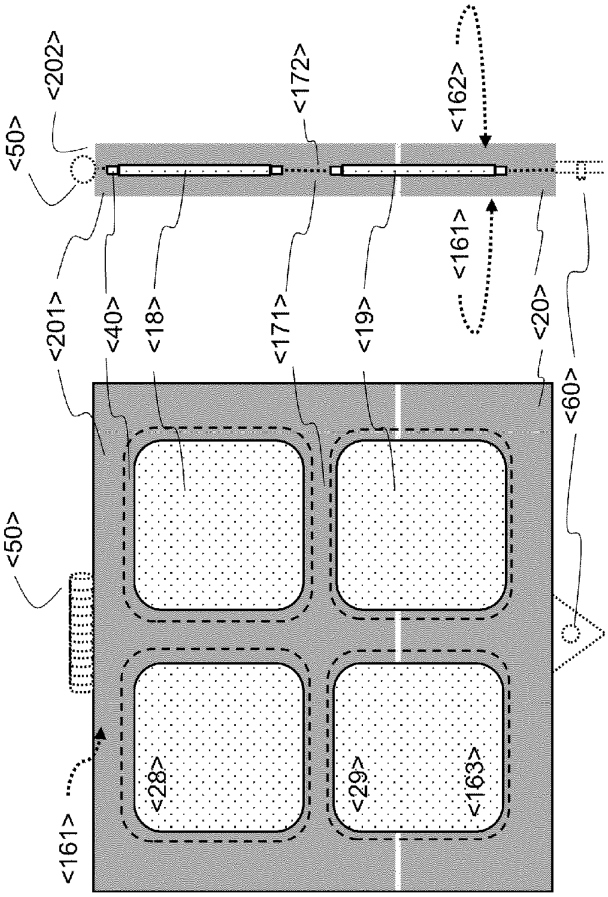
<50>
<202>
<172>
<162>
<201>
<40>
<18>
<171>
<19>
<161>
<20>
<60>
<50>
<161>
<28>
<29>
<163>

Fig. 7 A

Fig. 8

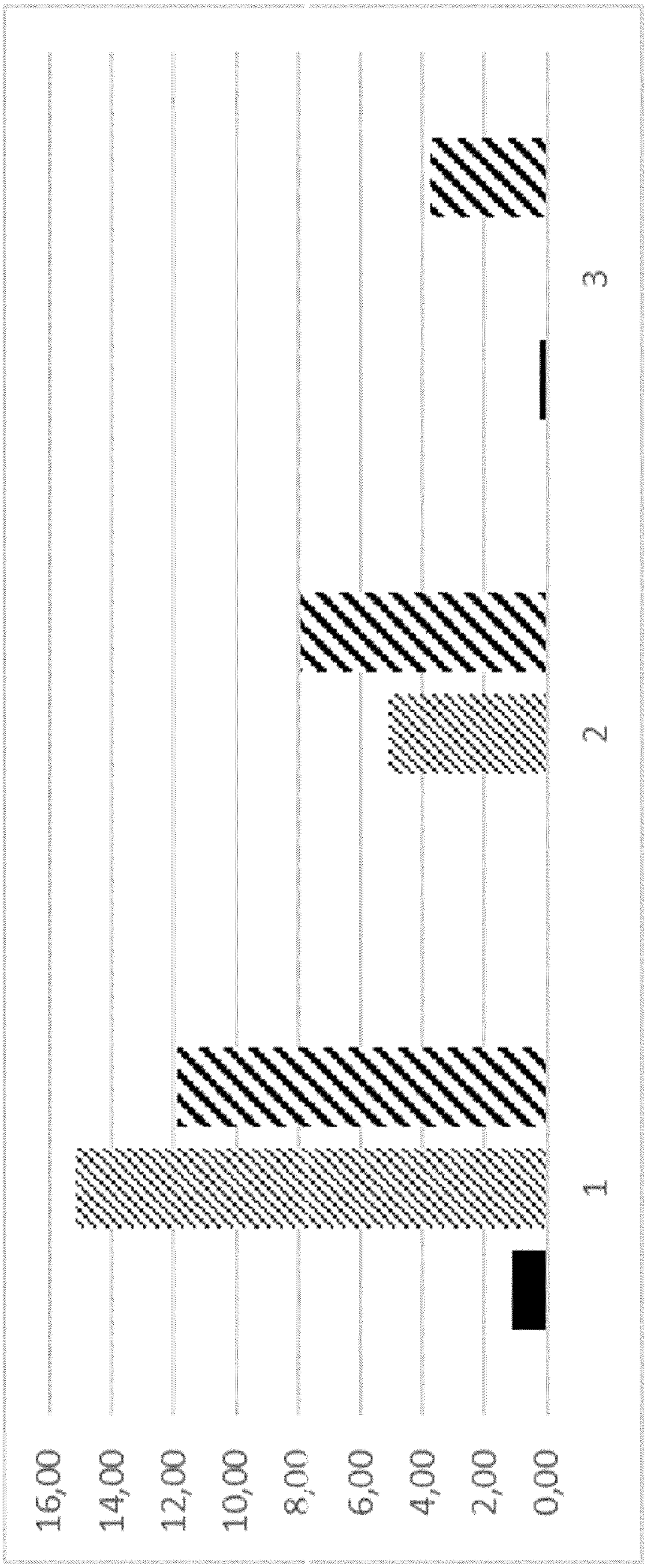

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- GB 784248 A
- JP 2000309663 A
- US 4355175 A
- EP 0723951 A1
- US 3222299 A
- WO 2020002999 A2
- EP 1457479 A1
- DD 258143 A3
- US 20060226022 A1
- WO 2014008410 A1
- DE 10360758 A1
- WO 2007048712 A2
- DE 102010062804 A1
- US 4831146 A
- WO 2008076327 A1
- US 20100044242 A1
- US 20160204459 A1
- DE 102015013155 A1
- WO 2012048032 A2
- DE 10032899 C2


### In der Beschreibung aufgeführte Nicht-Patentliteratur


- *CHEMICAL ABSTRACTS*, 959-26-2
- Polyethylenterephthalate, RD-16-03258. **W. CASERI** ; **F. BÖCKLER** ; **B. DILL** ; **G. EISENBRAND** ; **F. FAUPEL** ; **B. FUGMANN** ; **T. GAMSE** ; **R. MATISSEK** ; **G. POHNERT** ; **A. RÜHLING**. RÖMPP. Georg Thieme Verlag, 2009
- **T. YOSHIOKA** ; **N. OKAYAMA** ; **A. OKUWAKI**. *Ind. Eng. Chem. Res.*, 1998, vol. 37, 336-340
- **S.R. SHUKLA** ; **A.M. HARAD**. *Journal of Applied Polymer Science*, 2005, vol. 97, 513-517
- **N.D. PINGALE** ; **S.R. SHUKLA**. *European Polymer Journal*, 2008, vol. 44, 4151-4156
- *CHEMICAL ABSTRACTS*, 107-21-1
- **M.A. HICKNER** ; **A.M. HERRING** ; **E.B. COUGHLIN**. *Journal of Polymer Science, Part B: Polymer Physics*, 2013, vol. 51, 1727-1735
- **C.G. ARGES** ; **V. RAMANI** ; **P.N. PINTAURO**. Electrochemical Society Interface. 2010, vol. 19, 31-35
- **VOLKMAR M. SCHMIDT**. Elektrochemische Verfahrenstechnik: Grundlagen, Reaktionstechnik, Prozessoptimierung. 08 October 2003, 181
- *CHEMICAL ABSTRACTS*, 31175-20-9
- **S.A. MAREEV** ; **D.YU. BUTYLSKII** ; **N.D. PISMENSKAYA** ; **C. LARCHET** ; **L. DAMMAK** ; **V.V. NIKONENKO**. *Journal of Membrane Science*, 2018, vol. 563, 768-776
- **N. ANANTHARAMULU** ; **K. KOTESWARA RAO** ; **G. RAMBABU** ; **B. VIJAYA KUMAR** ; **VELCHURI RADHA** ; **M. VITHAL**. *J Mater Sci*, 2011, vol. 46, 2821-2837
- Poly(Ethylen Terephthalate) Polymerization - Mechanism, Catalysis, Kinetics, Mass Transfer and Reactor Design'' auf Seite 92 des Buches. **TH. RIECKMANN** ; **S. VÖLKER**. Modern Polyesters: Chemistry and Technology of Polyesters and Copolyesters. John Wiley & Sons, Ltd, 2003